# EUROPÄISCHE PATENTSCHRIFT

(11) **EP 1 920 777 B1**
(45) Veröffentlichungstag und Bekanntmachung des Hinweises auf die Patenterteilung: **07.09.2011**
(21) Anmeldenummer: 06023573.6
(22) Anmeldetag: 13.11.2006
(51) Int. Cl.: A61K 36/185, C12C 3/08, A01N 65/00, A23L 3/3472, A61P 31/04, A61P 25/20, A23L 1/30

(54) **Extraktionsverfahren zur klassifizierten Gewinnung und Trennung von pflanzlichen Inhaltsstoffen und deren Verwendung**
Extraction method for the fractioned preparation and separation of plant ingredients and use thereof
Procédés d'extraction pour la production fractionné et la séparation de composés d'origine végétale et leur utilisation

(43) Veröffentlichungstag der Anmeldung: 14.05.2008
(73) Patentinhaber: Nookandeh-Baumgärtner, Aslieh, Dr., 66111 Saarbrücken (DE)
(72) Erfinder: Nookandeh-Baumgärtner, Aslieh, Dr., 66111 Saarbrücken (DE)
(74) Vertreter: Becker Kurig Straus

(56) Entgegenhaltungen:
- EP-A- 1 543 834
- WO-A-01/26647
- DE-A1- 10 240 065
- DE-A1- 10 255 481
- DE-A1- 10 308 864
- DE-A1- 19 846 432
- DE-A1- 19 939 350
- DE-A1-102004 012 830
- DE-A1-102005 035 864
- DE-B1- 2 127 618
- GB-A- 459 635
- JP-A- 9 067 250
- JP-A- 10 025 247
- JP-A- 2002 051 757
- US-A1- 2001 014 365
- US-A1- 2002 051 804
- US-A1- 2005 043 404
- US-A1- 2005 220 914
- US-A1- 2007 009 619
- US-B1- 6 391 346

## Beschreibung

Die vorliegende Erfindung betrifft Extraktionsverfahren zur klassifizierten Gewinnung und Trennung von pflanzlichen Inhaltsstoffen mittels eines mehrstufigen Auswaschungsvorgangs. Insbesondere betrifft die vorliegende Erfindung die Trennung von pflanzlichen Inhaltsstoffen, so beispielsweise zur Gewinnung und Trennung von Hopfeninhaltsstoffen, unter Nutzung der pflanzlichen Eigencellulose als stationäre Phase. Weiterhin betrifft die vorliegende Erfindung die Verwendung von derart gewonnenen Hopfenextrakten bei der Herstellung von Bieren, die mit Phytohormon an- bzw. abgereichert sind, sowie Biere, die mit Xanthohumol angereichert sind.

Die Möglichkeit der Isolierung und Trennung von Naturstoffen pflanzlicher Herkunft basiert einerseits auf der unterschiedlichen chemischen Struktur der Verbindungen. Andererseits wird die Auftrennung von Naturstoffen durch die Natur der Bindung des zu isolierenden Stoffes an das pflanzliche Material, d.h. an die Eigencellulose, beeinflußt. Eine Trennung und Gewinnung definierter pflanzlicher Inhaltstoffe ist für die pharmazeutische und lebensmitteltechnische Industrie, beispielsweise im Rahmen der Entwicklung von Arzneimitteln und Nahrungsmittelzusatzstoffen, von höchstem Interesse.

Naturstoffe pflanzlicher Herkunft enthalten chemische Verbindungen oder Gemische davon, welche für die chemische und pharmazeutische Forschung, beispielsweise für die Entwicklung von Arzneimitteln, von hohem Interesse sind. Zahlreiche Verfahren wurden in den letzen Jahrzehnten entwickelt, um chemische Verbindungen oder Gemische von eng verwandten chemischen Verbindungen aus natürlichem Material zu isolieren und aufgrund der unterschiedlichen Bindungseigenschaften zu charakterisieren. Neben herkömmlichen, destillativen Abtrennverfahren oder Säure-Base-Trennungsgängen wurden in neuerer Zeit insbesondere chromatographische Verfahren eingesetzt und immer weiter verfeinert. Zu den chromatographischen Verfahren, die eine Auftrennung von Substanzen mit hoher chemischer Ähnlichkeit ermöglichen, gehören z.B. die Hochleistungsflüssigkeitschromatographie (HPLC, High-Performance Liquid Chromatography). Als Stationäre Phase dient in diesem Falle ein Gel, das Silikate, Cellulose u.a. enthalten kann. Die Stärke der Bindung an die stationäre Phase (aufgrund unterschiedlicher hydrophiler bzw. lipophiler Bindungskräfte) der zu trennenden Moleküle und die Polarität des Elutionsmittels bestimmen die Trennbarkeit.

Unterschiedliche chromatographische Verfahren sind auf der Basis des oben genannten Grundprinzips in vielen Publikationen beschrieben worden. Beispielhaft seien genannt: die analytische und die präparative HPLC (High-Performance Liquid Chromatography; Schneider, B. et al., Phytochem. Anal. 9, 237-244 (1997); Yu Zhao et al., J. Chromatography A 837, 83-91 (1998)), die Festphasen-Extraktion (Öllers, S. et al., J. Chromatography A 911, 225-234 (2001)), die LC-NMR (Liquid Chromatography-Nuclear Magnetic Resonance Spectroscopy) und die LC-MS (Liquid Chromatography-Mass Spectroscopy; Schütze, W. et al., Agroindustria 3, 399-401 (2004); Wijaya, C. H. et al., J. Agric. Food Chem. 53, 1637-1641 (2005); Ziegert, K. et al., 40. Vortragstagung der DGQ in Karlsruhe, 14.-15.03.2005, Tagungsband 75-78 (2005); Ritzdorf. I. et al., Phytochemistry 50, 995 (1999); Effers, K. et al., Eur. J. Org. Chem. 2793 (1999); Mamer, F.-J. et al., Phytochemistery 60, 301 (2001)), welche die Identifizierung, Isolierung, Reinigung und Strukturaufklärung von Stoffen, die in pflanzlichen Organismen vorkommen, bis hin zur analytischen Identifizierung von Wirkstoffen und Metaboliten im Rahmen von in vitro und in vivo Versuchen (Nookandeh, A. et al., Phytochemistry 65, 561-570 (2004)) ermöglichen. Die soeben genannten chromatographischen Verfahren sind insofern nachteilig, weil diese sehr kostenintensiv, arbeits- und zeitaufwendig sind. Sie benötigen eine ständige Überwachung durch ausgebildete Mitarbeiter und können für die Durchführung mit hoher Präzision nur schwer automatisiert werden. Außerdem ist der Materialverbrauch sehr hoch, da große Mengen eines teuren Lösungsmittels benötigt werden. Darüber hinaus besteht beim Einsatz chromatographischer Verfahren die Gefahr, daß das Säulenmaterial (stationäre Phase) eine Zersetzungsreaktion in den aufzutrennenden Substanzen induziert und somit eine aufzutrennende Substanz häufig mit geringerer Ausbeute eluiert wird.

Eine weitere Gruppe von Trennungsmethoden beruht auf der Elektrophorese. In diesem Verfahren werden nur geladene Partikel im elektrischen Feld aufgetrennt. Die Kapillarelektrophorese erlaubt zum Beispiel eine sehr gute Trennung von geladenen Teilchen in kleinsten Mengen. Durch Anlegen einer Hochspannung (2 - 35 kV ) werden die geladenen Teilchen aufgrund ihrer unterschiedlichen Ladung und Mobilität im elektrischen Feld getrennt (Seidel, B.S. et al., J. Biomed. Opt. 2, 326-331 (1997)).

Die Gelpermeationschromatographie (GPC) und die Molekularsiebchromatographie nutzen die unterschiedlichen Diffusionswege von Molekülen unterschiedlicher Größe durch Poren hindurch in Hohlräume hinein, die durch Vernetzung der Kohlenhydrate entstehen und die festen Bestandteile der Gele bilden. Große Moleküle werden von kleinen dadurch getrennt, daß sie ein kleineres Volumen für ihre räumliche Verteilung vorfinden. Die Gelchromatographie und die Molekularsiebchromatographie beruhen auf dem gleichen Prinzip. Die am häufigsten verwendeten Dextran-Gele werden mikrobiologisch z.B. aus Bakterien gewonnen. Sephadex ist ein Agarose-Gel. Hydrophile stationäre Phasen dienen der Trennung polarer, wasserlöslicher Analyte. Organophile stationäre Phasen eignen sich zur Trennung unpolarer Analyte, die in organischen Lösungsmitteln ( Polystyrol-Gel oder organophiles Dextran-Gel) gelöst sind. Die Molekularsiebchromatographie wird hauptsächlich zur Trennung von Gasen und Flüssigkeiten mit niedriger Molmasse angewandt. Darüber hinaus dient diese Art der Chromatographie bei Anwendung z.B. der Zeolithe zum Trocknen von Gasen und Flüssigkeiten oder zum Entfernen von Ethanol aus Chloroform. Die Vorteile dieser Verfahren bestehen in der Möglichkeit, die Moleküle nach Größe und Form zu trennen und die stationäre Phase wiederholt zu benutzen. Nachteile ergeben sich daraus, daß die gebräuchlichsten Gele bei Druckanwendung sintern und beim Wechsel des Elutionsmittels die Säule neu gepackt werden muß, da die Strömungsgeschwindigkeit wegen der Sinterung oder der zunehmenden Packungsdichte geringer wird.

Im Falle der Papierchromatographie handelt es sich um ein chromatographisches Trennverfahren für kleine Substanzmengen. In diesem Falle dient das Filterpapier als stationäre und das Lösungsmittel oder eine Lösungsmittelmischung als mobile Phase. Eine kleine Menge einer Substanzmischung wird - in gelöster Form - auf ein Blatt Filterpapier aufgebracht. Nach dem Trocknen wird das Filterpapier mit dem unteren Rand in das Lösungsmittel getaucht und zwar so, daß die zuvor aufgebrachte Probe nicht in das Lösungsmittel eintaucht. Bedingt durch die Kapillarwirkung des Filterpapiers steigt das Lösungsmittel langsam nach oben. Dabei wird auch die Substanzmischung von dem nach oben wandernden Lösungsmittel erfaßt. Die in der Mischung enthaltenen Einzelsubstanzen zeigen spezifische Wechselwirkungen (Adsorption und Desorption) mit dem Filterpapier, so daß sich für jede Substanz der Substanzmischung eine spezifische Wanderungsgeschwindigkeit in dem nach oben steigenden Lösungsmittel ergibt. Aufgrund dieser unterschiedlichen Wanderungsgeschwindigkeit erfolgt nach und nach eine Auftrennung der Mischung in ihre Einzelkomponenten. Die Papierchromatographie ist beendet, kurz bevor die Lösungsmittelfront die Oberkante des Filterpapiers erreicht. Bestand die Substanzmischung beispielsweise aus farbigen Komponenten, so finden sich farbige, voneinander getrennte Flecken auf dem Papier. Die chromatographische Trennung pflanzlicher Inhaltsstoffe an der Eigencellulose ist der Verteilungschromatographie, d.h. der Papierchromatographie, sehr ähnlich.

Mit Hilfe extraktiver Trennungsverfahren werden bestimmte Stoffe oder Stoffgemische aus ihrer Bindung an feste Stoffe durch ein Extraktionsmittel herausgelöst. Es ist ein Nachteil, daß die auf diese Weise gewonnenen Extrakte oft nicht erwünschte, begleitende pflanzliche Bestandteile, wie z.B. Fette, Cholorophylle und Wachse, enthalten, welche die Wirkstoffgewinnung sowohl in angereicherter und als auch in reiner Form erschweren.Ein weiterer Nachteil besteht darin, dass derartige Verfahren eine saubere Phasentrennung voraussetzen, welche oftmals jedoch nicht auftritt.

Im Falle der üblicherweise angewandten Extraktionsverfahren wird das pflanzliche Extraktionsgut diskontinuierlich oder kontinuierlich mit dem Lösungsmittel versetzt und das Extraktionsgut nach dem Auswaschen vom die pflanzlichen Inhaltstoffe enthaltenden Lösungsmittel getrennt. Dieser Extraktionsvorgang liefert mehr oder minder die Gesamtheit der ungetrennten pflanzlichen Inhaltstoffe in Lösung. Im Gegensatz zu den üblicherweise verwendeten Extraktionsverfahren erfolgt bei Anwendung des neuartigen, hier beschriebenen Verfahrens der Elutions- bzw. Auswaschprozeß stufenweise mit wechselnden, definierten Lösungsmitteln und/oder Lösungsmittelmischungen unterschiedlicher Zusammensetzung. Die pflanzlichen Inhaltsstoffe werden nacheinander, je nach Stärke ihrer Bindung an die pflanzliche Eigenzellulose, aus dieser spezifischen Bindung und somit aus dem Extraktionsgut herausgelöst. Dabei kann im Falle des hier beschriebenen Verfahrens durch Steuerung der Elutionsprozedur die Trennung der einerseits gewünschten von den andererseits möglicherweise die Auswaschung störenden Inhaltsstoffen dadurch erreicht werden, daß beispielsweise die störenden Inhaltsstoffe mit einem ersten Lösungsmittel oder Lösungsmittelgemisch herausgelöst werden und anschließend die Extraktion des oder der interessierenden Inhaltsstoffe erfolgt. Dieser Vorgang kann auch in der Umkehrung durchgeführt werden.

Mit einigen der vorstehend beschriebenen Verfahren erfolgt die Gewinnung von Hopfenbestandteilen zur Verwendung in der Brauindustrie bereits seit dem Jahr 1890. Unter Hopfen wird im Folgenden die Hopfendolde sowohl im erntefrischen als auch im zurückgetrockneten, handelsüblichen Zustand verstanden. Desweiteren sind Hopfenprodukte, beispielweise Hopfenpulver oder mit Lupulin angereichertes Hopfenpulver in Form von Pellets, Hopfenpreßsaft, Hopfenextrakten oder Hopfenextraktpulvern, zur Gewinnung von Extrakten für die Brauindustrie geeignet. Hopfen enthält Harze, Öle, Wachse, wasserlösliche Verbindungen (insbesondere Zucker, Gerbstoffe und Proteine) und ein festkörperartiges, Gel enthaltendes Grundgerüst, nämlich die Eigencellulose.

Die Vorgehensweise bestand in der Vergangenheit bekannterweise darin, daß die im Rahmen des Bierbrauens unerwünschten Komponenten des Hopfens vollständig entfernt wurden, instabile Komponenten zunächst isoliert und in späteren Schritten des Brauvorganges wieder zugesetzt wurden. Erwünschte Komponenten wurden vor dem Zusetzen veredelt, um z.B. deren Stabilität zu erhöhen oder den Biergeschmack zu verändern bzw. zu verbessern. Inhaltsstoffe, wie z.B. die Bitterstoffe, wurden in konzentrierter Form bereits genutzt. Die Verwendung des Hopfens für die Brauwirtschaft geht auf eine lange Tradition zurück. Schon im Jahre 1890 wurde das Lupulin gemäß der DE-PS 54 812 zunächst aus den Hopfendolden extrahiert und später wieder als wäßriger Extrakt den lupulin-freien Dolden in gewünschter Konzentration zugesetzt. Gemäß der DE-PS 535 841 aus dem Jahre 1928 werden Öle und Harze des Hopfens derart extrahiert, daß in einer Art Umlaufverdampfer in einem geschlossenen System unter Vakuum Wasser und Alkohol, teilweise als Kondensat, durch den Hopfen geführt werden, was jedoch prozeßtechnisch sehr kompliziert und kostenaufwendig ist. Erst die zu Beginn der zwanziger Jahre entwickelten Extraktionsverfahren mit Hilfe von Methylchlorid führten zu einem für die damalige Zeit wirtschaftlichen und technischen Erfolg.

Ethanol als eines der bekanntesten Extraktionsmittel wurde aus den verschiedensten Gründen lange nicht verwendet, obwohl gerade diese Substanz als produktspezifischer Bierbestandteil im Brauereiwesen nicht zu beanstanden wäre. Von großem Nachteil war, daß mit Hilfe der Alkohol-Extraktion kein von Nichtbitterstoffen freier Bitterstoffextrakt erhalten werden konnte. Es ist das Ziel bis zum heutigen Tag, für die Brauindustrie ein standardisiertes Präparat bereitzustellen. Erschwerend ist, daß ein Hopfenextrakt hergestellt wird, der mit Äther oder Alkohol und dann mit einen Alkohol/Wasser-Gemisch mehrstufig extrahiert und in die Bestandteile zerlegt wird, um die auf diese Weise gewonnenen Bestandteile in einer gewünschten Zusammensetzung zu einem neuen Präparat zusammenzumischen.

Ein Verfahren zur Extraktion von gewünschten Komponenten aus natürlichen Hopfenprodukten, bei dem keine Verunreinigungen durch Reste organischer Lösungsmittel auftreten, besteht in der Anwendung eines superkritischen Fluids, z.B. Kohlendioxid, zur Extraktion (DE 21 27 618 A). Weitere Anwendungsbeispiele für dieses Verfahren finden sich in JP 44 864 B (1973), JP 41376 B (1989), US 4,104,409 und US 4,344,978. Nachteilig sind in allen oben genannten Fällen die hohen Kosten und der hohe prozeßtechnische Aufwand. Die verwendeten Apparaturen müßen einem Druck bis zu 400 bar bei einer Extraktionstemperatur von bis zu 100 °C standhalten. Weitere Nachteile sind die großen Mengen das benötigten frischen Kohlendioxids und die Tatsache, daß die bei der CO2-Extraktion gewonnenen Gemische beispielweise noch Chlorophyll, Fette und pflanzliche Hormone, Gerbstoffe usw. enthalten.

Im Patent DE 3103617 A1 aus dem Jahr 1982 wird ein Verfahren zur Gewinnung bitterstoff- und gerbstoffreicher Hopfenextrakte zur Verwendung in der Brauindustrie beschrieben, das 80 bis 96 %-iges Ethanol als Lösungsmittel verwendet, bei dessen Anwendung es aber erforderlich ist, in einem weiteren Verfahrensschritt durch Wasserdampfbehandlung den Gerbstoffanteil abzutrennen. Nachteilig ist der hohe Arbeitsaufwand und Energiebedarf, da eine Vielzahl von Prozeßschritten durchzuführen ist.

In der DE 102 40 065 wird ein Verfahren beschrieben, in dem aus Hopfen eine unpolare, in Wasser unlösliche Fraktion, nämlich den Ethanol-Reinharzextrakt, als CO₂-Extraktionsrückstand gewonnen wird. Anschließend wird Xanthohumol nach dem Auswaschen der löslichen Anteile als unlöslicher Rückstand erhalten. Es wird somit eine Phasentrennung durchgeführt, wobei einer polare und eine unpolare Fraktion entsteht. Das in der unpolaren Fraktion vorhandene Xanthohumol wird durch eine nachfolgende Extraktion von den mehr oder weniger polaren Begleitstoffe mit Hilfe von Alkohol/Wasser-Gemischen befreit, ohne vollständig rein zu sein.

In der DE 199 39 350 wird ein Verfahren zur Herstellung eines Hopfenextraktes beschrieben. Hierbei wird Hopfen oder Doldenhopfen als Ausgangsmaterial verwendet, der/die in alkalisiertem Wasser eine Stunde bei 95°C behandelt wird/werden. Der daraus gewonnene Dünnextrakt wird dann an Absorber-Harze gebunden und die pflanzlichen Stoffe mittels Flüssig-Flüssig-Extraktion unter Einsatz organischer Lösungsmittel aus dem Absorber-Harz zurück gewonnen. In Dünnextrakt sind Flavonoide, wie beispielsweise das Xanthohumol, nur sehr schwer löslich. Xanthohumol ist daher nicht an Absorber-Harze gebunden sondern im Rückstand angereichert, aus dem durch Extraktion bei 50°C bis 70°C Xanthohumol-reiche Lösungen gewonnen werden.

In der GB 459 632 wird die Durchführung einer Zwei-Stufenextraktion beschrieben, zuerst mit Hilfe von organischen, unpolaren Lösungsmitteln, z.B. mit Hilfe von Ethylether oder Methylendichlorid, zum Entzug von Hopfen-Ölen und Tannin und anschließend mit Hilfe von organischen, polaren Lösungsmitteln zur Extraktion von Tannin.

In der DE 10 2004 012 830 wird ein zweistufiges Extraktionsverfahren unter Einsatz von verdichtetem Kohlendioxid und unter Zusatz eines Cosolvens zur Extraktion von Prenylchalkonen und Prenylflavonen offenbart. Das auf diese Weise an diesen beiden Stoffen angereicherten Extrakte fassen die jeweilige Stoffklasse weiterhin ungetrennt als Gemisch zusammen. Das Extraktionsverfahren setzt Temperaturen bis zu 80°C, Drücke von bis zu 500 bar und Extraktionszeiten von bis zu 5 Stunden voraus.

In der DE 21 27 618 wird ein zweistufiges Extraktionsverfahren beschrieben, wobei Hopfenmaterial zuerst mit CO₂ unter hohem Druck und bei hoher Temperatur extrahiert wird. Das zurückbleibend extrahierte Hopfenmaterial wird in der zweiten Stufe ebenfalls bei höheren Temperaturen mit Wasser nochmals extrahiert und der Rückstand (die Eigenzellulose) abfiltriert. Das nach dem Filtrieren durch Einengen erhaltene Pulver wird anschließend wieder mit dem CO₂-Extrakt (1. Stufe) zusammengeführt und in verschiedenen Anteilen gemischt.

In der EP 1 543 834 wird ein Extraktionsverfahren offenbart, das mit flüssigen oder überkritischem CO₂ durchgeführt wird.

Alle oben genannten Extraktionsverfahren haben den Nachteil, daß bei der Extraktion von Hopfen (Dolden), sei es mit überkritischem Kohlendioxid oder mit Gemischen aus Wasser und organischen Lösungsmitteln, große Mengen an Rückständen verbleiben und die gewonnenen Hopfenextrakte in der Regel eine hohe Viskosität (honigartig) aufweisen. Außerdem enthalten die so gewonnenen Extrakte noch Chlorophylle, Fette usw.. Es stehen somit sehr viele verschiedenartige Verfahren zur Verfügung, mit deren Hilfe aus dem Hopfen die gewünschten Komponenten unter konstanten Bedingungen abgetrennt werden können. Diese Verfahren sind jedoch aus wirtschaftlichen und betriebstechnischen Gründen nachteilig, da die erforderlichen Einrichtungen teuer und kompliziert sowie die Verfahrenskontrolle aufwendig ist.

Daher besteht ein Bedarf nach alternativen Verfahren zur Trennung von pflanzlichen Inhaltsstoffen, welche die vorstehend genannten Nachteile der chromatographischen und extraktiven Trennungsverfahren des Stands der Technik überwinden. Ein derartiges Verfahren sollte lediglich eine vergleichsweise moderate Menge an Lösungsmitteln, insbesondere an schwer abbaubaren organischen Lösungsmitteln, benötigen und den Vorteil aufweisen, dass es zumindest in Teilbereichen, vergleichsweise einfach automatisiert werden kann. Darüber hinaus sollte ein Trennungsverfahren, wie es von der vorliegenden Erfindung angestrebt wird, eine gezielte Anreicherung oder Isolierung von bestimmten pflanzlichen Inhaltsstoffen aus einem Naturstoffgemisch in technischem Maßstab in einfacher und wirtschaftlicher Weise ermöglichen.

Die vorliegende Erfindung löst diese Aufgabe durch die Bereitstellung eines Extraktionsverfahren zur klassifizierten Gewinnung und Trennung pflanzlicher Inhaltsstoffe mittels mehrerer Auswaschungsvorgänge, welches die Schritte umfasst: Zerkleinern der die pflanzlichen und/oder tierischen Inhaltsstoffe umfassenden pflanzlichen Ausgangsmaterialien und/oder Bereitstellen der pflanzlichen Ausgangsmaterialien in fluider Form, Befüllen eines Extraktionsbehälters mit den zerkleinerten pflanzlichen Ausgangsmaterialien und/oder mit den pflanzlichen Ausgangsmaterialien in fluider Form, wobei der Extraktionsbehälter mindestens einen Zufluss und mindestens einen Abfluss aufweist, wobei vor dem mindestens einen Abfluss ausschließlich eine oder mehrere Rückhaltevorrichtungen für das zerkleinerte Material angeordnet sind, Durchführen eines ersten kontinuierlichen Auswaschungsvorgangs mit einem ersten Lösungsmittel oder Lösungsmittelgemisch, Durchführen eines zweiten kontinuierlichen Auswaschungsvorgangs mit einem zweiten Lösungsmittel oder Lösungsmittelgemisch, wobei sich das zweite Lösungsmittel oder Lösungsmittelgemisch von dem ersten Lösungsmittel oder Lösungsmittelgemisch unterscheidet, optional Durchführen weiterer kontinuierlicher Auswaschungsvorgänge mit jeweils einem weiteren Lösungsmittel oder Lösungsmittelgemisch, wobei sich jedes weitere Lösungsmittel oder Lösungsmittelgemisch von jedem zuvor verwendeten Lösungsmittel oder Lösungsmittelgemisch unterscheidet.

Die vorliegende Erfindung kombiniert die bei der Papierchromatographie (Verteilungschromatographie) auftretenden Prozesse mit den Vorgängen, die im Falle der Extraktionsverfahren maßgeblich sind. Die Erfindung betrifft also ein Auswaschverfahren mittels einer mehrstufigen Elution zur Ablösung von pflanzlichen Inhaltsstoffen aus der Bindung der auszuwaschenden Moleküle an die pflanzliche Eigenzellulose als stationäre Phase. Zur Mobilisierung der pflanzlichen Inhaltsstoffe aus ihrer Bindung an die pflanzliche Eigenzellulose wird ein Wasser/Ethanol-Gemisch in Stufen, nicht aber als Gradient, mit dem Ziel der Gewinnung von Extrakten mit angereichertem Wirkstoff bis hin zu reinen Wirkstoffen im Technikum-Maßstab eingesetzt, um diese für deren Verwendung in der Brauwirtschaft, in der Pharmazie, Biotechnologie, Medizin, Lebensmittelchemie sowie Lebensmitteltechnologie und in der Kosmetik bereitzustellen.

Die vorliegende Erfindung beruht im Wesentlichen in der Erkenntnis, daß eine Extraktion als wichtigste Komponente die pflanzliche Eigencellulose als feste Phase bzw. Matrix enthält.

Die Eigencellulose ist hierbei unabdingbarer; integraler Bestandteil und ist zur Auswaschung (Elution) reiner, pflanzlicher Inhaltsstoffe befähigt. Die unterschiedlichen Bindungskräfte, mit der die pflanzlichen Inhaltsstoffe an die Eigencellulose gebunden sind, erlauben bei Änderung der Polarität des Elutionsmittels die schrittweise Auswaschung der pflanzlichen Inhaltsstoffe. Die spezifischen Wechselwirkungen zwischen der Eigencellulose und den Inhaltsstoffen werden genutzt, um in einer Folge von möglichst wenigen Prozeßschritten und auf möglichst schonende Weise mit hoher Ausbeute und einfachem, apparativem Aufwand - und somit preisgünstig - Bestandteile der Pflanzen (Pflanzenprodukte), entweder als Stoffgruppen oder in reiner Form, für die Verwendung in der Nahrungsmittelindustrie und in anderen Industriezweigen zu gewinnen.

Weiterhin stellt die vorliegende Erfindung, ein Extraktionsverfahren zur klassifizierten Gewinnung und Trennung von Hopfenmaterial mittels mehrerer Auswaschungsvorgänge bereit, welches die folgenden Schritte aufweist: Zerkleinern des Hopfenmaterials und/oder Bereitstellen des Hopfenmaterials in fluider Form, Befüllen eines Extraktionsbehälters mit dem zerkleinerten Hopfenmaterial und/oder mit dem Hopfenmaterial in fluider Form, Durchführen eines ersten kontinuierlichen Auswaschungsvorgangs mit einem ersten Lösungsmittel oder Lösungsmittelgemisch, Durchführen eines zweiten kontinuierlichen Auswaschvorgangs mit einem zweiten Lösungsmittel oder Lösungsmittelgemisch, wobei sich das zweite Lösungsmittel oder Lösungsmittelgemisch von dem ersten Lösungsmittel oder Lösungsmittelgemisch unterscheidet, optional Durchführen weiterer kontinuierlicher Auswaschvorgänge mit jeweils einem weiteren Lösungsmittel oder Lösungsmittelgemisch, wobei sich jedes weitere Lösungsmittel oder Lösungsmittelgemisch von jedem zuvor verwendeten Lösungsmittel oder Lösungsmittelgemisch unterscheidet.

Das erfindungsgemäße Extraktionsverfahren eignet sich für eine Extraktion verschiedener pflanzlicher Ausgangsmaterialien, die pflanzliche Inhaltsstoffe von Interesse umfassen, beispielsweise für pflanzliche Rohmaterialien beziehungsweise halbtechnische oder technische Produkte aus pflanzlichen Materialien und ebenso für Abfallmaterialien aus Produktionsprozessen, die pflanzlichen Ursprungs sind. (Alle derartige Ausgangsmaterialien oder Gemische hiervon werden nachstehend auch als Extraktionsgut bezeichnet.) Das erfindungsgemäße Verfahren bietet sich in vorteilhafter Weise für eine Trennung von empfindlichen chemischen Verbindungen oder Verbindungsgemischen an, die sich in Gegenwart von gängigen Säulenfüllmaterialien leicht zersetzen oder bei chromatographischen Verfahren nur mit unzufrieden stellender Ausbeute eluiert werden.

In den Abbildungen zeigt
Fig. 1: eine schematische Ansicht eines Extraktionsverfahrens gemäß der vorliegenden Erfindung, das auf einer Produktionsanlage gemäß des Stands der Technik mit beispielsweise den Komponenten A - G beruht. Durch eine geeignete Nacheinanderschaltung der Komponenten wird eine schrittweise Elution reiner Stoffe mit dem Ziel der Trennung und Gewinnung derselben gewährleistet. Die wichtigste Komponente ist der Eluator (B), der die Eigencellulose (B1) als stationäre Phase und zugleich als pflanzlichen Rohstoff für die Gewinnung der Inhaltsstoffe enthält.
Fig. 2 a: ein Polysaccharid, das aus 1,4-verknüpften Glucose-Molekülen aufgebaut ist; und b: eine Darstellung der Wasserstoff-Brücken im Polysaccharid.
Fig. 3: ein Modell der pflanzlichen Primärzellwand (verändert nach Markus Pauly, Max Planck Institut für Molekulare Pflanzenphysiologie).

Die vorliegende Erfindung stellt ein chromatographisches Elutionsverfahren zur klassifizierten Gewinnung und Trennung von pflanzlichen Inhaltsstoffen mittels mehrerer Auswaschungsvorgänge bereit. Das Verfahren umfasst hierbei die Schritte von Zerkleinern der die pflanzlichen Inhaltsstoffe umfassenden pflanzlichen Ausgangsmaterialien und/oder Bereitstellen der pflanzlichen Ausgangsmaterialien in fluider Form; Befüllen eines Elutionsbehälters mit den zerkleinerten pflanzlichen Ausgangsmaterialien und/oder mit den pflanzlichen Ausgangsmaterialien in fluider Form, wobei der Elutionsbehälter mindestens einen Zufluss und mindestens einen Abfluss aufweist, wobei vor dem mindestens einen Abfluss ausschließlich eine oder mehrere Rückhaltevorrichtungen für das zerkleinerte Material angeordnet sind; Durchführen eines "ersten" kontinuierlichen Auswaschungsvorgangs mit einem ersten Lösungsmittel oder Lösungsmittelgemisch; Durchführen eines zweiten kontinuierlichen Auswaschungsvorgangs mit einem zweiten Lösungsmittel oder Lösungsmittelgemisch, wobei sich das zweite Lösungsmittel oder Lösungsmittelgemisch von dem ersten Lösungsmittel oder Lösungsmittelgemisch unterscheidet; optional Durchführen weiterer kontinuierlicher Auswaschungsvorgänge mit jeweils einem weiteren Lösungsmittel oder Lösungsmittelgemisch, wobei sich jedes weitere Lösungsmittel oder Lösungsmittelgemisch von jedem zuvor verwendeten Lösungsmittel oder Lösungsmittelgemisch unterscheidet, wobei die Elution unter Verwendung der pflanzlichen Eigencellulose der pflanzlichen Ausgangsmaterialien als feste/stationäre Phase durchgeführt wird und wobei die Auswaschungsvorgänge bei Raumtemperatur (20-24°C) und Normaldruck (950-1060 mbar) durchgeführt werden.

Der Begriff "Extraktionsverfahren zur klassifizierten Gewinnung und Trennung von Inhaltsstoffen" umfasst, dass durch das erfindungsgemäße Verfahren eine reproduzierbare Auftrennung der Inhaltstoffe des eingesetzten Ausgangsmaterials bereitgestellt wird und eine Auftrennung zu einem bestimmten Verbindungsgemisch, das heißt zu einer bestimmten Klasse von Verbindungen mit im Wesentlichen einheitlichen chemischen Eigenschaften, insbesondere mit einem im Wesentlichen einheitlichen Lösungsverhalten, erfolgt oder eine Auftrennung zu einer bestimmten Verbindung in reiner oder im Wesentlichen reiner Form erfolgt. Der Begriff "Verbindung in im Wesentlichen reiner Form" umfasst, dass das Gewicht dieser Verbindung mindestens 60 Gew.-%, vorzugsweise mindestens 90 %, bevorzugt mindestens 95 % des Gesamtgewichts der betrachteten (gegebenenfalls weiter aufgereinigten) Fraktion, bezogen auf das Trockengewicht der betrachteten Fraktion und Verbindungssubstanz, ausmacht.

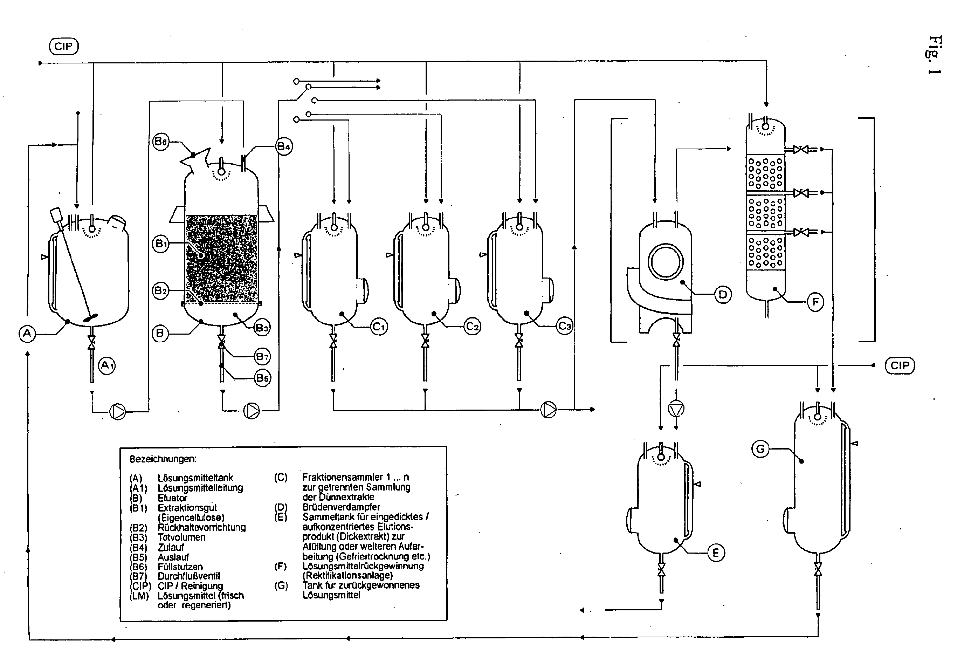

Das erfindungsgemäße Extraktionsverfahren beruht hierbei auf einer einfachen Produktionsanlage mit den Komponenten A - G. Durch eine geeignete Nacheinanderschaltung der Komponenten (siehe Fig. 1) wird eine schrittweise Elution reiner Stoffe mit dem Ziel der Trennung und Gewinnung derselben gewährleistet. Die wichtigste Komponente ist der Eluator (B), der die Eigencellulose (B1) als stationäre Phase und zugleich als pflanzlichen Rohstoff für die Gewinnung der Inhaltsstoffe enthält. Die pflanzlichen Inhaltsstoffe sind an die Eigencellulose des Pflanzenmaterials mehr oder weniger stark gebunden.

Nach erfolgter Elution kann die Eigencellulose sogar in einer anderen Nutzungsart, sozusagen als Chromatographiematerial, nochmals z.B. für die Auftrennung von pflanzlichen Stoffen, die in ihrem natürlichen Zustand nicht an eben diese Eigencellulose gebunden sind, mit gutem Erfolg verwendet werden. So kann z.B. Eigencellulose des Hopfens zur Trennung und zur Reinigung von Naturstoffen aus Apfelsaft eingesetzt werden. Dieses Beispiel steht für die Auswaschung von pflanzlichen Inhaltsstoffen aus beliebigen pflanzlichen Rohmaterialien unter Einsatz der Eigencellulose des Hopfens.

Die Elution erfolgt mittels eines oder mehrerer Auswaschungsvorgänge unter dem sukzessiven, stufenweisen Einsatz - nicht aber als Gradient - von Wasser, einer oder mehrerer Wasser/Alkohol-Mischungen und reinem Alkohol als mobile Phase. Die einzelnen nacheinander ablaufenden Prozeßschritte sind wie folgt zu beschreiben: 1. Zerkleinern (Mahlen, Pulverisieren, Häckseln) des getrockneten pflanzlichen Materials, das die Eigencellulose als Hauptbestandteil enthält. 2. Befüllen des Eluators (B) durch den Füllstutzen (B6) hindurch mit den pulverisierten, pflanzlichen (feuchten oder trockenen) Ausgangsmaterialien, wobei der Eluator mindestens einen Zufluß (B4) und mindestens einen Abfluß (B5) mit Regulierventil (B7) aufweist. Mindestens vor einem Abfluß sind eine oder mehrere Rückhaltevorrichtungen (Sieb- oder Filterplatten, B2), auf denen das zerkleinerte Material gepackt ist, angeordnet. 3. Quellung und Spülung unter Einsatz von Trinkwasser, wobei die pflanzliche Eigencellulose Wasser bindet und infolgedessen quillt. Als Resultat dieser "Wässerung" erhält man die konditionierte Eigencellulose, die nun im Rahmen der weiteren Durchführung des Verfahrens als stationäre Phase wirksam ist und eine chromatographische oder chromatographie-ähnliche Trennung der pflanzlichen Inhaltsstoffe im Zusammenspiel mit geeigneten Elutionslösungen bzw. Lösungsmittelmischungen ermöglicht. Das bei der Konditionierung der Eigencellulose gewonnene Spülwasser enthält Verunreinigungen (Bakterien, Hefen und Schadstoffe) und wird daher verworfen. 4. Danach wird im fünften Arbeitsschritt nochmals Wasser, jetzt zur Elution, durch das auszuwaschende Material (Extraktionsgut) hindurchgeleitet und zwar so lange, bis die wasserlöslichen Bestandteile vollständig herausgelöst sind und sich im Eluat (Fraktion 1) befinden. Die so gewonnene Fraktion I wird über eine Leitung (B5) in den Sammelbehälter (C1) gepumpt und dort gesammelt. 5. In den folgenden Arbeitsschritten der stufenweisen Elution werden Auswaschmittel im Lösungsmitteltank A hergestellt und über das im Behälter B befindliche Extraktionsgut geleitet. Durch Umschalten auf unterschiedliche Mischungsverhältnisse Wasser : Alkohol werden sukzessiv die verschiedenen Pflanzeninhaltsstoffe rein oder in Klassen bzw. Gruppen eluiert und in den entsprechenden Sammelbehältern C2,3,..n gesammelt. Jeder Elutionsschritt bzw. jede Elutionsstufe wird so lange fortgeführt, bis die jeweilige Klasse von Inhaltsstoffen vollständig aus der Eigencellulose herausgewaschen ist. Erst danach beginnt die nächste Stufe des Auswaschprozesses.

Sämtliche Auswaschschritte werden unter Normalbedingungen durchgeführt, d.h. bei Raumtemperatur und unter Normaldruck, wobei Raumtemperatur ein Temperatur zwischen 20 und 24°C, bevorzugt 22°C, entspricht und der Begriff Normaldruck einen Druck von 950-1060 mbar, vorzugsweise 980-1030 mbar umfasst. Als Elutionsmittel kommen organische Lösungsmittel infrage, die mit Wasser mischbar sind, insbesondere solche, die in jedem Verhältnis mit Wasser mischbar sind. Deshalb werden Alkohole (wie z.B. Methanol, Ethanol, Isopropanol, n-Propanol usw.) und Ketone (wie z.B. Aceton usw.) vorzugsweise verwendet. Bevorzugt wird jedoch Ethanol eingesetzt, das aus nahrungsmitteltechnologischen Gründen am unbedenklichsten ist. Der Unterschied der nacheinander folgenden Elutionsschritte besteht in der stufenweisen Abnahme der Polarität durch Steigerung des Anteils an organischen Lösungsmitteln im Gemisch mit Wasser. Die prozentuale Zusammensetzung der Lösungsmittelmischungen wird so optimiert, daß für die Elution der verschiedenen Gruppen von Pflanzeninhaltsstoffen eine am besten geeignete Lösungsmittelmischung bereitsteht, um die reinen Pflanzeninhaltsstoffe in optimaler Weise zu trennen. Durch eine entsprechende Abfolge der Lösungsmittelmischungen lassen sich so nacheinander alle interessierenden Pflanzeninhaltsstoffe gewinnen und separat in den Behältern C2,3,..,n auffangen. Nach der in dieser Weise durchgeführten Auswaschung und Ablösung von der Eigencellulose sind die gewonnenen Pflanzeninhaltsstoffe so rein, daß nachfolgende Reinigungsverfahren, wie z.B. chromatographische Trennmethoden usw., vereinfacht werden können oder aber gänzlich entfallen.

Das Prinzip der Trennung unter Verwendung der Eigencellulose ist praktisch auf alle bekannten pflanzlichen Rohmaterialien anwendbar. Die Lösungsmittelmischungen müssen entsprechend den nacheinander zu gewinnenden Inhaltsstoffen hinsichtlich ihrer Volumenanteile der Komponenten an das spezielle Extraktionsgut angepaßt werden. Die Volumenverhältnisse der beiden Lösungsmittelkomponenten varieren zwischen 0 % Wasser und 100 % reinem Alkohol bzw. organischem Lösungsmittel. Die Temperatur kann zwischen 0 °C und 100 °C, der Druck zwischen 10 mbar und 800 mbar varieren. Die physikalischen Bedingungen werden gleichfalls von Fall zu Fall für spezifische Anwendungen modifiziert und optimiert.

Enthält das auszuwaschende, pflanzliche Rohmaterial ätherische Öle oder andere besonders wertvolle flüchtige Stoffe, so bietet es sich darüber hinaus an, vor der Spülung und Quellung der Eigencellulose und vor der Auswaschung der pflanzlichen Inhaltsstoffe so lange Wasserdampf hindurchzuleiten, bis die leicht flüchtigen Komponenten ganz oder teilweise entfernt sind. In diesem Falle ist es erforderlich, die Elutionsanlage mit einem Kondensator zu verbinden, zweckmäßigerweise dem Eluator unmittelbar nachgeschaltet, damit der mit den ätherischen Ölen oder sonstigen leicht flüchtigen Stoffen beladene Wasserdampf kondensiert und die leicht flüchtigen Komponenten durch Phasentrennung gewonnen werden können. In diesem Zusammenhang ist die Möglichkeit gegeben, den Wasserdampf sowohl von oben nach unten als auch von unten nach oben durch den Eluator zu leiten; in diesen Fällen muß eine thermische Isolierung des Eluators bzw. die Möglichkeit zur Beheizung des Eluators gegeben sein, damit es während der Dampfbehandlung zu keinen Kondensationen der Duftstoffe im Eluator kommt.

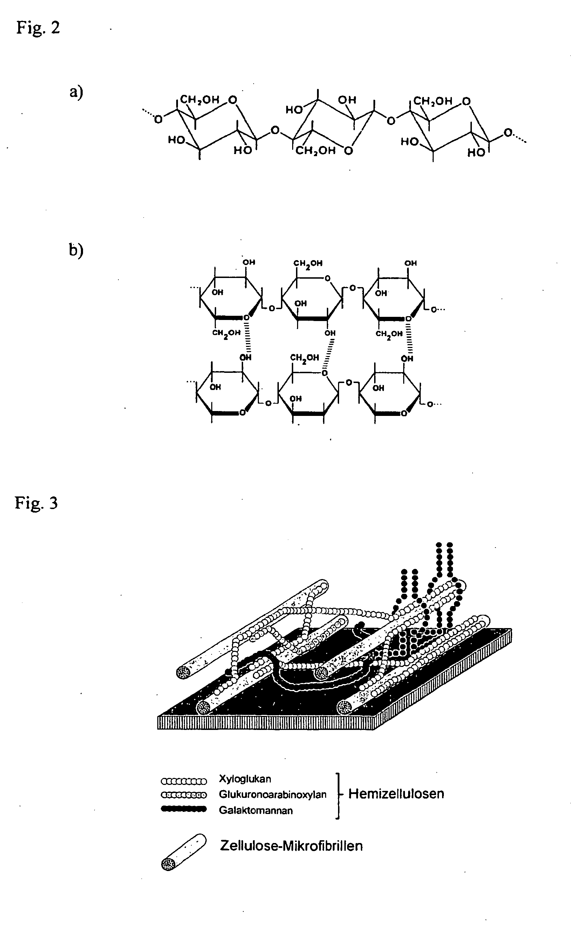

Eigencellulose kommt in allen Pflanzen als Hauptbestandteil der Zellwände vor. Sie ist die Gerüstsubstanz der Pflanzen. Chemisch gesehen ist die Eigencellulose ein unverzweigtes Polysaccharid, das aus Beta-Glucose-Molekülen kettenförmig aufgebaut ist (siehe Fig. 2). Diese Ketten bestehen aus mehr als 10.000 Glucose-Molekülen. Zwischen parallel verlaufenden Ketten bilden sich Wasserstoff-Brücken aus, so daß drei-dimensionale, geordnete, kristalline Strukturen entstehen. In Pflanzen wechseln jedoch kristalline mit nichtkristallinen, sog. parakristallinen Bereichen ab, wobei sich sogenannte Micellen bilden, in denen die einzelnen Cellulose-Ketten ungeordnet, nicht parallel verlaufen, wodurch die Wasserstoff-Brückenbildung erschwert wird. Aufgrund ihrer hochmolekularen Struktur ist die Eigencellulose in Wasser und in den meisten organischen Lösungsmitteln unlöslich.

Die spezifischen Eigenschaften der Eigencellulose, besonders im Hinblick auf ihre überraschende Fähigkeit, Stoffe zu binden, beruhen auf ihrer besonderen Struktur in der pflanzlichen Zellwand. Die Zellwand besteht aus mehreren Schichten der Cellulose, die sich in ihrer Orientierung unterscheiden können und in die teilweise weitere stabilisierende Substanzen, wie z.B. Lignin, eingelagert sind (siehe Fig. 3).

Molekulare Hauptbestandteile einer jeden Zellwand sind die Polysaccharide. In der Zellwand läßt sich eine kristalline, faserige Komponente, die sogenanten Cellulose-Mikrofibrillen, die eine wäßrige, gel-artige Matrix bilden, nachweisen. Diese gel-artige Matrix besteht hauptsächlich aus Hemicellulosen und Pektinen. Das Netzwerk aus verschiedenen Hemicellulosen hat eine hohe Bindungsaffinität zur Zellulose und bildet daher ein zugfestes Cellulose-/Hemicellulose-Netzwerk. Wahrscheinlich ist dieses Netzwerk für die Stabilität der Zellwand verantwortlich. Pektine bilden ein weiteres Netzwerk und bestehen aus strukturell mannigfaltigen Einzelbausteinen (siehe Fig. 3), die sich alle durch das Vorkommen eines negativ geladenen Zuckers, der Galakturonsäure, auszeichnen. Das Pektin-Netzwerk bestimmt die Porosität der Zellwand und daher die Art und Größe der Stoffe, welche die Zellwand passieren können. Weiterhin ist das Pektin-Netzwerk für die Bindung von Ionen und geladenen Molekülen, aber auch für die Stabilität der Zellwand wichtig. Neben Polysacchariden sind in einigen Zellen auch Glykoproteine Bestandteile der Zellwand. Zusätzlich wird in der Sekundärzellwand (sie entsteht beim Wachstum der Pflanzen aus der Primärzellwand) eine große Menge einer Polyphenyl-Substanz, das sogenannte Lignin, eingelagert. Lignin verleiht der Zellwand Härte und wasserabstoßende Eigenschaften. Das von der Zellwand umschlossene Volumen wird praktisch vollständig durch den Protoplasten (Pflanzenzelle) ausgefüllt. Der Protoplast ist der von der Zellmembran umschlossene, im Gegensatz zur toten Zellwand lebende Teil der pflanzlichen Zelle.

Wird frisches oder getrocknetes pflanzliches Gewebe mit Wasser oder organischen Lösungsmitteln behandelt, so wird spätestens dann der Protoplast zerstört. Die in den Protoplasten gebildeten pflanzlichen Inhaltsstoffe werden mehr oder minder vollständig in Lösung gebracht, während die Zellwände, die einen das pflanzliche Gewebe stützenden Verband bilden, nicht in Lösung gehen.

Die industriell verwertete Cellulose wird aus Naturstoffen gewonnen. Für bestimmte Anwendungen, z.B. als feste Phase in der Chromatographie, werden die OH-Gruppen substituiert, z.B. durch Bildung von Sulfonaten, um neuartige Polymere zur Verfügung zu stellen ("New Polymers from Cellulose Sulfonate". Heinze, T. et al., J. Pulp & Paper Science 25, 136-140 (1999)).

Das gewählte und zerkleinerte Extraktionsgut kann entweder direkt verwendet werden oder aber zusammen mit geeigneten Träger- oder Auflockerungsmaterialien, wie Sand, Kieselgur, Zellulose, oder anderen geeigneten und gegenüber den zu verwendenden Lösungsmitteln hinreichend beständigen Materialien eingesetzt werden. Der geeignete Zerkleinerungsgrad beziehungsweise die Erfordernis zur Verwendung von Träger- beziehungsweise Auflockerungsmaterialien ist abhängig von dem Extraktionsgut und kann gemäß dem allgemeinen Fachwissen eines Fachmanns durchgeführt werden. Beispielsweise kann dem Ausgangsmaterial zwischen 1 und 90 Gew.-%, vorzugsweise zwischen 5 und 30 Gew.-% an Träger- beziehungsweise Auflockerungsmaterial, jeweils bezogen auf die Trockensubstanz zugesetzt werden.

Das derart vorbereitete Material wird dann in einen geeigneten Extraktionsbehälter eingefüllt. Ein derartiger Extraktionsbehälter kann mindestens einen Zu- und Ablauf aufweisen, wobei vor dem mindestens einen Ablauf ausschließlich eine oder mehrere Rückhaltevorrichtungen für das zerkleinerte Material und/oder das Trägermaterial angeordnet sein können. Eine derartige Rückhaltevorrichtung kann beispielsweise ein Sieb mit Poren, insbesondere ein Sieb mit Pore 3, sein. Alternativ kann beispielsweise ein Wattepfropfen vor dem Ausfluss angebracht werden, ein Büchner-Trichter gegebenenfalls in Kombination mit Filterpapier eingesetzt werden oder es kann eine andere geeignete Rückhaltevorrichtung gemäß dem allgemeinen Wissen eines Fachmanns gewählt werden. Eine derartige Rückhaltevorrichtung führt in bevorzugter Weise zu keiner oder im Wesentlichen keiner Auftrennung und/oder Retention der im Extrakt enthaltenen, gelösten Inhaltsstoffe. Die Zusammensetzung des Extrakts nach Durchgang durch die Rückhaltevorrichtung sollte im Wesentlichen gleich der Zusammensetzung vor Durchgang durch die Rückhaltevorrichtung sein. Dies kann beispielsweise dadurch festgestellt werden, indem bestimmt wird, dass der Gehalt an den drei mengenmäßigen Hauptkomponenten des Extrakts nach Durchgang durch die Rückhaltevorrichtung um nicht mehr als jeweils ± 5% von dem Gehalt vor Durchgang abweicht.

So kann insbesondere der Extraktionsbehälter in Fließrichtung des Extrakts unterhalb der das Ausgangsmaterial in zerkleinerter und/oder fluider Form umfassenden Schicht frei von einer Schicht aus zugegebenen Material mit Auftrennungseigenschaften vor der Rückhaltevorrichtung und/oder dem Ausfluss sein. Der Begriff "zugegebenes Material mit Auftrennungseigenschaften" umfasst in der vorliegenden Anmeldung insbesondere dem Fachmann bekannte Substanzen für eine chromatographische Auftrennung, wie beispielsweise Kieselgele oder Aluminiumoxide, und/oder mit chemischen Verbindungen versehene Feststoffmaterialien, beispielsweise Ionenaustauscherkügelchen. Derartige Materialien mit fluftrennungseigenschaften werden, wie es dem Fachmann bekannt ist, Gefäßen und Behältern zugegeben. Insbesondere sei angemerkt, dass von dem Begriff "zugegebenes Material mit Auftrennungseigenschaften" einstückig mit dem Extraktionsbehälter verbundene Einrichtungen, beispielsweise Platten oder so genannte Fritten, beispielsweise aus Keramik- oder Glasmaterial, nicht umfasst sein sollen.

Die Extraktion erfolgt dann durch Hindurchleitung von Lösungsmitteln oder Lösungsmittelgemischen aus zwei oder mehr Lösungsmitteln, deren Zusammensetzung variiert werden kann, wobei die Änderung des Lösungsmittels oder Lösungsmittelgemisches davon abhängt, welcher Inhaltsstoff oder welche Mischung von Inhaltsstoffen aus dem Extraktionsgut extrahiert werden soll.

Als Lösungsmittel kann jedes bekannte Lösungsmittel verwendet werden, wobei das Lösungsmittel vorzugsweise ein für eine Extraktion von Lebensmitteln geeignetes Lösungsmittel ist, insbesondere falls die Substanz(en) des Extrakts für den menschlichen Verzehr vorgesehen sind. Falls erwünscht, kann darüber hinaus vor der Extraktion das Lösungsmittel oder Lösungsmittelgemisch mit einer Säure oder Base auf einen bestimmten pH-Wert eingestellt werden. Die Lösungsmittel oder Lösungsmittelgemische der aufeinanderfolgenden Auswaschungsvorgänge können eine steigende, eine fallende oder eine im Wesentlichen konstante Polarität und/oder einen steigenden, eine fallenden oder einen im Wesentlichen konstanten pH-Wert aufweisen.

So kann beispielsweise bei dem ersten, zweiten und optional dritten, vierten, fünften oder weiteren Auswaschvorgang eine Auswaschung mit mindestens einem Lösungsmittel oder Lösungsmittelgemisch erfolgen, das ausgewählt ist, aus der Gruppe bestehend aus Wasser, Gemisch bestehend aus Wasser und Ethanol, Gemisch umfassend mindestens 70 Gew.-%, vorzugsweise mindestens 80 Gew.-%, an Wasser und/oder Ethanol, und Ethanol.

Im Gegensatz zu gängigen Extraktionsverfahren wird bei dem erfindungsgemäßen Extraktionsverfahren also nicht das Extraktionsgut in einem Lösungsmittel oder Lösungsmittelgemisch aufgeschwemmt, wobei die Aufschwemmung im Anschluss filtriert wird, um das Lösungsmittel mitsamt den extrahierten Stoffen vom verbleibenden Rest abzutrennen, sondern es werden in einem permanenten oder kontinuierlichen Auswaschungsvorgangs mit wechselnden Lösungsmitteln und/oder Lösungsmittelgemischen verschiedener Zusammensetzung aus dem Extraktionsgut nach und nach die Inhaltskomponenten herausgelöst. Dabei kann das Verfahren in Abhängigkeit von einerseits gewünschten und andererseits unerwünschten oder die Extraktion potentiell störenden Inhaltsstoffen so gesteuert werden, dass beispielsweise die unerwünschten und/oder störenden Inhaltsstoffe mit einem ersten Lösungsmittel oder Lösungsmittelgemisch herausgelöst werden, bevor die Extraktion des oder der eigentlich interessierenden Inhaltsstoffe beginnt. Umgekehrt kann die Extraktion derart gesteuert werden, dass die unerwünschte Substanz(en) bis zur Beendigung der Gewinnung der Fraktion von Interesse in dem Extraktionsgut verbleiben. Der Begriff "kontinuierlicher Auswaschvorgang" umfasst, dass während eines Auswaschvorgangs der Extraktabfluss vorzugsweise nicht unterbrochen wird und ein ständiger kontinuierlicher Extraktabfluss erfolgt. Gegebenenfalls auftretende Unterbrechungen sollten eine Zeitdauer von 10 Minuten, vorzugsweise 1 min nicht überschreiten.

Falls erwünscht, können die jeweils gewonnenen Extraktfraktionen nach ihrer Gewinnung teilweise oder vollständig wieder kombiniert werden. Insbesondere besteht die Möglichkeit, dass aus einer oder mehreren Fraktionen eine oder mehrere unerwünschte Substanzen gemäß dem Fachmann bekannten Verfahren abgetrennt werden, und die derart aufbereiteten oder gereinigten Fraktionen mit den anderen Extraktionsfraktionen wieder vereinigt werden, um einen Extrakt zu erhalten, der von bestimmten unerwünschten, beispielsweise gesundheitsschädlichen, Substanzen befreit ist.

Von hohem Interesse ist hierbei, dass aus Extraktfraktionen oder Kombinationen von Extraktfraktionen erhaltene Substanzen oder Substanzgemische durch synergistische Wechselwirkungen oder durch Ausschluss von Substanzen mit gegenteiliger Wirkung bestimmte Wirkeigenschaften von Hopfen in verstärktem Maß zeigen können. Von Vorteil bei den erfindungsgemäßen Hopfenextrakten ist ebenso, dass sie zur Herstellung von Bieren verwendet werden können, die die Erfordernisse des deutschen Reinheitsgebots erfüllen.

Insbesondere liegt ein Vorteil des erfindungsgemäßen Verfahrens in der Steuerungsmöglichkeit der Extraktion, so dass durch geeignete Lösungsmittel beziehungsweise Lösungsmittelmischungen ein sukzessives Herauslösen der Inhaltsstoffe aus dem Extraktionsmaterial möglich ist und dadurch nachfolgende Reinigungsverfahren, wie beispielsweise chromatographische Trennmethoden, entfallen oder aber vereinfacht werden. Falls erwünscht können die erfindungsgemäßen Verfahren natürlich auch unter Schutzgasatmosphäre, unter Verwendung von entgasten und/oder mit Schutzgas gesättigten Lösungsmitteln oder Lösungsmittelgemischen und falls notwendig mit abgedunkelten Behältern durchgeführt werden.

Von besonderem Vorteil ist das erfindungsgemäße Verfahren bei einer Trennung von Materialien, die empfindliche Substanzen und Substanzgemische umfassen, beispielsweise mehrfach ungesättigte Verbindungen oder mono- oder polyzyklische Verbindungen, bei denen eine Ringeinheit mit mehr als einer Hydroxy- oder Ketogruppe substituiert ist, insbesondere für Substanzen und Substanzgemische, die Flavonoide oder α-Bittersäuren (beispielsweise Humulon, Cohumulon oder Adhumulon) oder β-Bittersäuren (beispielsweise Lupulon, Colupulon oder Adlupulon) umfassen.

Der mehrstufige Auswaschungsvorgang kann mindestens zwei der nachstehenden Stufenextraktionen umfassen, A) Extraktion mit Wasser, B) Extraktion mit Ethanol/Wasser-Mischung 25 - 35 Vol.-%, vorzugsweise 29 - 31 Vol.-%, insbesondere 30 % an Ethanol, C) Extraktion mit Ethanol/Wasser-Mischung 45 bis 55 Vol.-%, vorzugsweise 49 bis 51 Vol.-%, insbesondere 50 % an Ethanol, D) Extraktion mit Ethanol/Wasser-Mischung mit 75 bis 85 Vol.-%, vorzugsweise 79 bis 81 Vol.-%, insbesondere 80 Vol.-% an Ethanol; E) Extraktion mit Ethanol/Wasser-Mischung mit 90 bis 96 Vol.-%, vorzugsweise 94 bis 96 Vol.-%, insbesondere 96 % an Ethanol, jeweils bezogen auf das Gesamtvolumen der Ethanol/Wasser-Mischung. Bevorzugt umfasst das erfindungsgemäße Verfahren zur Bereitstellung einer höheren Fraktionierung, mindestens drei, mindestens vier oder alle fünf vorstehend aufgeführten Stufenextraktionen. Optional können gemäß dem Wissen des Fachmanns eine oder mehrere andere Stufenextraktionen vor, zwischen oder nach den vorstehend angegebenen Stufenextraktionsschritten durchgeführt werden.

Bei Stufe A erfolgt eine Gewinnung von Gerbstoffen, zuckerhaltigen Verbindungen, α-Bittersäuren, Pre- und Post-Humulon beziehungsweise Pre- und Post-Lupulon. Diese Fraktion kann beispielsweise als Schäumungsmittel verwendet werden und umfasst die Substanz, die in Bier Kopfschmerzen verursacht.

Bei Stufe B werden α-Bittersäuren gewonnen. Falls eine hohe Reinheit erwünscht ist, kann gegebenenfalls gemäß dem Wissen eines Fachmanns eine weitere Reinigung erfolgen. Fraktion 2 kann beispielsweise als Schäumungsmittel und/oder als Konservierungsmittel verwendet werden. Weiterhin zeigt Fraktion 2 eine Aktivität gegen Staphylokokken, die multiple Antibiotika-Resistenzen (MRS) aufweisen und gegen Helicobacter pylori und/oder kann als Pestizid eingesetzt werden.

In Stufe C werden Flavonoide, derart wie beispielsweise Xanthohumol, gewonnen. Gegebenenfalls können durch nachfolgende laborübliche Verfahren, wie beispielsweise VLC (vacuum liquid chromatography), angereicherte Flavonoide oder Flavonoide in reiner Form oder in einer Form mit hoher Reinheit erhalten werden.

Bei Stufe D werden α-Bittersäuren und β-Bittersäuren gewonnen. Falls erwünscht kann der bei Stufe D gewonnene Hopfenextrakt (Fraktion 4) gemäß dem Fachmann bekannter Verfahren weiter aufgetrennt werden, wobei reine Bittersäuren beziehungsweise angereicherte Lupulon-Extrakte und/oder angereicherte Humulon-Extrakte gewonnen werden. Insbesondere kann dies beispielsweise durch Extraktion einer organischen Phase des Gemisches aus α-Bittersäuren und β-Bittersäuren mit einer wässrigen Phase, mit saurem, neutralen oder basischem pH-Wert, beispielsweise durch einen dem Fachmann bekannten Säure-Base-Trennungsgang erfolgen. Der Hopfenextrakt von Stufe D und/oder die darin enthaltenen Substanzen, insbesondere α-Bittersäuren und β-Bittersäuren, weisen beruhigende und schlaffördernde Eigenschaften auf und können (nicht zur Erfindung gehörend) daher zur Herstellung eines beruhigenden oder schlaffördernden Medikaments verwendet werden.

In Stufe E werden Fette und Chlorophylle und weitere, nicht wasserlösliche oder in einem Gemisch aus Wasser und Ethanol mit einem Ethanolgehalt von weniger als 90 % nicht lösliche Inhaltsstoffe gewonnen.

Falls erwünscht kann das erfindungsgemäße Verfahren auch derart durchgeführt werden, dass mindestens zwei, vorzugsweise mindestens drei, bevorzugterweise mindestens vier oder fünf Extraktionsschritte gemäß der Reihenfolge Stufenextraktionsschritt E) Extraktion mit Ethanol/Wasser-Mischung mit 90 bis 96 Vol.-%, vorzugsweise 94 bis 96 Vol.-%, insbesondere 96 % an Ethanol D) Extraktion mit Ethanol/Wasser-Mischung mit 75 bis 85 Vol.-%, vorzugsweise 79 bis 81 Vol.-%, insbesondere 80 Vol.-% an Ethanol, C) Extraktion mit Ethanol/Wasser-Mischung 45 bis 55 Vol.-%, vorzugsweise 49 bis 51 Vol.-%, insbesondere 50 % an Ethanol, B) Extraktion mit Ethanol/Wasser-Mischung 25 - 35 Vol.-%, vorzugsweise 29-31 Vol.-%, insbesondere 30 Vol.-% an Ethanol, jeweils bezogen auf das Gesamtvolumen der Ethanol/Wasser-Mischung, und A) Extraktion mit Wasser erfolgen.

Falls erwünscht können die jeweiligen Extrakt-Fraktionen der vorstehend aufgeführten Schritte A-E hinsichtlich bestimmter Inhaltsstoffe weiter angereichert oder abgereichert werden oder die Inhaltsstoffe können hieraus in einer Reinheit gewonnen werden, die für die jeweiligen gewünschten Anwendungen geeignet ist. Hierfür können verschiedene, dem Fachmann bekannte Verfahren, beispielsweise chromatographische Reinigungsverfahren, verwendet werden.

Die erfindungsgemäßen Verfahren ermöglichen insbesondere eine vollständige oder teilweise Abtrennung der im Hopfen vorkommenden Phytohormone 6-Prenylnaringenin und 8-Prenylnaringenin, bei denen es sich um Stoffe handelt, die im Verdacht stehen beziehungsweise von denen nachgewiesen ist, dass sie beim Menschen hormonähnlich wirken. Die Wirkung ist ähnlich zu der von weiblichen Hormonen aus der Gruppe der Östrogene. Speziell bei 8-Prenylnaringenin ist nachgewiesen, dass es ähnlich dem 17 β-Östradiol wirkt. Um unerwünschte oder gesundheitsschädliche Auswirkungen auf Konsumenten, insbesondere auf männliche Konsumenten, jedoch auch in bestimmten Fällen auf Frauen, sicher zu vermeiden, besteht daher der Wunsch nach Produkten auf Hopfenbasis, aus denen diese Inhaltstoffe ganz oder teilweise entfernt sind. Dies gilt nicht nur für Bier oder Brauereiprodukte, sondern auch für hopfenprodukthaltige Kosmetika und Pharmaka.

Hierzu wird ein erfindungsgemäßes Extraktionsverfahren durchgeführt, das den vorstehend angegebenen Stufenextraktionsschritt C umfasst, wobei bei dem Stufenextraktionsschritt C 6-Prenylnaringenin und 8-Prenylnaringenin abgetrennt wird. Ein Hopfenextrakt, der frei von 6-Prenylnaringenin und 8-Prenylnaringenin ist, kann dann erhalten werden, indem die Extrakte, die bei dem mindestens einen weiteren (vorhergehenden und/oder nachfolgenden) Stufenextraktionsschritt gewonnen werden, vereinigt werden, wobei optional auch der mittels dem Fachmann bekannter, üblicher Verfahren von 6-Prenylnaringenin und 8-Prenylnaringenin befreite Hopfenextrakt des Schritts C, zugesetzt werden kann.

Insbesondere betrifft die vorliegende Erfindung daher auch eine Verwendung eines Phythohormon-freien oder Phythohormon-abgereicherten Gemisches von Hopfeninhaltsstoffen, vorzugsweise eines Gemisches von Hopfeninhaltsstoffen, das frei ist von 6-Prenylnaringenin und/oder 8-Prenylnaringenin oder an 6-Prenylnaringenin und/oder 8-Prenylnaringenin abgereichert ist, zur Herstellung von Getränken und Brauereiprodukten, insbesondere von Bier, das ebenfalls gemäß des deutschen Reinheitsgebots hergestellt werden können,

Auf diese Weise ermöglicht die vorliegende Erfindung in wiederholbarer Weise die Gewinnung eines Hopfenextrakts, der frei von 6-Prenylnaringenin und 8-Prenylnaringenin ist oder in starken Maße daran abgereichert ist. In der vorliegende Erfindung bedeutet der Begriff "in starken Maße an einer Substanz abgereichert", dass die Substanz mit einem Gehalt vorliegt, der weniger als 50 %, vorzugsweise weniger als 80 %, bevorzugterweise weniger als 95 % des Gehalts der Substanz im Ausgangsmaterial beträgt, jeweils bezogen auf das Trockengewicht. Der Begriff "an einer Substanz abgereichert" ("an einer Substanz angereichert") bedeutet, dass die Substanz mit einem Gehalt vorliegt, der weniger als 10 % (mehr als 110 %), vorzugsweise weniger als 40 % (mehr als 200 %), bevorzugterweise weniger als 80 % (mehr als 300 %) des Gehalts der Substanz im Ausgangsmaterial beträgt, jeweils bezogen auf das Trockengewicht.

Weiterhin stellt die vorliegende Erfindung bereit, dass bei einem Auswaschungsschritt ein Extrakt gewonnen wird, der aus den wasserlöslichen Hopfeninhaltsstoffen, insbesondere Gerbstoffen, besteht. Hierzu wird in einem ersten Schritt das Extraktionsgut mit Wasser extrahiert und der derart gewonnene Hopfenextrakt aufgefangen.

Die vorliegende Erfindung stellt daher Verfahren bereit, die überraschenderweise in sehr einfacher, aber äußerst effektiver Weise eine Abtrennung oder Anreicherung von bestimmten Naturstoffen, insbesondere Flavonoiden, ermöglichen. Das Erstellen von mit Flavonoiden, wie beispielsweise Xanthohumol, angereicherten Fraktionen ist aufgrund ihres zellschützenden Potentials wünschenswert. So werden Flavonoide allein oder in Kombination mit anderen aktiven Substanzen, wie beispielsweise Vitaminen, bei der Vorbeugung bzw. Behandlung von Zellalterung eingesetzt.

Darüber hinaus bietet das erfindungsgemäße Verfahren die Möglichkeit eines vergleichsweise geringen Lösungsmittelbedarfs und bietet die Möglichkeit zu einer Automatisierung.

Insbesondere ermöglicht die vorliegende Erfindung die Gewinnung eines Substanzgemisches oder einer Substanz, ausgewählt aus der Gruppe bestehend aus, Xanthohumol, Xanthohumol-abgereicherter Hopfenextrakt, Xanthohumol-angereicherter Hopfenextrakt, 6-Prenylnaringenin, 6-Prenylnaringenin-abgereicherter Hopfenextrakt, 6-Prenylnaringenin-angereicherter Hopfenextrakt, 8-Prenylnaringenin, 8-Prenylnaringenin-abgereicherter Hopfenextrakt, 8-Prenylnaringenin-angereicherter Hopfenextrakt, Adlupulon, Adlupulon-abgereicherter Hopfenextrakt, Adlupulon-angereicherter Hopfenextrakt, Colupulon, Colupulon-abgereicherter Hopfenextrakt, Colupulon-angereicherter Hopfenextrakt, Lupulon, Lupulon-abgereicherter Hopfenextrakt, Lupulon-angereicherter Hopfenextrakt, Humulon, Humulon-abgereicherter Hopfenextrakt, Humulon-angereicherter Hopfenextrakt, Adhumulon, Adhumulon-abgereicherter Hopfenextrakt, Adhumulon-angereicherter Hopfenextrakt, Cohumulon, Cohumulon-abgereicherter Hopfenextrakt, Cohumulon-angereicherter Hopfenextrakt, α-Bittersäure-Gemische, α-Bittersäuren-abgereicherter Hopfenextrakt, α-Bittersäuren-angereicherter Hopfenextrakt, β-Bittersäure-Gemische, β-Bittersäuren-abgereicherter Hopfenextrakt, β-Bittersäuren-angereicherter Hopfenextrakt und Gemische hiervon.

Hierbei können spezifische Hopfeninhaltsstoffe in reiner Form oder in einer Form mit hoher Reinheit (beispielsweise mit einer Reinheit von 85% oder mehr, vorzugsweise von 90% oder mehr, bevorzugterweise von 97 % oder mehr) gewonnen werden. Außerdem können mit der vorliegenden Erfindung ein Gemisch von α-Bittersäuren in reiner Form oder in einer Form mit hoher Reinheit, ein Humulon-angereichertes Gemisch von α-Bittersäuren, ein Lupulon-angereichertes Gemisch von β-Bittersäuren oder Humulon oder Lupulon in reiner Form oder in einer Form mit hoher Reinheit gewonnen werden.

Die vorliegende Erfindung betrifft zudem die vorstehend aufgeführten Substanzen und Substanzgemische. Der Begriff Hopfenextrakt kann in der vorliegenden Erfindung sowohl den Lösungsmittel-haltigen Hopfenextrakt, als auch das (die) nach Trocknung hieraus erhaltene Substanzgemisch (Substanz) bezeichnen.

Diese Hopfeninhaltsstoffe oder Substanzgemische von Hopfeninhaltsstoffen können für zahlreiche Anwendungen eingesetzt werden. Eine besonders wichtige Anwendung (nicht zur Erfindung gehörend) ist eine Verwendung eines oder mehrerer der vorstehend genannten Hopfeninhaltsstoffe oder eines der vorstehend genannten an- oder abgereicherten Substanzgemische von Hopfeninhaltsstoffen zur Herstellung eines schlaffördernden und/oder beruhigenden Medikaments. Vorzugsweise werden hierfür α-Bittersäure-Gemische, α-Bittersäuren-abgereicherter Hopfenextrakt, α-Bittersäuren-angereicherter Hopfenextrakt, β-Bittersäure-Gemische, β-Bittersäuren-abgereicherter Hopfenextrakt, β-Bittersäuren-angereicherter Hopfenextrakt und Gemische hiervon verwendet.

Ein derartiges Medikament (nicht zur Erfindung gehörend) kann beispielsweise das Einschlafen erleichtern, aber auch die Länge der Schlafphasen einer Person verlängern. Ein derartiges Medikament ist insbesondere zur Anwendung bei Menschen und Säugetieren gedacht. Die spezifische Menge an einem oder mehreren Hopfeninhaltsstoff(en) oder einem oder mehreren an- oder abgereicherten Substanzgemisch(en) von Hopfeninhaltsstoffen kann von einem Fachmann gemäß seines allgemeinen Wissens gewählt werden. Vorzugsweise ist ein derartiges Medikament frei von 6-Prenylnaringenin und 8-Prenylnaringenin oder enthält weniger als 5 Gew.-%, vorzugsweise weniger als 3 Gew.-%, an 6-Prenylnaringenin und/oder 8-Prenylnaringenin, bezogen auf das Trockengesamtgewicht der Hopfeninhaltsstoffe.

Vorzugsweise ist ein derartiges Medikament (nicht zur Erfindung gehörend) zur oralen Einnahme geeignet. Die spezifische Formulierung des Medikaments kann beispielsweise in Form von Tabletten, Kapseln oder in einer flüssigen Formulierungsform vorliegen. Die Verabreichung kann beispielsweise oral, mittels i.v. oder i.m. Injektion, einer Verabreichung mittels Spray, nasal, mittels Inhalation, vaginal, anal oder topisch oder in einer anderen dem Fachmann bekannten Verabreichungsweise erfolgen. Beispielsweise kann eine derartige Formulierung eine Dosis an einem Hopfeninhaltsstoff oder an einem Gemisch von Hopfeninhaltstoffen im Bereich von 0,001 g bis 10 g enthalten, wobei eine derartige Formulierung einmal oder mehrmals täglich eingenommen werden kann. Optional kann das Medikament weitere dem Fachmann bekannte Arzneiwirkstoffe, insbesondere zur Schlafförderung oder als Beruhigungsmittel bekannte Arzneiwirkstoffe, und geeignete Formulierungszusatzstoffe umfassen.

Weiterhin kann einer der vorstehend genannten Hopfeninhaltsstoffe oder eines der vorstehend genannten Substanzgemische von Hopfeninhaltsstoffen zur Herstellung eines Medikaments (nicht zur Erfindung gehörend) zur Behandlung und/oder Vorbeugung von Erkrankungen, die durch eine Infektion mit Staphylokokken, insbesondere durch Staphylokokken, die multiple Antibiotika-Resistenzen aufweisen, verursacht werden und/oder zur Behandlung und/oder Vorbeugung von Erkrankungen, die durch eine Infektion mit Helicobacter pylori verursacht werden, verwendet werden. Vorzugsweise werden hierfür α-Bittersäure-Gemische, α-Bittersäurenabgereicherter Hopfenextrakt, α-Bittersäuren-angereicherter Hopfenextrakt, β-Bittersäure-Gemische, β-Bittersäuren-abgereicherter Hopfenextrakt, β-Bittersäuren-angereicherter Hopfenextrakt und Gemische hiervon verwendet.

Vorzugsweise ist ein derartiges Medikament (nicht zur Erfindung gehörend) frei von 6- Prenylnaringenin und 8-Prenylnaringenin oder enthält weniger als 5 Gew.% an 6- Prenylnaringenin und/oder 8-Prenylnaringenin, vorzugsweise weniger als 3 Gew.-%, an 6-Prenylnaringenin und/oder 8-Prenylnaringenin, jeweils bezogen auf das Trockengesamtgewicht der Hopfeninhaltsstoffe beziehungsweise das Trockengesamtgewicht an 6-Prenylnaringenin und/oder 8-Prenylnaringenin.

Die spezifische Formulierung des Medikaments (nicht zur Erfindung gehörend) kann beispielsweise in Form von Tabletten, Kapseln oder in einer flüssigen Formulierungsform vorliegen. Die Verabreichung kann beispielsweise oral, mittels i.v. oder i.m. Injektion, einer Verabreichung mittels Spray, nasal, mittels Inhalation, vaginal, anal oder topisch oder in einer anderen dem Fachmann bekannten Verabreichungsweise erfolgen. Beispielsweise kann eine derartige Formulierung eine Dosis an einem Hopfeninhaltsstoff oder an einem Gemisch von Hopfeninhaltstoffen im Bereich von 0,001 g bis 10 g enthalten, wobei eine derartige Formulierung einmal oder mehrmals täglich eingenommen werden kann. Optional kann das Medikament weitere dem Fachmann bekannte Arzneiwirkstoffe, insbesondere zur Bekämpfung von Staphylokokken bekannte Arzneistoffe, beispielsweise Antibiotika, und/oder zur Bekämpfung von Helicobacter pylori bekannte Arzneistoffe, und geeignete Formulierungszusatzstoffe umfassen.

Darüber hinaus können die genannten Hopfeninhaltsstoffe oder eines der vorstehend genannten Substanzgemische von Hopfeninhaltsstoffen als Konservierungsmittel (nicht zur Erfindung gehörend) eingesetzt werden. Besonders geeignet und in hohem Maße von den Verbrauchern akzeptiert wird eine Anwendung als Konservierungsmittel für Lebensmittel und kosmetische Produkte sein. Vorzugsweise werden hierfür α-Bittersäure-Gemische, α-Bittersäuren-abgereicherter Hopfenextrakt, α-Bittersäuren-angereicherter Hopfenextrakt, β-Bittersäure-Gemische, β-Bittersäuren-abgereicherter Hopfenextrakt, β-Bittersäuren-angereicherter Hopfenextrakt und Gemische hiervon verwendet. Den zu konservierenden Produkten können dabei beispielsweise zwischen 0,001 und 40 Gew.-%, vorzugsweise zwischen 0,005 und 2 Gew.-% an Hopfeninhaltsstoffen oder Substanzgemischen von Hopfeninhaltsstoffen zugesetzt werden, bezogen auf das Gewicht des zu konservierenden Produkts. Darüber hinaus können die Hopfeninhaltsstoffe oder Substanzgemische von Hopfeninhaltsstoffen auch als Gemisch mit einem oder mehreren weiteren anorganischen und/oder organischen Konservierungsmitteln eingesetzt werden.

Weiterhin können die genannten Hopfeninhaltsstoffe oder eines der vorstehend genannten Substanzgemische von Hopfeninhaltsstoffen als Pestizid, (nicht zur Erfindung gehörend) insbesondere Insektizid verwendet werden. Vorzugsweise werden hierfür α-Bittersäure-Gemische, α-Bittersäuren-abgereicherter Hopfenextrakt, α-Bittersäuren-angereicherter Hopfenextrakt, β-Bittersäure-Gemische, β-Bittersäuren-abgereicherter Hopfenextrakt, β-Bittersäuren-angereicherter Hopfenextrakt und Gemische hiervon verwendet. Die spezifische Formulierung des Pestizids erfolgt dabei gemäß dem Wissen des Fachmanns. Insbesondere kann die Pestizid- oder Insektizidformulierung weitere Substanzen oder Substanzgemische umfassen, die eine pestizide oder insektizide Wirkung aufweisen.

Die vorliegende Erfindung betrifft weiterhin Bier, das durch ein Verfahren hergestellt wird in welchem ein erfindungsgemäßer Hopfenextrakt verwendet wird. Bier kann hierbei jede Art von Bier sein, dass gemäß irgendeines dem Fachmann bekannten Verfahrens hergestellt ist. Das Verfahren kann beispielsweise die Schritte von Maischen, Läutern, Hopfenkochen, und alternativ von Kühlen und Vergären umfassen.

Die Erfindung wird nun eingehender durch die folgenden Beispiele erläutert, welche die Erfindung jedoch lediglich erläutern und nicht in ihrem Schutzbereich einschränken sollen.

### Beispiele

### a) Vorbereitung des Extraktionsguts:

Die Anwendung der Erfindung, d.h. des stufenweisen Auswaschverfahrens unter Ausnutzung der zentralen Funktion der pflanzlichen Eigenzellulose, soll am Beispiel der Gewinnung und der Trennung von Hopfeninhaltsstoffen aus Hopfenmaterial vorgestellt werden.

Getrocknete Hopfendolden, Hopfen-Lupulin und halbtechnische oder technische Hopfenprodukte, wie z.B. die kommerziell erhältlichen Hopfen-Pellets, die nach alkoholischer oder CO2-Extraktion erhalten werden, stellen das Ausgangsmaterial dar. Die oben genannten trockenen pflanzlichen Rohstoffe werden z.B. mit einer Alpin-Rotoplex-Schneidemühle vom Typ Ro 20/10 B fein zermahlen. Die Partikel haben nach diesem Prozeß eine maximale Größe von 1 mm³. Der optimale Zermahlungsgrad ist unterschiedlich und zwar abhängig von der Verwendung der Hopfendolden oder der Verwendung der daraus gewonnenen Hopfenrohprodukte. Das so vorbereitete Material wird in den Elutionsbehälter (B) gefüllt. Es wird auf eine möglichst homogene Befüllung des Elutionsbehälters mit dem Rohmaterial geachtet. Diese homogene, dicht gepackte Befüllung bzw. Beschickung mit Extraktionsgut soll eine verbesserte Trennung der Hopfeninhaltsstoffe durch Elution von der Eigenzellulose des Hopfens ermöglichen.

Die Lösungsmittel bzw. die Lösungsmittelmischungen (Alkohol-/Wasser-Gemisch) werden für jede Stufe der Elution so ausgewählt, daß die Hopfeninhaltsstoffe ais Einzelstoffe oder in Gruppen bzw. Klassen getrennt anfallen und ausgewaschen werden. Die Elution kann entweder als Vorwärtselution mit steigendem Anteil des Alkohols im Elutionsmittel oder als Rückwärtselution mit abnehmendem Alkohol-Anteil im Elutionsmittel durchgeführt werden.

### b) Durchführung der Elutionsschritte in der Vorwärtselution:

Sobald der Elutionsbehälter (B) durch den Füllstutzen (B6) mit dem Extraktionsgut (B1) befüllt ist, wird das Extraktionsgut bei Raumtemperatur mit Leitungswasser (Trinkwasserqualität) überschichtet. Das Wasser wandert durch das Extraktionsgut hindurch, wobei sich die Eigenzellulose des Hopfenmaterials mit Wasser belädt und infolgedessen quillt. Durch diese Wässerung wird die Eigencellulose des Hopfens konditioniert. Während der Konditionierung mit Wasser werden die im Hopfenmaterial vorhandenen Hefen, Keime und eventuell noch vorhandenen Spuren von Pflanzenschutzmitteln herausgespült und verworfen.

Danach wird nochmals reines Wasser (Trinkwasserqualität) als Elutionsmittel (Lösungsmittel) LM1 so lange über das Elutionsgut geleitet, bis die wasserlöslichen Bestandteile vollständig herausgelöst sind. Die auf diese Weise gewonnene Fraktion (Fraktion 1) wird über eine Leitung B5 in den Fraktionssammler (C1) gepumpt und dort gesammelt. Die Fraktion 1 wird über den Verdampfer (D) geleitet, um das Lösungsmittel zu verdampfen. Der Wasserdampf wird in die Rektifikationsanlage (F) überführt und durch Destillation zurückgewonnen. Das zurückgewonnene Lösungsmittel LM1 wird in einem oder in mehreren Lösungsmitteltanks (G) gesammelt und kann wiederverwendet werden. Das im Verdampfer zurückgebliebene, aufkonzentrierte Elutionsprodukt (Dickextrakt) wird in den Sammeltank (E) überführt. Dort wird der Dickextrakt für die Weiterverarbeitung oder die Abfüllung als (Vor-)Produkt breitgestellt. Die Fraktion 1 enthält die wasserlöslichen Gerbstoffe. Sie beinhaltet zahlreiche, chemisch unterschiedliche Verbindungen, die hinsichtlich ihrer chemischen Natur und biologischen Wirksamkeit u.a. zu den Polyphenolen und Anthocyanen zählen und insbesondere antioxidative Wirkungen aufweisen. Ferner enthält Fraktion 1 zuckerhaltige Verbindungen, α-Bittersäuren (Humulon, Adhumulon, Cohumulon, Prehumulon und Posthumulon) und andere in Wasser gut lösliche Hopfeninhaltsstoffe. Die zuckerhaltigen Verbindungen können beispielweise als Beruhigungsmittel (Sedativa) eingesetzt werden.

Die Durchführung eines zweiten Schrittes des Auswaschvorganges erfolgt mit Hilfe eines Lösungsmittelgemisches LM2, bestehend aus einer Ethanol/Wasser-Mischung 20 bis 40 Vol.- %, vorzugsweise mit einem Gehalt von 25 bis 35 Vol.-%, insbesondere mit einem Gehalt von 30 Vol.-% Ethanol. LM2 wird im Lösungsmitteltank (A) mit Hilfe eines Rührmotors durch Mischen hergestellt. Das auf diese Weise bereitgestellte LM2 wird über eine Leitung (A1) durch den Zulauf (B4) auf das Extraktionsgut geleitet. Durch Elution mit LM2 wird ein kleinerer Anteil der α-Bittersäuren (Humulon, Cohumulon und Adhumulon) in fast reiner Form und ein kleiner Anteil der Flavonoide (Xanthohumol und Isoxanthohumol) ausgewaschen (Fraktion 2). Die Fraktion 2 wird über die Leitung B5 in den Sammelbehälter (C2) überführt. Die Fraktion 2 wird in einen Verdampfer (D) geleitet und das Verfahren so fortgesetzt, wie bereits im Falle der Nachbehandlung der Fraktion 1 beschrieben.

Es folgt anschließend die Elution mit einem dritten Lösungsmittelgemisch LM3, bestehend aus einer Ethanol/Wasser-Mischung mit einem Anteil von 40 bis 70 Vol.-%, vorzugsweise 45 bis 60 Vol.-%, insbesondere 45 bis 55 Vol.-% Ethanol. In dieser Elutionsstufe (Fraktion 3) werden die Hopfen-Flavonoide, wie beispielweise Xanthohumol, eluiert. Fraktion 3 wird im Fraktionssammler (C3) gesammelt. Die Herstellung des Lösungsmittels LM3 erfolgt durch Mischen im Lösungsmitteltank (A). Mit LM3 wird das Extraktionsgut überschichtet, das Eluat gesammelt und der Gewinnung von Dickextrakt zugeführt. Die Rückgewinnung des Lösungsmittels erfolgt, wie im Falle der Stufen 1 und 2 bereits beschrieben. Gegebenenfalls können durch nachfolgende Anwendung labor-üblicher Verfahren, wie z.B. VLC (Vacuum Liquid Chromatography), Flavonoide in reiner Form oder in einer Form mit hoher Reinheit erhalten werden.

In einem vierten Auswaschvorgang werden unter Einsatz des Lösungsmittelgemisches LM4, bestehend aus einer Ethanol/Wasser-Mischung mit einem Anteil von 70 bis 90 Vol.-%, vorzugsweise 75 bis 85 Vol.-%, und insbesondere 80 bis 85 Vol.-% Ethanol, α-Bittersäuren und β-Bittersäuren in Form eines Gemisches (Fraktion 4) gewonnen. Diese Fraktion 4 wird im Fraktionssammler (C4) gesammelt. Die Fraktion 4 kann direkt oder nach 10-facher Eindickung als Zusatz zum Bier, als Wirkstoff oder als Zusatzstoff in der Kosmetik- und Lebensmittel-Branche verwendet werden. Das Produkt kann entweder eine wäßrige oder alkoholische, wenn auch eingedickte Lösung sein oder ein Feststoff nach weiterem Lösungsmittelentzug.

Abschließend kann noch eine Elution des Extraktionsguts mit einer Ethanol/Wasser-Mischung LM5, die 90 bis 96 Vol.-%, vorzugsweise 94 bis 96 Vol.-%, und insbesondere 96 Vol.-% Ethanol enthält, durchgeführt werden. Auf diese Weise werden Chlorophylle und Fette sowie weitere nicht in Wasser lösliche oder in einem Gemisch aus Wasser und Ethanol mit einem Ethanol-Gehalt von weniger als 90 % unlösliche Inhaltstoffe gewonnen (Fraktion 5).

Die einzelnen Elutionsschritte sind dann beendet, wenn im eluierenden Lösungsmittel bzw. Lösungsmittelgemisch keine gelösten Substanzen mehr nachweisbar sind. Eine schrittweise Durchführung der oben beschriebenen Elutionsstufen liefert die Fraktionen 1 bis 5. Die selektive Gewinnung nur einer einzelnen Fraktion ist ebenfalls möglich. Wenn man zum Beispiel mit LM3 eluiert, wird zunächst das Extraktionsgut von den Fraktionen 1 bis 3 freigewaschen. Anschließend gewinnt man mit LM4 die Fraktion 4.

Vorzugsweise führt das erfindungsgemäße Verfahren zu einer höheren Fraktionierung, wenn mindestens zwei, mindestens drei, mindestens vier oder alle fünf vorstehend aufgeführten Stufenelutionen durchgeführt werden. Gemäß dem Wissen des Fachmannes können in optimaler Weise eine oder mehrere weitere Stufenelutionen durchgeführt werden, nämlich solche, die den Auswaschbereich vor, zwischen oder nach den vorstehend benannten Stufenelutionen erfassen.

### c) Inverses Elutionsverfahren zur Trennung der Hopfeninhaltsstoffe:

Im Falle des erfindungsgemäßen Verfahrens können die bereits vorher beschriebenen Elutionsschritte auch in umgekehrter Reihenfolge ausgeführt werden (inverse Elution) und zwar dergestalt, daß die Elution der Hopfeninhaltsstoffe mittels eines oder mehrerer Auswaschgänge, d.h. durch sukzessiven, stufenweisen Einsatz - nicht aber als Gradient - von Alkohol, einer oder mehrerer Wasser/Alkohol-Mischungen und reinem Wasser als mobile Phase, erfolgt. Die bei der inversen Elution nacheinander ablaufenden Prozeßschritte werden wie folgt beschrieben:
1. Zerkleinern (Mahlen, Pulverisieren, Häckseln) des getrockneten (halb getrockneten, frischen) pflanzlichen Materials, das die Eigencellulose als stationäre Phase und Hauptbestandteil enthält. 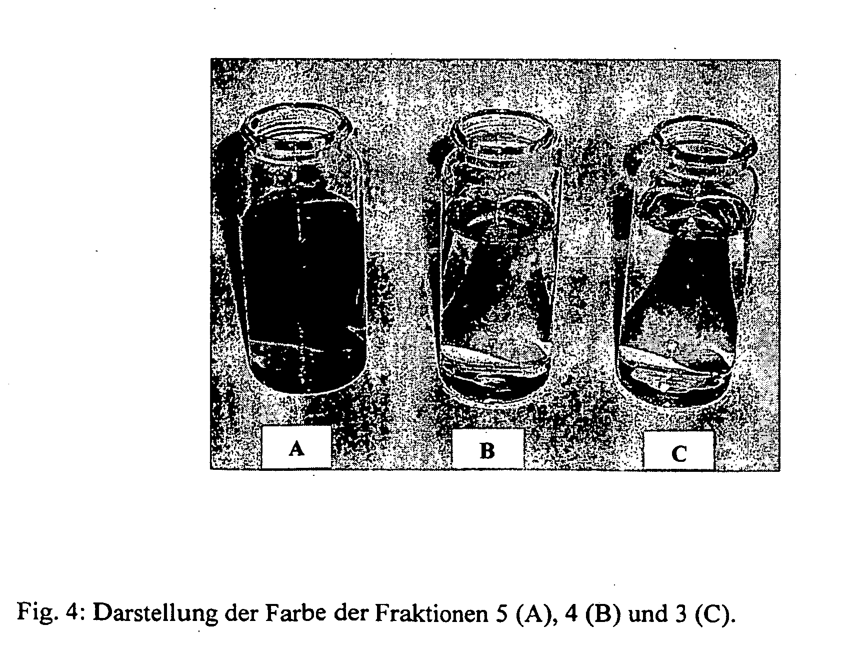
2. Befüllen des Eluators (B) auf dem Wege durch den Füllstutzen (B6) mit den pulverisierten pflanzlichen (feuchten oder trockenen) Ausgangsmaterialien, wobei der Eluator mindestens einen Zufluß (B4) und mindestens einen Abfluß (B5) mit Regulierventil (B7) besitzt. Vor der Abflußöffnung in jedem Falle, soweit erforderlich auch innerhalb des Eluators, sind eine oder mehrere Rückhaltevorrichtungen (z.B. B2 als Sieb, Filterplatte oder Pfropf aus Glasswolle), auf denen das zerkleinerte Material gepackt ist, eingebaut. Die Bedingungen, unter denen die inverse Elution in der Regel durchgeführt wird, sind Raumtemperatur, Normaldruck und die Gegenwart von Luft. Prinzipiell können jedoch die Elutionsbedingungen dahingehend geändert werden, dass zur Bearbeitung bei anaeroben Bedingungen Stickstoff-Atmosphäre herrscht oder Temperaturänderungen und unterschiedliche Drucke bestehen.

Sobald der Elutionsbehälter (B) mit dem Extraktionsgut (B1) befüllt ist, wird das Extraktionsgut bei Raumtemperatur mit 96%-igem Ethanol überschichtet. Im Falle des erfindungsgemäßen Verfahrens kann die Änderung der Polarität des Elutionsmittels zum Zwecke der schritt weisen Auswaschung der pflanzlichen Inhaltsstoffe auch so erfolgen, daß mindestens zwei, bevorzugter weise jedoch mindestens vier oder fünf Elutionsschritte nacheinander mit einer Ethanol/Wasser-Mischung (LM5), die 90 bis 96 Vol.-%, vorzugsweise 94 bis 96 Vol.-%, und insbesondere 96 Vol.-% Ethanol enthält. Unter dem Einfluß des Ethanols quillt die Hopfenzellulose nicht (oder nicht merklich). Die Füllhöhe des Extraktionsgutes im Elutionsbehälter bleibt (nahezu) konstant. Auf diese Weise wird ein Teil der Hopfeninhaltsstoffe, insbesondere die Hopfen-Flavonoide, und ein großer Teil der α- und β-Bittersäuren in Form eines Gemisches als braun-grüne Lösung (Fraktion 5) erhalten (siehe Fig. 4, Flasche A). Die Elution mit Hilfe von LM 5 wird so lange durchgeführt, bis ein Farbumschlag nach grün-gelb zu beobachten ist.

In einem nachfolgenden zweiten Auswaschvorgang werden unter Einsatz des Lösungsmittels LM4, bestehend aus einer Ethanol/Wasser-Mischung mit einem Anteil von 70 bis 90 Vol.-%, vorzugsweise 75 bis 85 Vol.-%, und insbesondere 80 bis 85 Vol.-% Ethanol, α-Bittersäuren und β-Bittersäuren, ein Teil der Flavonoide und Gerbstoffe (Fraktion 4) als eine grün-gelbe Lösung gewonnen (siehe Fig. 4, Flasche B). Die Eigencellulose des Hopfens quillt deutlich sichtbar während dieser Elution aufgrund des zunehmenden Wasseranteils in LM4. Der Auswaschvorgang wird so lange fortgesetzt, bis ein Farbumschlag nach helle-gelb erfolgt.

Es schließt sich die Elution mit dem dritten Lösungsmittel LM3, bestehend aus einer Ethanol/Wasser-Mischung mit einem Anteil von 40 bis 70 Vol.-%, vorzugsweise 45 bis 60 Vol.-%, insbesondere 45 bis 55 Vol.-% Ethanol, an. In dieser Elutionsstufe, erhält man eine gelb gefärbte Lösung (Fraktion 3, siehe Fig. 4, Flasche C). in dieser Fraktion 3 werden die Hopfen-Flavonoide, wie z. B. Xanthohumol, ein kleiner Teil der α-Bittersäuren und β-Bittersäuren und ein großer Teil anderer Hopfeninhaltsstoffe gesammelt.

Die Durchführung eines vierten Elutionsschrittes erfolgt mit Hilfe des Lösungsmittels LM2, bestehend aus einer Ethanol/Wasser-Mischung mit einem Gehalt von 20 bis 40 Vol.- %, vorzugsweise mit einem Gehalt von 25 bis 35 Vol.-%, insbesondere mit einem Gehalt von 30 Vol.-% Ethanol. Durch Elution mit LM2 wird die hell-gelbe Fraktion 2 ausgewaschen, die zu 90 bis 95 Gew.-% eine bisher nicht charakterisierte Substanz enthält. Der kleinere Anteil (< 5 %) besteht aus α-Bittersäuren (Humulon, Cohumulon und Adhumulon), β-Bittersäuren und Flavonoiden (Xanthohumol) bzw. Gerbstoffen, die jedoch in großer Menge in Fraktion 5 enthalten sind.

Abschließend erfolgt eine Elution des Extraktionsguts mit reinem Wasser (Trinkwasserqualität), nämlich mit dem Lösungsmittel LM1. In diesem letzen Schritt wird LM1 solange über das Extraktionsgut geleitet, bis die wasserlöslichen Bestandteile, die hellgelb sind, vollständig herausgelöst sind (Fraktion 1).

Aufgrund des Anstiegs des Wassergehaltes in jedem der fünf Elutionsmittel quillt die Hopfen-Eigencellulose, so daß sich das Volumen des Extraktionsguts bei jedem Elutionsschritt ausgedehnt. Demzufolge verlangsamt sich auch der Durchtritt des Lösungsmittels.

Beschreibung der Fraktionen 1 bis 5 und Nachweis der innerhalb der Fraktionen auftretenden Inhaltsstoffe nach Vorwärtselution
Die Fraktionen 1 bis 5, die mit Hilfe der zuvor beschriebenen Elutionsschritte gewonnen werden, zeigen eine in bestimmten Grenzen relativ konstante und reproduzierbare Zusammensetzung ihrer molekularen Komponenten. Die unter den Bedingungen der vorher 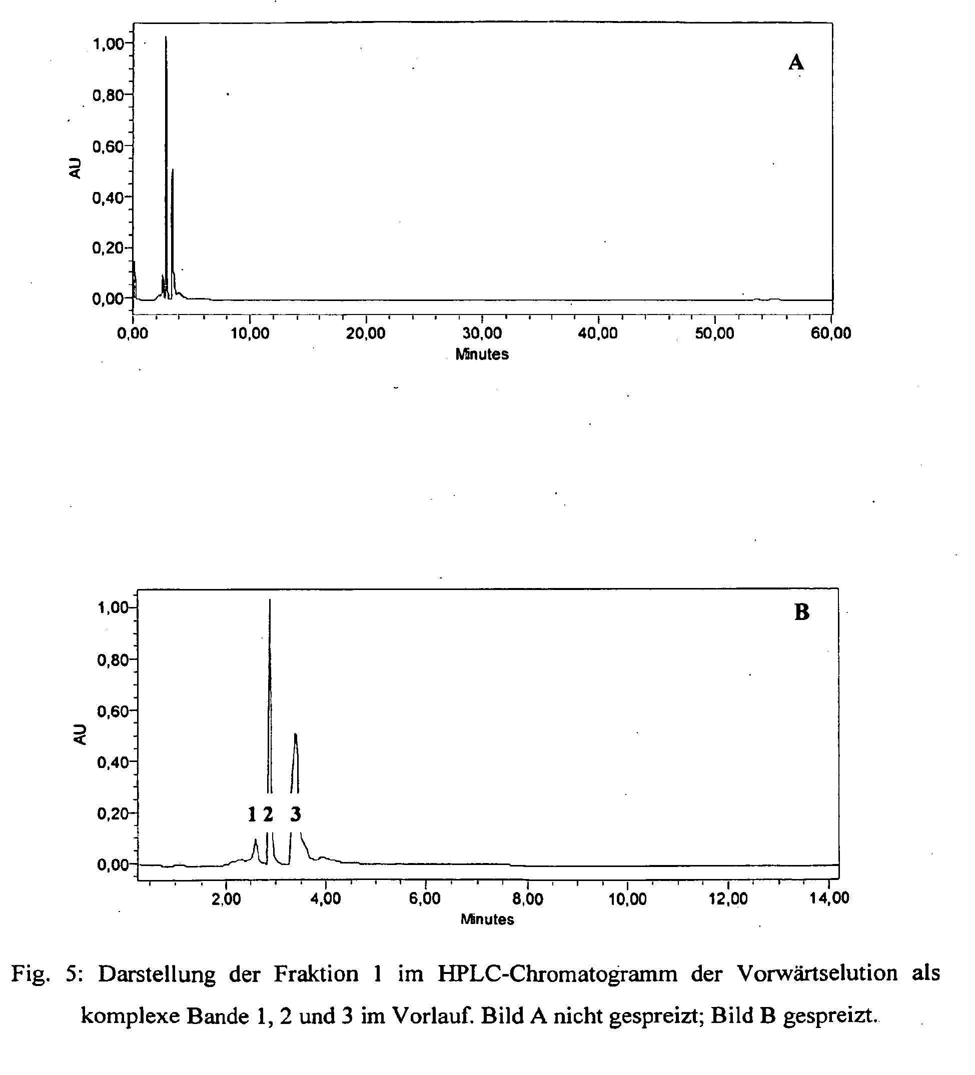 beschriebenen Elutionsschritte gewonnenen Fraktionen sind für die unter Schutz gestellte Trennmethode charakteristisch und wiederholbar. Die Fraktionen stellen in ihrer Komposition aus Einzelstoffen charakteristische Vorprodukte bzw. Produkte dar, die ebenfalls unter Schutz gestellt werden. Die in den Fraktionen enthaltenen chemisch unterschiedlichen, molekularen Komponenten lassen sich anschließend durch Anwendung bekannter klassischer Trennmethoden (z.B. HPLC und LC-MS) vollständig in molekulare Entitäten auftrennen und in reiner Form gewinnen. Die exakte molekulare Zusammensetzung einer jeden Fraktion läßt sich mit Hilfe von HPLC-Diagrammen quantitativ ermitteln. Die auflösbaren molekularen Komponenten werden bei der Wellenlänge 325 nm erfaßt und durch die Zahlen 1 bis 19 gekennzeichnet. Die bekanten Moleküle werden bestimmten Retentionszeiten zugeordnet. Nicht bekannte Moleküle werden als zur Zeit unbekannt benannt. Die wesentlichen Identifikationsmerkmale, insbesondere die prozentualen Mengenanteile, sind in Tabellen dargestellt.

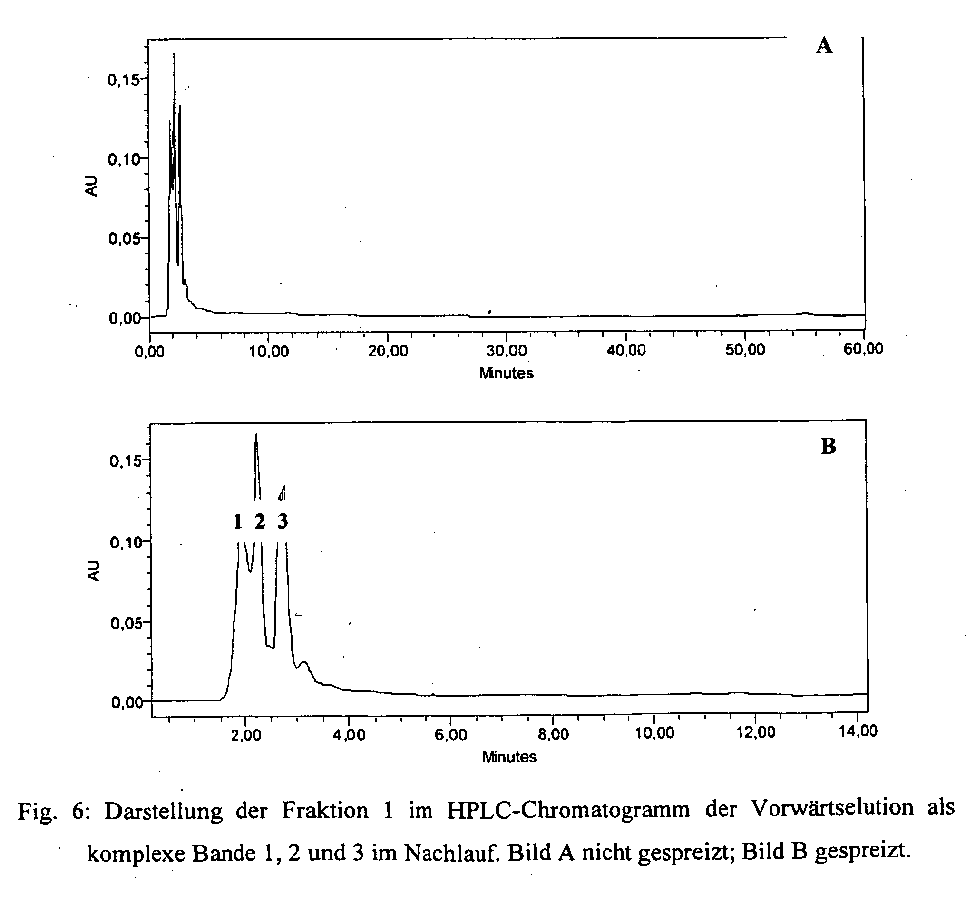

In der Fraktion 1 befinden sich die wasserlöslichen Gerbstoffe, die eine eigene Stoffklasse darstellen, zu der zahlreiche, chemisch unterschiedliche Verbindungen gehören, die aufgrund ihrer chemischen Natur und ihrer biologischen Wirkung vorwiegend zu der Gruppe der Polyphenole und der Anthocyane zählen.

Der mit reinem Wasser ausgewaschene Vorlauf der Fraktion 1 (siehe Fig. 5) zeigt im HPLC-Chromatogramm mindestens drei Banden und enthält ein schwer lösliches, leicht fällbares, hellbraunes Salz, dessen Zusammensetzung bisher noch nicht definiert wurde und wegen nicht bekannter Verwertbarkeit zur Zeit nicht gesammelt wird. Der ebenfalls mit reinem Wasser eluierte Nachlauf (siehe Fig. 6) zeigt im HPLC-Chromatogramm ebenfalls mindestens
drei aufgelöste Banden. Als Ergebnis erhält man in eine stark hygroskopische Substanz bzw. eine dunkel-braune, stark schäumende Lösung. Das Wasser läßt sich aus dieser Lösung äußerst schwer entfernen, so daß dieses Produkt in der Regel als Lösung vorliegt Der Nachlauf der Fraktion 1 enthält in größeren Mengen zuckerhaltige Verbindungen sowie andere in Wasser gut lösliche Hopfeninhaltsstoffe. Die Komplexität der molekularen Zusammensetzung der Fraktion 1 ist in den HPLC-Chromatogrammen der Fig. 5 (Bande 1 bis 3) und der Fig. 6 (Bande 1 bis 3) erkennbar. Die wesentlichen Identifikationsmerkmale, insbesondere die prozentualen Mengenanteile, werden in den Tabellen 1 und 2 dargestellt.

**Tab. 1: Identifikationsmerkmale der wesentlichen Komponenten in der Fraktion 1 (Vorlauf) aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 7,8 | unbekannt |
| 2 | 2,9 | 38,3 | unbekannt |
| 3 | 3.1 | 51,5 | unbekannt |
| Σ | | 96,6 | |

**Tab. 2: Identifikationsmerkmale der wesentlichen Komponenten in der Fraktion 1 (Nachlauf) aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 88,3 | unbekannt |
| 2 | 2.9 | 5.9 | unbekannt |
| 33b | 3,1,3,9 | 5,3 | unbekannt |
| Σ | | 99,5 | |

Die Fraktion 2 (siehe Fig. 7) wird im 2. Elutionsschritt gewonnen. Mit Hilfe der HPLC-Chromatographie werden in Fraktion 2 die Banden 1 bis 6, 8, 11 bis 13 und 16 bis 17 identifiziert. Die Fraktion 2 enthält im Wesentlichen zwei Stoffklassen, nämlich einen weiteren, weniger wasserlöslichen Anteil (ca. 49,6 Gew.%) der α-Bittersäuren Cohumulon (11), Humulon (12) und Adhumulon sowie einen kleineren Anteil der Flavonoide, z.B. Xanthohumol (die Bande 8; 3,7 Gew.%) und Isoxanthohumol (die Bande 6: 1,2 Gew.%). In den Banden 16 und 17 werden die β-Bittersäuren Colupulon (16) und Lupulon + Adlupulon (17), 1.8 Gew.% nachgewiesen. Die Bande 1 bis 3 entsprechen einem Anteil von 36,9 Gew.% und ist noch nicht charakterisierten Substanzen zuzuordnen. Das aus Fraktion 2 gewonnene gelbe Pulver enthält bis zu 49,6 Gew.% α-Bittersäuren. Die wesentlichen Identifikations-merkmale, insbesondere die prozentualen Mengenanteile, sind in Tabelle 3 dargestellt.

Die Fraktion 3 ist eine gelbe Lösung und enthält den größten Anteil der Flavonoide (siehe Fig. 8), die in der Retentionszeit 9 Minuten bis 27 Minuten erfaßt werden können. Es sind z.B. Desmethylxanthohumol, Dihydroxanthohumol, insbesondere das Xanthohumol (die Bande 8; 22,7 Gew.%) sowie Substanzen mit phytohormonaler Wirkung bzw. östrogenartig wirkende Substanzen (6- und 8-Prenylnaringenin). Durch Trocknung entsteht ein gelbes Pulver. Die Zuordnung der drei Banden 1, 2, und 3 ist noch nicht erfolgt. Die Bande 6 enthält Isoxanthohumol (1,1 Gew.%). Auch die Fraktion 3 enthält in den Banden 11, 12 und 13 die α-Bittersäuren Cohumulon (11), Humulon (12) und Adhumulon (13) in einem Gesamtmen-

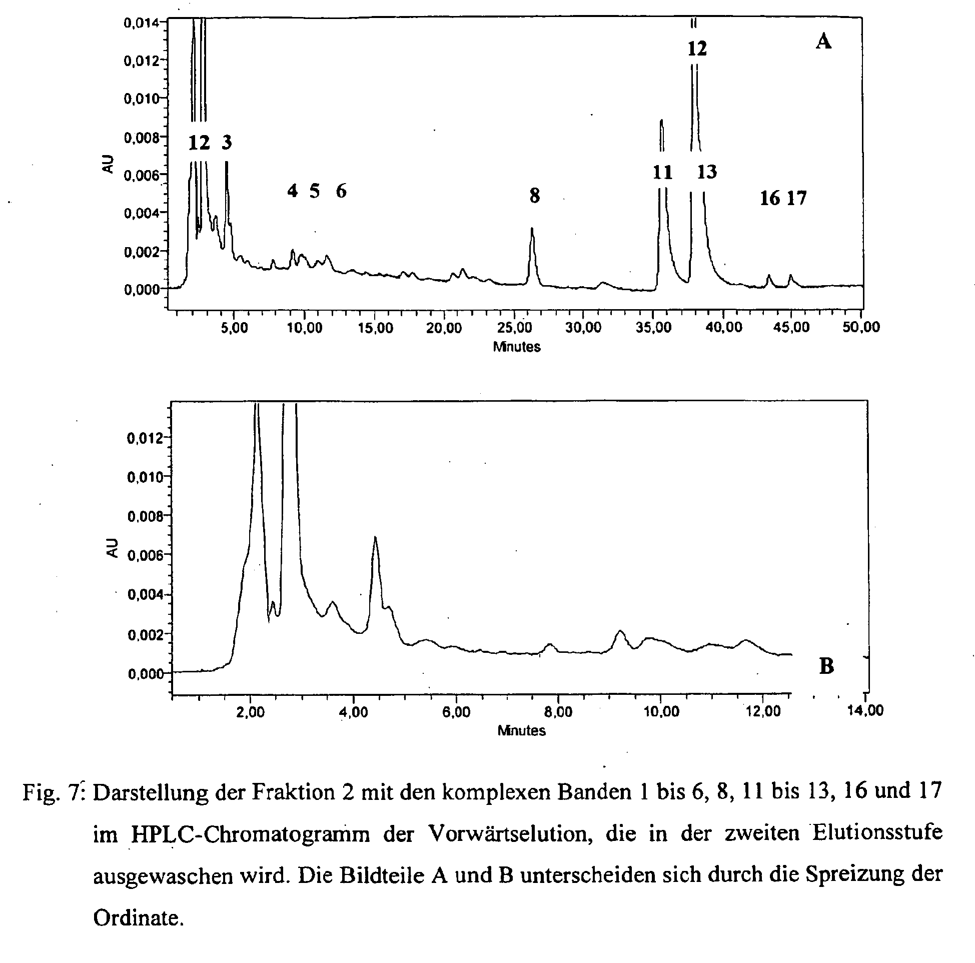

**Tab. 3: Identifikationsmerkmale der wesentlichen Komponenten in der Fraktion 2 aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 14,3 | unbekannt |
| 2 | 2,9 | 21,4 | unbekannt |
| 3 | 3,1 | 1,2 | unbekannt |
| 4 | 7,2 | 0,9 | unbekannt |
| 5 | 8,8 | 1,4 | unbekannt |
| 6 | 11,2 | 1,2 | Isoxanthohumol |
| 8 | 24,9 | 3,7 | Xanthohumol |
| 11 | 35,2 | 14,2 | Cohumulon |
| 12 | 37,4 | 35,4 | Humulon |
| 13 | 37,9 | - | Adhumulon |
| 16 | 42,6 | 0,6 | Colupulon |
| 17 | 44,1 | 1,2 | Lupulon + Adlupulon |
| Σ | | 95,5 | |

genanteil in Höhe von 67,5 Gew.%. In den Banden 16 und 17 werden die β-Bittersäuren Colupulon (16), Lupulon + Adlupulon (17) mit einem Gesamtmengenanteil in Höhe von 5,4 Gew.% nachgewiesen. Die Komplexität der molekularen Zusammensetzung der Fraktion 3 ist im HPLC-Chromatogramm der Fig. 8 als Bandenkomplex 1 bis 8 und 10 bis 17 dargestellt. Die wesentlichen Identifikationsmerkmale, insbesondere die prozentualen Mengenanteile, werden in Tabelle 4 mitgeteilt. Aus dem Komponentengemisch der Fraktion 3, die in der Retentionszeit von 5 bis 32 Minuten erfaßt wird, können gemäß dem allgemeinen Fachwissen unter Anwendung laborüblicher Trennmethoden, beispielweise mit Hilfe einer VLC (Vacuum Liquid Chromatography), monoprenylierte Flavonoide des Hopfen, ein mit Xanthohumol angereicherter Hopfenextrakt, insbesondere Xanthohumol - je nach Anwendungszweck in reiner Form oder in einer Form mit hoher Reinheit - (Produktname: Hopfenflavo natur), ein mit 6- und/oder 8-Prenylnaringenin angereicherter Hopfenextrakt, weiterhin 6- und/oder 8-Prenylnaringenin - je nach Anwendungszweck in reiner Form oder in einer Form mit hoher Reinheit - (Produktname: Hopfenphyto natur), sowie Desmethylxanthohumol, Isoxanthohumol und Dehydroxanthohumol, sowohl als monoprenylierte- als auch als diprenylierte Hopfenflavonoide, gewonnen werden.

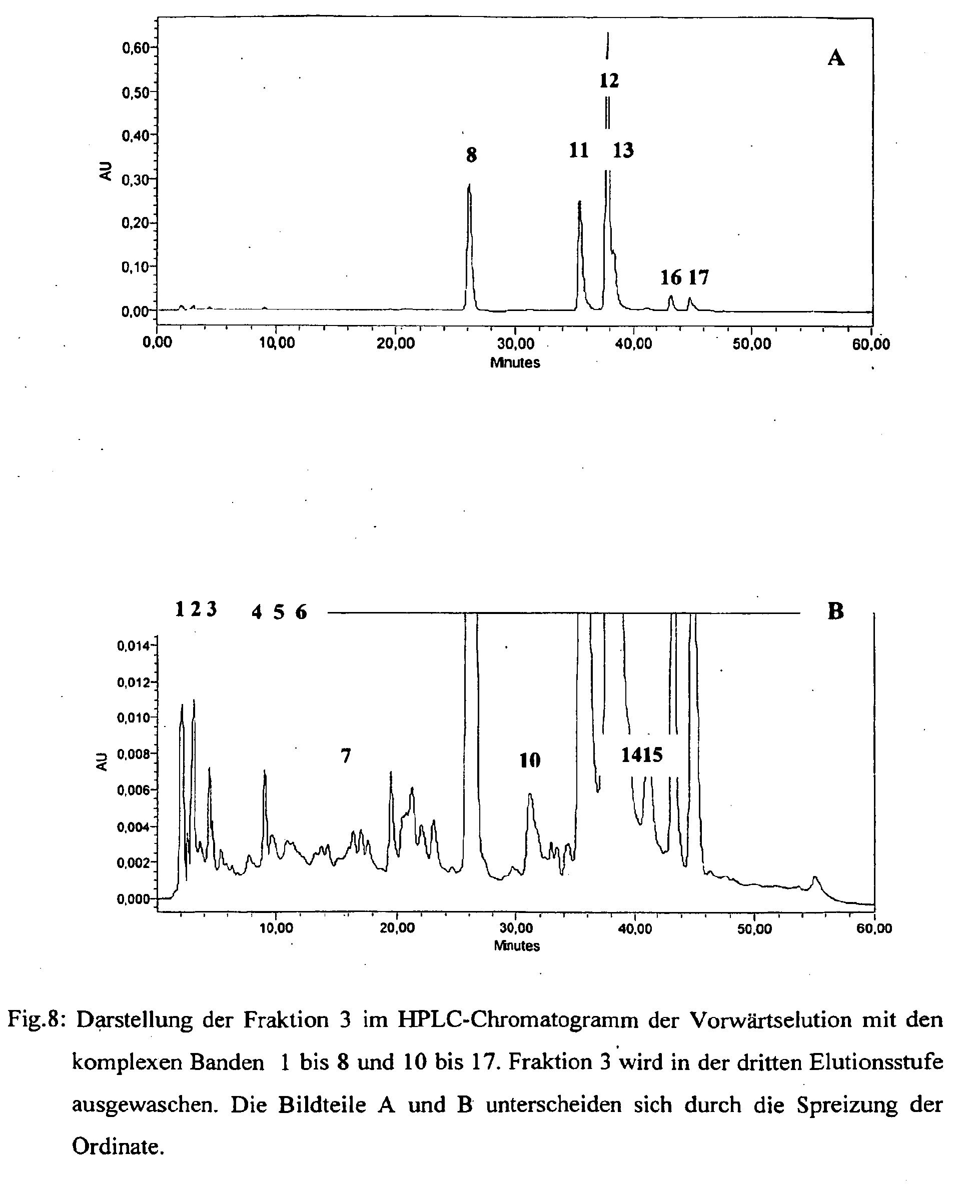

**Tab. 4: Identifikationsmerkmale der wesentlichen Komponenten der Fraktion 3 aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 0,6 | unbekannt |
| 2 | 2,9 | 0,1 | unbekannt |
| 3 | 3,1 | 0,5 | unbekannt |
| 4 | 7,2 | 0,3 | unbekannt |
| 5 | 8,8 | 0,1 | unbekannt |
| 6 | 11,2 | 0,1 | Isoxanthohumol |
| 7 | 18,5 | 0,4 | unbekannt |
| 8 | 24,9 | 22,7 | Xanthohumol |
| 10 | 30,9 | 0,3 | Xanthohumol D |
| 11 | 35,2 | 18,4 | Cohumulon |
| 12 | 37,4 | 40,2 | Humulon |
| 13 | 37,9 | 8,9 | Adhumulon |
| 15 | 40,6 | 0,6 | unbekannt |
| 16 | 42,6 | 2,3 | Colupulon |
| 17 | 44,1 | 2,5 | Lupulon + Adlupulon |
| Σ | | 98,0 | |

Die Fraktion 4 ist eine braun-gelbe Lösung, die bei längerem Stehen unter Luft eine rötliche Farbe annimmt. Nach dem Trocknen erhält man ein braun-gelbes Pulver. Die Lösung stellt 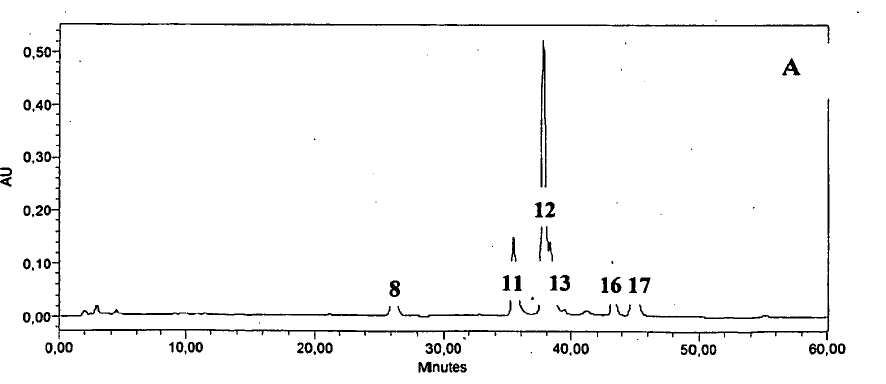 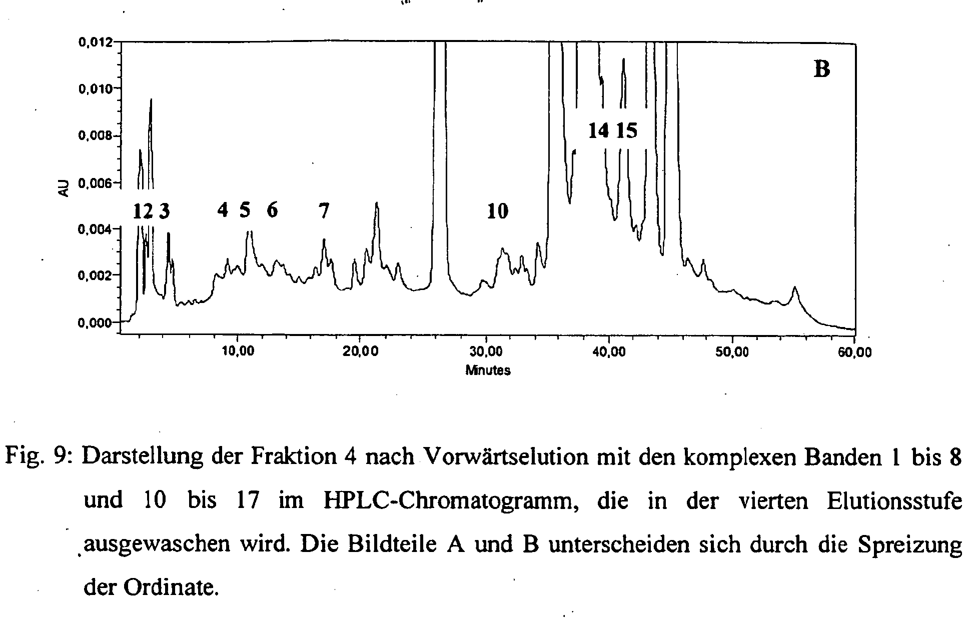 ein Gemisch der Hopfenbittersäuren (siehe Fig. 9) dar, nämlich bestehend aus den α-Bittersäuren Cohumulon (11), Humulon (12), Adhumulon (13), repräsentiert durch die Banden 11 bis 13, mit 66,9 Gew.% und aus den β-Bittersäuren Colupulon (16) und Lupulon + Adlupulon (17), repräsentiert durch die Banden 16 bis 17, mit 19,2 Gew.% in weitgehend reiner Form. Weiterhin enthält die Fraktion 4 Flavonoide, insbesondere Xanthohumol (die Bande 8; 9,8 Gew.-%). Die Banden in der Retentionszeit von 1,5 - bis 24,0 Minuten machen zusammen 1,6 Gew.-% aus. Sie repräsentieren Spuren der auch in den Fraktionen 2 und 3 vorkommenden Substanzen. Die Komplexität der molekularen Zusammensetzung der Fraktion 4 ist im HPLC-Chromatogramm der Fig. 9 als Bandenkomplexe 1 bis 8 und 10 bis 17 dargestellt. Die wesentlichen Identifikationsmerkmale, insbesondere die prozentualen Mengenanteile, erscheinen in Tabelle 5.

Unter Einsatz bekannter Trennverfahren können die α-Bittersäuren und die β-Bittersäuren in ihrem Gemisch vollständig aufgetrennt werden. Auf diese Weise können erstmals Bittersäuren in reiner Form und in großen Mengen, sowie angereicherte Lupulin-Extrakte und/oder angereicherte Humulon-Extrakte gewonnen werden.

Diese Trennung kann beispielsweise mit Hilfe des bekannten Säure-Base-Trennungsganges erfolgen. Hierzu wird die Fraktion 4 mit einer 5 bis 10 Gew.-%igen NaOH-Lösung versetzt und unter Durchmischung auf 50 bis 80 °C 30 bis 60 Minuten erwärmt. Nach Abkühlung finden sich im Filtrat die β-Bittersäuren und im Rückstand die α-Bittersäuren.

**Tab. 5: Identifikationsmerkmale der wesentlichen Komponenten der Fraktion 4 aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 0,7 | unbekannt |
| 2 | 2,9 | 0,2 | unbekannt |
| 3 | 3,1 | 0,5 | unbekannt |
| 5 | 8,8 | 0,2 | unbekannt |
| 8 | 24,9 | 9,8 | Xanthohumol |
| 10 | 30,9 | 0,3 | Xanthohumol D |
| 11 | 35,2 | 13,4 | Cohumulon |
| 12 | 37,4 | 40,7 | Humulon |
| 13 | 37,9 | 12,9 | Adhumulon |
| 14 | 38,9 | 0,4 | unbekannt |
| 15 | 40,6 | 1,1 | unbekannt |
| 16 | 42,6 | 8,0 | Colupulon |
| 17 | 44,1 | 10,1 | Lupulon + Adlupulon |
| Σ | | 98,3 | |

Insbesondere können je nach Anwendungszweck α-Bittersäuren in reiner Form oder in einer Form mit hoher Reinheit, ein mit Humulon angereichertes Gemisch von α-Bittersäuren, ein mit Lupulon angereichertes Gemisch von β-Bittersäuren oder Lupulon in reiner Form oder in einer Form mit hoher Reinheit gewonnen werden. Insbesondere bietet die vorliegende Erfindung die Möglichkeit, den Humulon-Gehalt in einem Gemisch mit α-Bittersäuren oder den Lupulon-Gehalt in einem Gemisch mit β-Bittersäuren reproduzierbar und zuverlässig einzustellen.

Die Fraktion 5, die in der Vorwärtselution ausgewaschen wird, enthält alle nur in 90 bis 97 Gew.-% Ethanol löslichen Hopfenbestandteile. Diese Fraktion ist zunächst grün gefärbt und 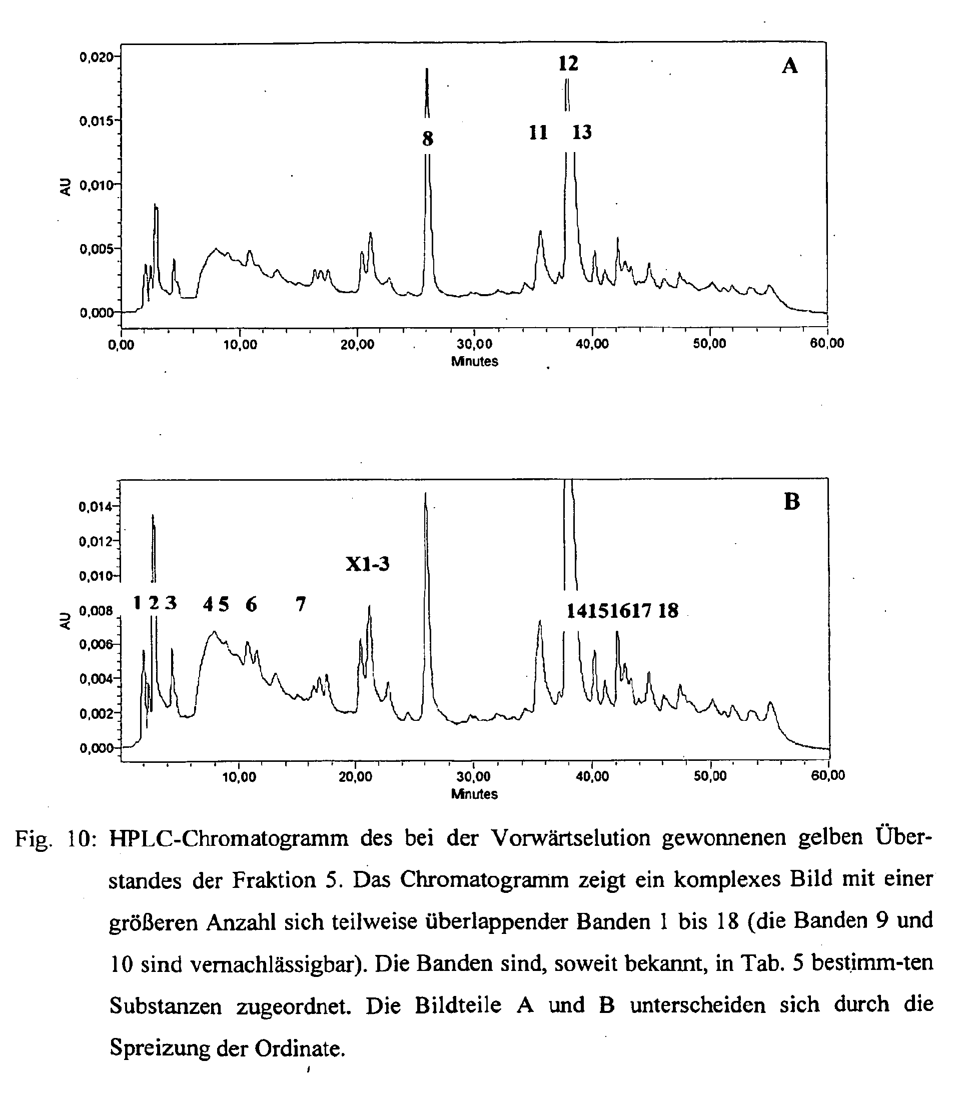 nimmt nach längerem Stehen bei Raumtemperatur unter Luft eine braune Farbe an. Wird das Lösungsmittel im Verdampfer weitgehend entfernt, so kommt es schließlich zur Abscheidung einer grünen zähflüssigen Masse. Der verbleibende gelbe Überstand und der grüne Rückstand werden durch Dekantieren getrennt.

Das HPLC-Chromatogramm des Überstandes (siehe Fig. 10) zeigt ein komplexes Bild mit einer größeren Anzahl kleiner und großer, teilweise überlappender Banden (1 bis 18). Der Bandenkomplex X1-3 ist besonderes gekennzeichnet und noch nicht zugeordnet. Die wesentlichen Charakteristika, insbesondere die prozentualen Mengenanteile, sind in Tab. 6 dargestellt.

Aus Tab. 6 ist zu entnehmen, daß der Überstand der Fraktion 5 Anteile in Höhe von 8,9 Gew.-% des Hopfenflavonoids Xanthohumol (Bande 8), in Höhe von 39,8 Gew.-% der α-Bittersäu-

**Tab. 6: Charakterisierung der im HPLC-Chromatogramm aufgelösten Banden (molekulare Komponenten) des Überstandes der Fraktion 5 (identifiziert im Absorptionsmaximum bei 325 nm).**

| Elutionsbande Nr. | Retentionszeit (min) | Mengenanteil Gew.-(%) | Substanz |
|---|---|---|---|
| 1 | 2,1 | 3,4 | unbekannt |
| 2 | 2,9 | 4,7 | unbekannt |
| 3 | 3,1 | 5,7 | unbekannt |
| 4 | 7,2 | 10,7 | unbekannt |
| 5 | 8,8 | 1,2 | unbekannt |
| 6 | 11,2 | 1,6 | Isoxanthohumol |
| 7 | 18,5 | 1,2 | unbekannt |
| 8 | 24,9 | 8,9 | Xanthohumol |
| 11 | 35,2 | 6,5 | Cohumulon |
| 12 | 37,4 | 17,1 | Humulon |
| 13 | 37,9 | 16,2 | Adhumulon |
| 14 | 38,9 | 1,8 | unbekannt |
| 15 | 40,6 | 0,8 | unbekannt |
| 16 | 42,6 | 2,7 | Colupulon |
| 17 | 44,1 | 1,1 | Lupulon + Adlupulon |
| 18 | 45,3 | 1,0 | unbekannt |
| 19 | 46,4 | 1,1 | unbekannt |
| X1-3 | 20-23 | 9,3 | Iso-α-Bittersäuren |
| Σ | | 95,0 | |

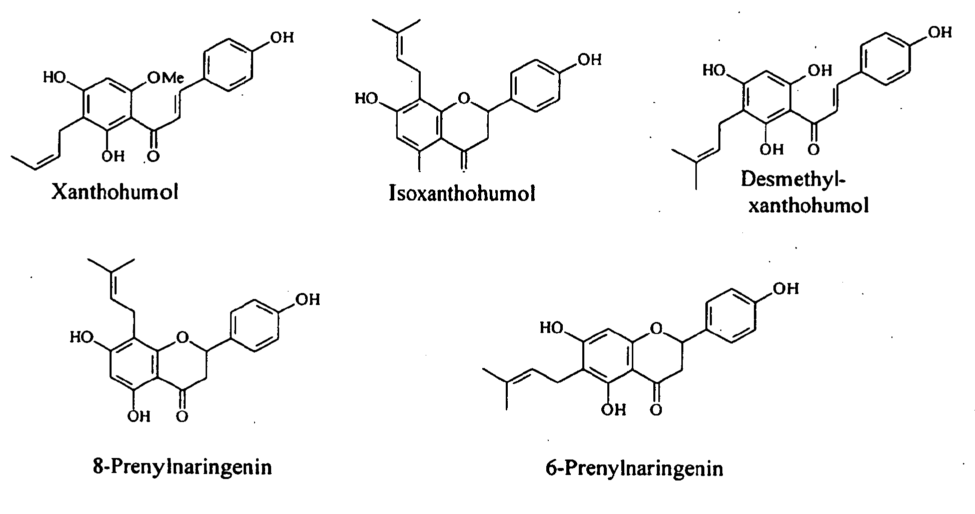

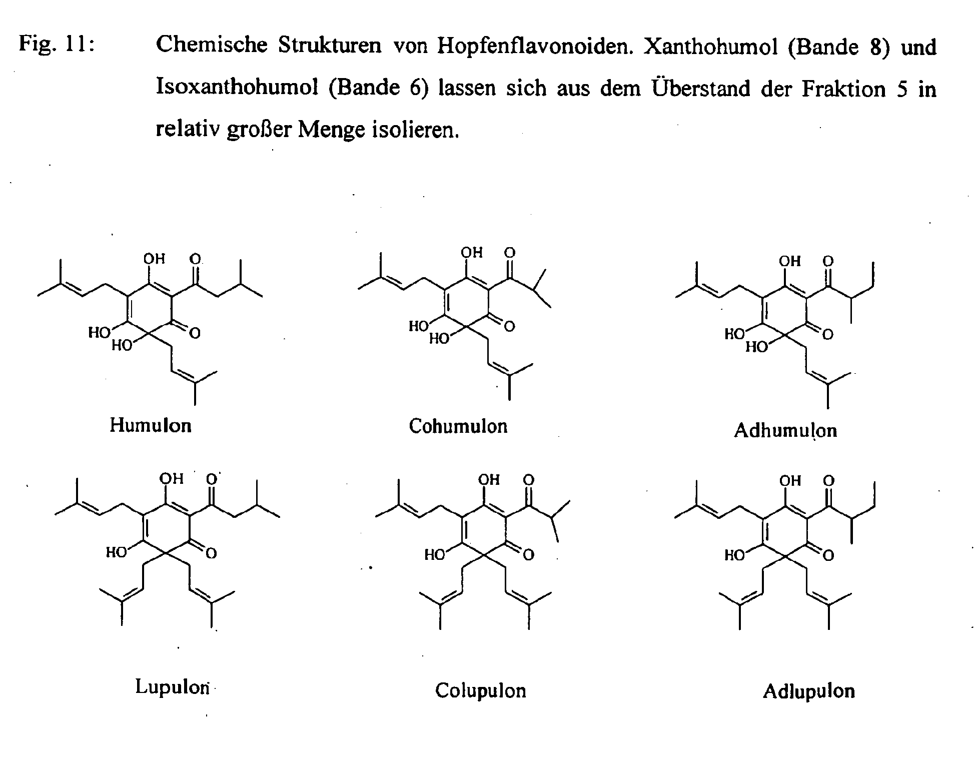

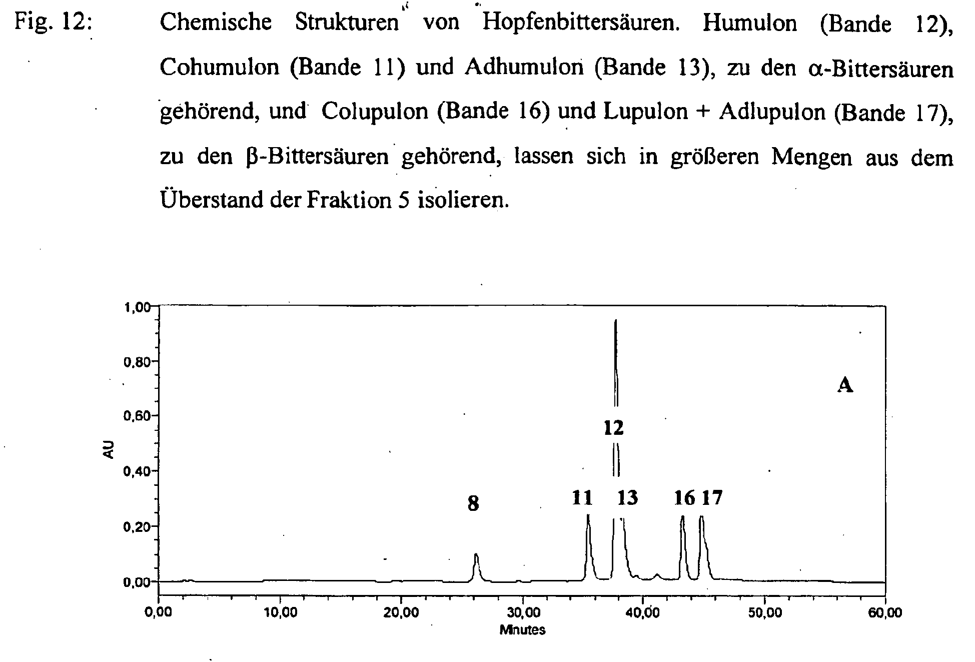

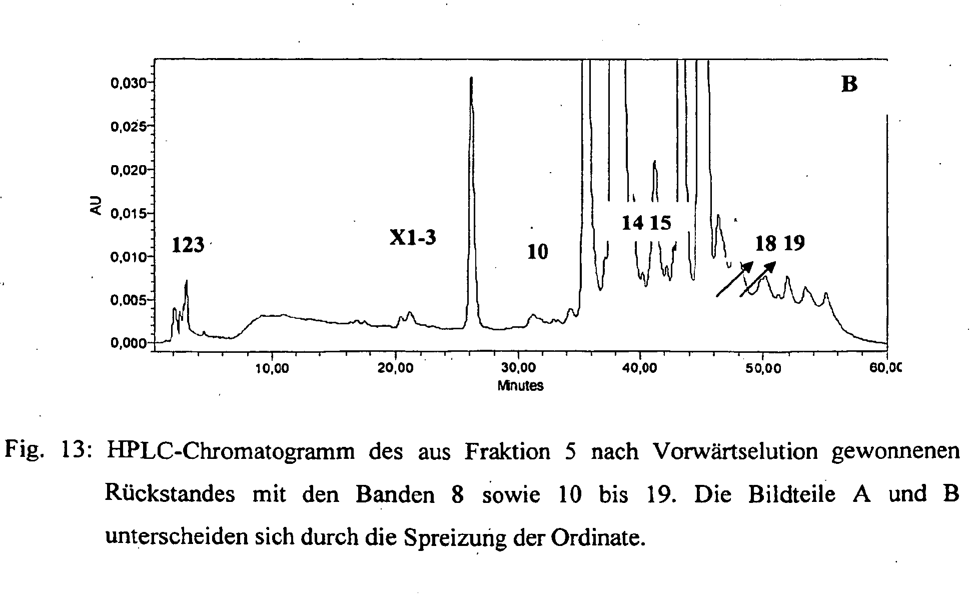

ren, nämlich Cohumulon (11), Humulon (12), Adhumulon (13), und in Höhe von 4,6 Gew.-% der β-Bittersäuren, nämlich Colupulon (16) und Lupulon + Adlupulon (17), enthält. Die chemischen Strukturen der Flavonoide sind in Fig. 11 und die der Hopfenbitterstoffe in Fig. 12 dargestellt.
Im HPLC-Chromatogramm des braunen Rückstandes repräsentiert die Bande 8 das Xanthohumol, welches 2,0 Gew.-% des Rückstandes ausmacht (siehe Fig.13). Die Banden 11 bis 13 sind den α-Bittersäuren Cohumulon (11), Humulon (12), und Adhumulon(13) mit einem Gesamtanteil von 52,7 Gew.-% zuzuschreiben. Die Banden 15 bis 17 lassen sich den β-Bittersäuren Colupulon (16) und Lupulon + Adlupulon (18) mit einem Gesamtgehalt von 35 Gew.-% zuordnen.

**Tab. 7: Charakterisierung der im HPLC-Chromatogramm aufgelösten Banden (molekulare Komponenten) des aus der Fraktion 5 nach Vorwärtselution gewonnenen Rückstandes (gemessen im Absorptionsmaximum bei 325 nm).**

| Elutionsbande Nr. | Retentionszeit (min) | Mengenanteil Gew.-(%) | Substanz |
|---|---|---|---|
| 1 | 2,1 | 0,2 | unbekannt |
| 2 | 2,9 | 0,1 | unbekannt |
| 3 | 3,1 | 0,3 | unbekannt |
| 8 | 24,9 | 2,0 | Xanthohumol |
| 10 | 30,9 | 0,1 | Xanthohumol D |
| 11 | 35,2 | 9,3 | Cohumulon |
| 12 | 37,4 | 32,6 | Humulon |
| 13 | 37,9 | 10,8 | Adhumulon |
| 14 | 38,9 | 1,1 | unbekannt |
| 15 | 40,6 | 2,3 | unbekannt |
| 16 | 42,6 | 12,1 | Colupulon |
| 17 | 44,1 | 20,6 | Lupulon + Adlupulon |
| 18 | 45,3 | 1,5 | unbekannt |
| 19 | 46,4 | 1,3 | unbekannt |
| X1-3 | 20-23 | 0,2 | Iso-α-Bittersäuren |
| Σ | | 93,2 | |

Die wesentlichen Charakteristika, insbesondere die prozentualen Mengenanteile, sind in Tab. 7 dargestellt. Die Hopfeninhaltsstoffe, insbesondere die mono- und diprenylierten Hopfenflavonoide, deren Banden im Zeitinterwall 8 bis 24 Minuten des HPLC-Chromatogrammes auftreten, sind praktisch nicht nachweisbar. In der Fraktion 5 sind also die Elutionsbanden 8 bis 18 (bei 325 nm gemessen) von allein entscheidender Bedeutung.

In der Vorwärtselution der Fraktion 5 wird im Rückstand das Produkt "Hopfenbitter pur" (siehe folgende Kapitel) als Bande 8 - 18 gewonnen. Dieses Produkt entspricht der Fraktion 5 (Bande 8 - 18), die aus der Rückwärtselution gewonnen wird.

Beschreibung der Fraktionen 5 bis 1 nach Rückwärtselution und Nachweis der innerhalb dieser Fraktionen auftretenden Inhaltsstoffe
Die Fraktionen 5 bis 1, die mit Hilfe der zuvor beschriebenen Rückwärtselution gewonnen werden, zeigen eine in bestimmten Grenzen relativ konstante und reproduzierbare Zusammensetzung ihrer molekularen Komponenten.
Die Fraktion 5 enthält sämtliche in 90 - 97%igem Ethanol gelösten Hopfeninhaltsstoffe, also mindestens 18 Komponenten (siehe Fig. 14). Die im HPLC-Chromatogramm aufgelösten Banden werden entsprechend den ansteigenden Elutionszeiten von 1 bis 18 durchnumeriert.
In der Fraktion 5 nach Rückwärtselution haben die Elutionsbanden 11 bis 18 (bei 325 nm ermittelt) aufgrund ihrer Menge eine herausragende Bedeutung. Die wesentlichen Identifikationsmerkmale, insbesondere die prozentualen Mengenanteile, sind in Tabelle 8 dargestellt.

Entsprechend den Retentionszeitintervallen lassen sich die verschiedenen Produkte klassifizieren: Im Zeitintervall von 22 bis 33 Minuten treten die Banden 8, 9 und 10 auf. In diesen drei Banden erscheinen Xanthohumol (8) und Xanthohumol D (9). Die Substanz der Bande 10 ist noch nicht identifiziert.

Im Zeitintervall zwischen 33 und 55 Minuten lassen sich die α-Bittersäuren Cohumulon (11), Humulon (12), Adhumulon (13), Prehumulon und Posthumulon nachweisen. Die β-Bittersäuren Colupulon (16), Lupulon + Adlupulon (17), Prelupulon und Postlupulon treten im Zeitsegment der Banden 15 bis 17 auf. Die Banden 14 und 18 sind noch nicht bestimmten Stoffen zugeordnet.

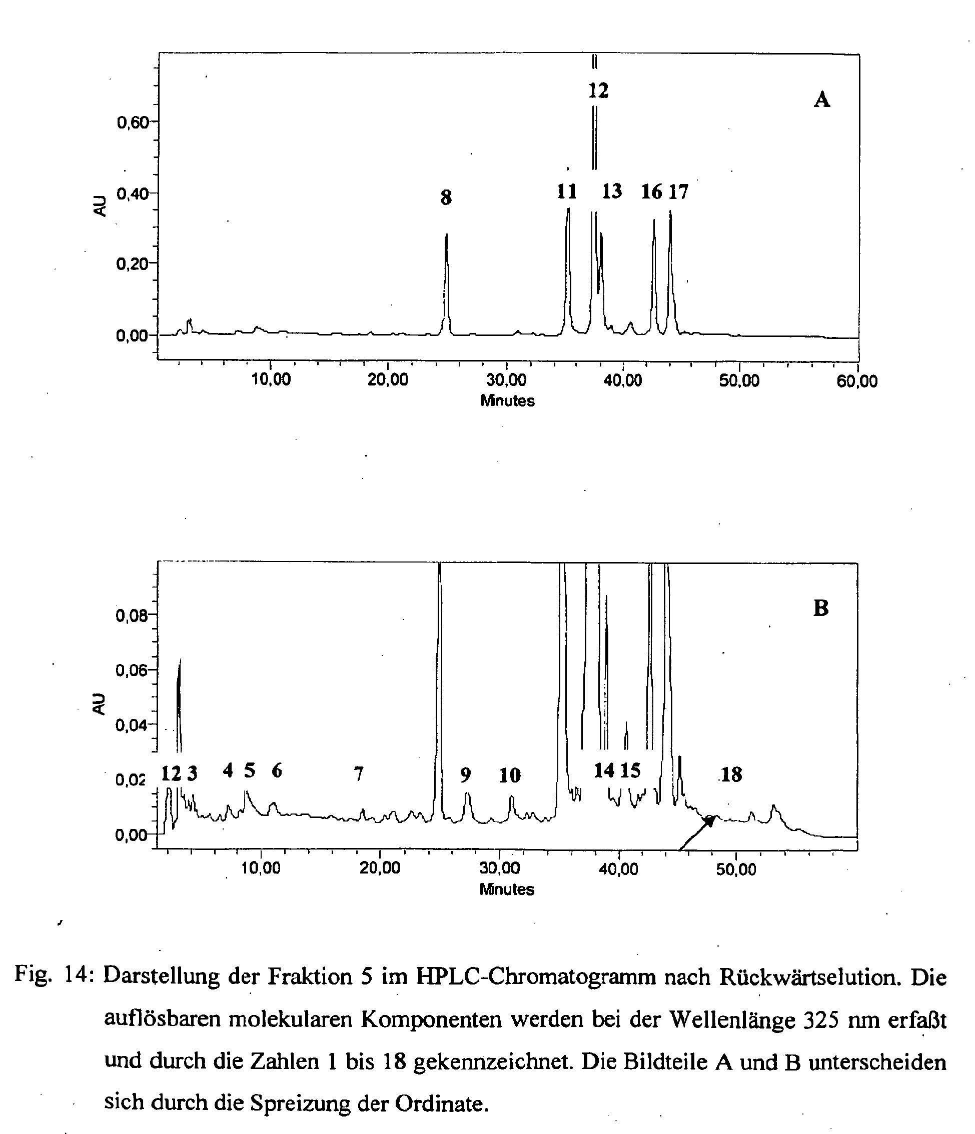

Die Fraktion 5 ist eine grün-braun gefärbte, viskose Lösung. Wenn das Lösungsmittel weitgehend entfernt wird, erhält man eine grün-braune Masse von zäher Konsistenz. Die Fraktion 5 liefert demzufolge in ihrer Gesamtheit das Produkt "Hopfenbitter natur". Wenn man die Banden 11 bis 18 von den übrigen Komponenten durch Fraktionieren trennt, erhält man das Produkt "Hopfenbitter pur". Die Bande 8 bildet nach Fraktionierung das Produkt "Hopfen pharma".

Die Fraktion 5 (Hopfenbitter natur), aus dem Gemisch aller Komponenten 1 bis 18 bestehend (siehe Tab. 8), stellt ein Produkt dar, das dem deutschen Reinheitsgebot entspricht und als wäßrige, alkoholische oder nach Eindickung als viskose Lösung dem Bier zugesetzt oder als Wirkstoff bzw. als Zusatzstoff in der Kosmetik- und Lebensmittel-Branche verwendet werden kann.

**Tab. 8: Identifikationsmerkmale der wesentlichen Komponenten der Fraktion 5 nach Rückwärtselution aufgrund von Absorptionsmessungen im Maximum bei 325 nm.**

| Elutionsbande Nr. | Retentionszeit (min) | Mengenanteil Gew.-(%) | Substanz |
|---|---|---|---|
| 1 | 2,1 | 0,5 | unbekannt |
| 2 | 2,9 | 0,6 | unbekannt |
| 3 | 3,1 | 0,5 | unbekannt |
| 4 | 7,2 | 0,5 | unbekannt |
| 5 | 8,8 | 1,1 | unbekannt |
| 6 | 11,2 | 0,2 | Isoxanthohumol |
| 7 | 18,5 | 0,2 | unbekannt |
| 8 | 24,9 | 8,7 | Xanthohumol |
| 9 | 26,9 | 0,1 | unbekannt |
| 10 | 30,9 | 0,4 | Xanthhumol D |
| 11 | 35,2 | 14,0 | Cohumulon |
| 12 | 37,4 | 37,0 | Humulon |
| 13 | 37,9 | 9,1 | Adhumulon |
| 14 | 38,9 | 0,7 | unbekannt |
| 15 | 40,6 | 1,6 | unbekannt |
| 16 | 42,6 | 9,5 | Colupulon |
| 17 | 44,1 | 12,7 | Lupulon + Adlupulon |
| 18 | 45,3 | 0,1 | unbekannt |
| Σ | | 97,4 | |

Das mit Hilfe der Fraktion 5 gewonnene Produkt enthält nahezu alle Hopfeninhaltsstoffe, insbesondere einen Mengenanteil in Höhe von 60,1 % an α-Bittersäuren (siehe Tab. 8, Bande 11 bis 13). Deshalb kann man die Fraktion 5 unter der Bezeichnung "Hopfenbitter natur" als Ethanol-Extrakt des Hopfens, der dem Deutschen Reinheitsgebot entspricht, verwenden.

Im Vergleich mit der Fraktion 5, die aus der Rückwärtselution gewonnen wird, führt die Fraktion 5, die aus der Vorwärtselution als Rückstand erhalten wird, zu einer vollständigen oder weitgehenden Abtrennung der im Hopfen vorkommenden Substanzen 6-Prenylnaringenin und 8-Prenylnaringenin. Die zuletzt genannten Stoffe stehen im Verdacht bzw. es wurde in Bezug auf diese Stoffe bereits nachgewiesen, daß sie im Menschen östrogene Wirkung besitzen. Im Falle des 8-Prenylnaringenin ist diese Wirkung ähnlich wie die des 17β-Östradiols. Um unerwünschte oder gesundheitsschädliche Wirkungen bei den Konsumenten mit Sicherheit zu vermeiden, besteht daher ein dringender Bedarf an Produkten auf Hopfenbasis, aus denen die hormon-ähnlichen Inhaltsstoffe ganz oder teilweise entfernt wurden. Diese Forderung gilt nicht nur für Biere oder Brauereiprodukte sondern auch für hopfenprodukthaltige Lebensmittel, Kosmetika und Pharmaka. Diese Forderung ist bei Verwendung von "Hopfenbitter pur" erfüllt.

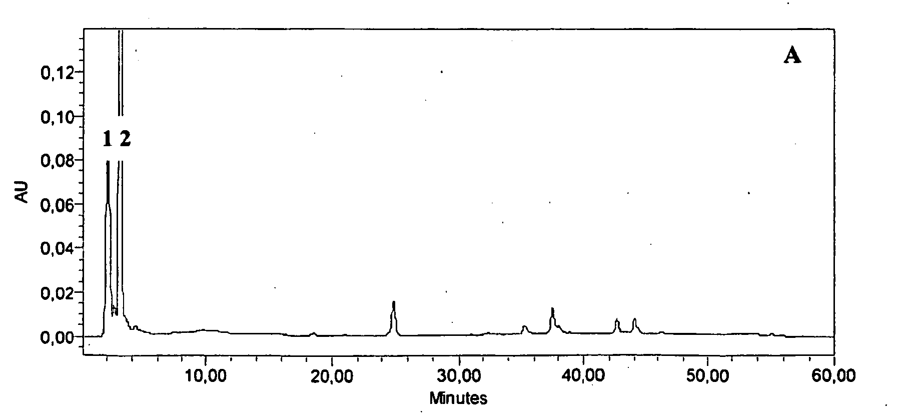

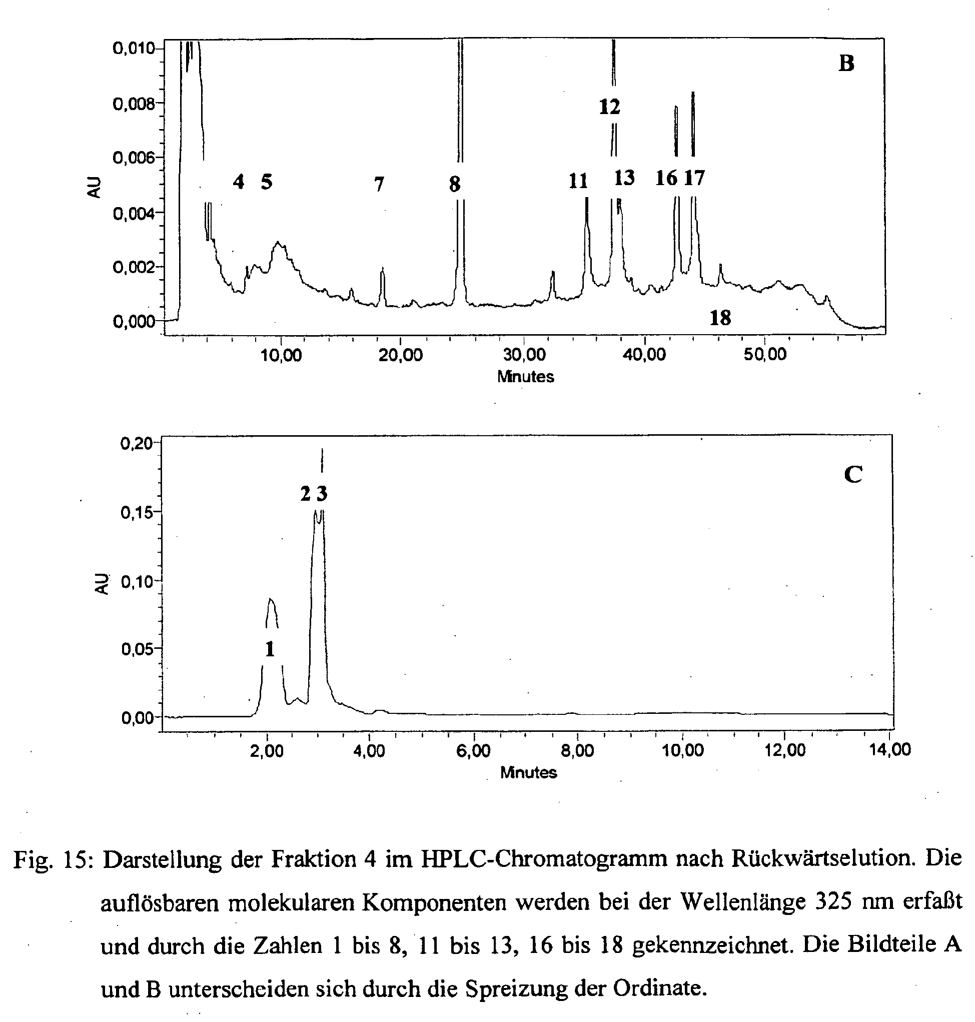

Die Fraktion 4, die durch Rückwärtselution gewonnen wird, enthält in sehr kleinen Mengen die Hopfenbittersäuren im Gemisch, nämlich die α-Bittersäuren Cohumulon (11), Humulon (12), Adhumulon (13), Prehumulon und Posthumulon sowie die β-Bittersäuren Colupulon (16), Lupulon + Adlupulon (17), Prelupulon und Postlupulon. Zusätzlich enthält die Fraktion 4 in kleinen Mengen Flavonoide, insbesondere Xanthohumol (8). In dieser Fraktion lassen sich jedoch mit dem größten Gewicht im Zeitintervall zwischen 1 und 6 Minuten die Elutionsbanden 1 bis 3 (bei 325 nm gemessen) nachweisen.

Die wesentlichen Identifikationsmerkmale, insbesondere die prozentualen Mengenanteile, sind in Tabelle 9 dargestellt. Die im HPLC-Chromatogramm (siehe Fig. 15) getrennten Komponenten werden entsprechend den ansteigenden Elutionszeiten von 1 bis 18, wie in Fraktion 5 durchnumeriert. In diesem Chromatogramm sind die Banden 6, 9, 10, 14 und 15 bei 325 nm praktisch nicht nachweisbar.

**Tab. 9: Darstellung der Identifikationsmerkmale der wesentlichen Komponenten der Fraktion 4 aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 30,0 | unbekannt |
| 2 | 2,9 | 28,7 | unbekannt |
| 3 | 3,1 | 20,2 | unbekannt |
| 4 | 7,2 | 0,1 | unbekannt |
| 7 | 18,5 | 0,4 | unbekannt |
| 8 | 24,9 | 5,0 | Xanthohumol |
| 9 | 26,9 | 0,1 | unbekannt |
| 11 | 35,2 | 1,3 | Cohumulon |
| 12 | 37,4 | 3,9 | Humulon |
| 13 | 37,9 | 1,0 | Adhumulon |
| 16 | 42,6 | 1,8 | Colupulon |
| 17 | 44,1 | 1,3 | Lupulon + Adlupulon |
| 18 | 45,3 | 0,1 | unbekannt |
| Σ | | 93,9 | |

Die Banden 1, 2 und 3, die in Fraktion 4 nach Rückwärtselution auftreten, nehmen einen Anteil in Höhe von 78,8 % ein. Andererseits zeigt die Fraktion 4, die bei der Vorwärtselution als Rückstand gewonnen wird, eine vollständige bzw. weitgehende Abtrennung der im Hopfen vorkommenden Substanzen 6-Prenylnaringenin und 8-Prenylnaringenin. Außerdem wird ein großer Mengenanteil in Höhe von 86.2 % an Hopfenbittersäuren, in Höhe von 67,0 % an α-Bittersäuren und in Höhe von 19,2 % an β-Bittersäuren nachgewiesen. Der Rückstand der Fraktion 4 entspricht dem Rückstand der Fraktion 5 und wird deshalb vereinigt. So erhält man das Produkt "Hopfenbitter pur". Das Filtrat der Fraktion 5 und der Fraktion 4, gewonnen aus der Vorwärtselution, wird mit der Fraktion 3, die aus der Vorwärtselution gewonnen werden vereinigt.

Die Fraktion 3, die durch Rückwärtselution erhalten wird, ist nicht mit der Fraktion 3, die durch Vorwärtselution gewonnen wird, identisch oder vergleichbar. Sie enthält nämlich im Wesentlichen die Banden 1 und 2 mit einem Anteil von 80,5 Gew.-% (siehe Fig. 16 und Tab. 10). Diese beiden Banden sind jedoch noch nicht bestimmten, charakterisierten Substanzen zugeordnet (siehe Tab. 10). Die Banden 3 bis 6 und 7 sind nicht in der Fraktion 3 nach Rückwärtselution nachweisbar. Die Bande 8 enthält das Flavonoid Xanthohumol. Die Fraktion 3 enthält außerdem in kleinen Mengen die α-Bittersäuren Cohumulon (11), Humulon

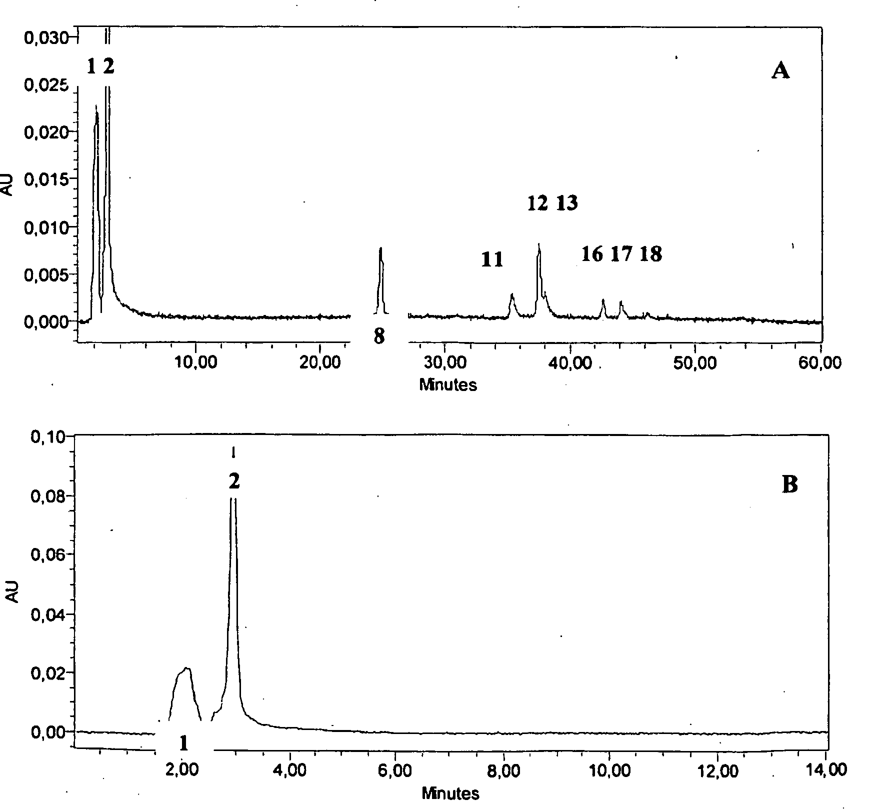

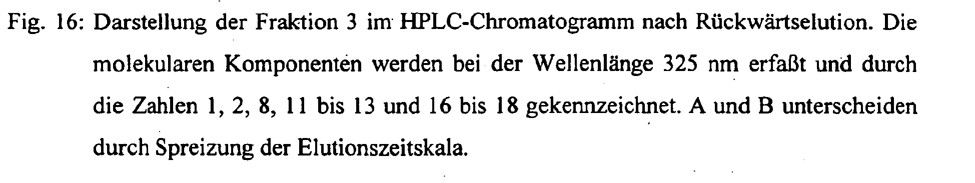

**Tab. 10: Identifikationsmerkmale der wesentlichen molekularen Komponenten in der Fraktion 3 aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 29,1 | unbekannt |
| 2 | 2,9 | 51,4 | unbekannt |
| 8 | 24,9 | 1,4 | Xanthohumol |
| 11 | 35,2 | 2,0 | Cohumulon |
| 12 | 37,4 | 6,6 | Humulon |
| 13 | 37,9 | 1,0 | Adhumulon |
| 16 | 42,6 | 2,5 | Cohumulon |
| 17 | 44,1 | 3,0 | Lupulon + Adlupulon |
| Σ | | 97,0 | |

(12), Adhumulon (13) sowie die β-Bittersäuren Colupulon (16) und Lupulon + Adlupulon (17) (siehe Tab. 10). Die Fraktion 3 ist eine gelb gefärbte Lösung und schäumt beim Entzug des Lösungsmittels stark. Praktisch ist ein vollständiger Lösungsmittelentzug (im Rotationsverdampfer) unmöglich. Nach dem vollständigen Entzug des Lösungsmittels mit Hilfe der Gefriertrocknung erhält man einen braun gefärbten Feststoff.

Die Fraktion 2, die im 4. Rückwärtselutionsschritt gewonnen wird, zeigt in der HPLC-Chromatographie die Banden 1, 2, 8, 11 bis 13, 16 und 17 (siehe Fig. 17). In dieser Fraktion 2 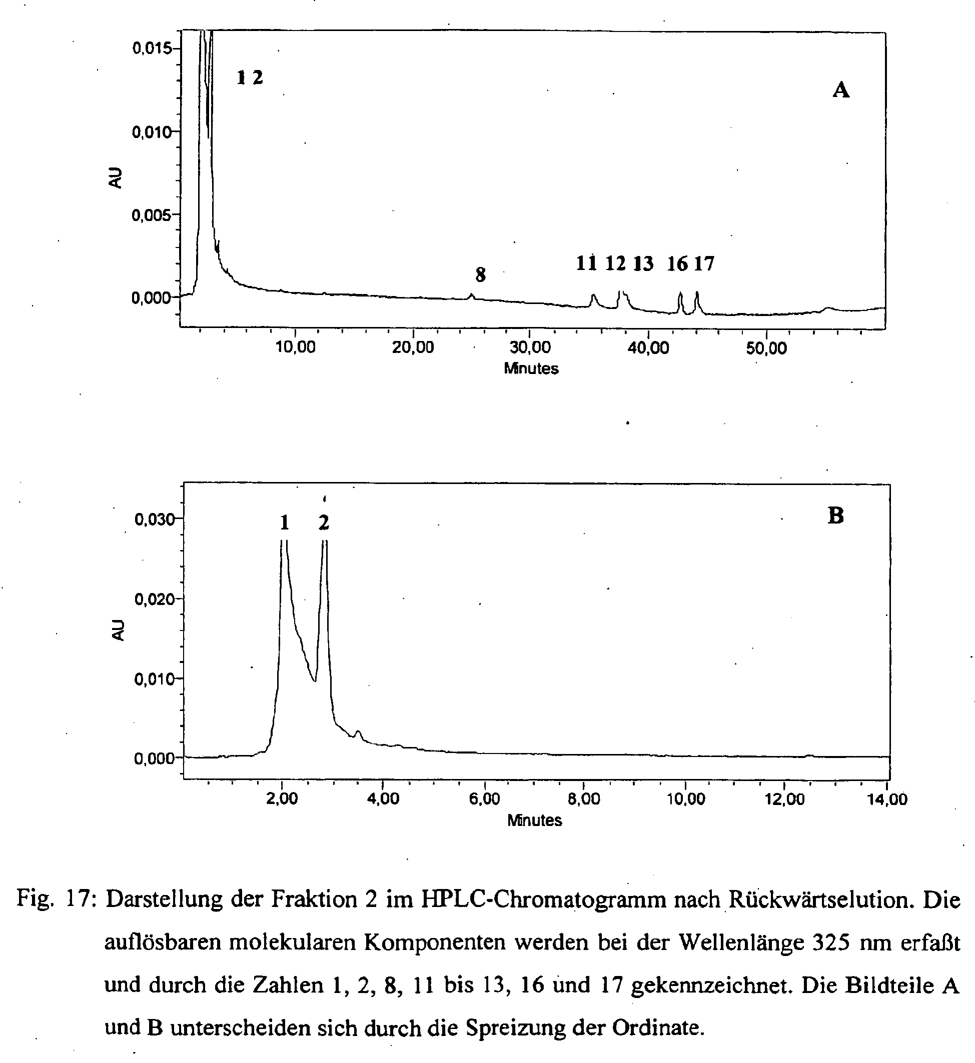 lassen sich im Wessentlichen schwer wasserlösliche Anteile der α-Bittersäuren Cohumulon (11), Humulon (12) und Adhumulon (13) sowie der β-Bittersäuren Colupulon (16) und Lupulon + Adlupulon (17) nachweisen. Ferner wird ein kleinerer Anteil des Flavonoids Xanthohumol (8) identifiziert. Die Hauptbanden 1 und 2 sind molekular noch nicht zugeordnet und charakterisiert. Die prozentualen Mengenanteile der Banden 1, 2, 8, 11 bis 13, 16 und 17 sind in Tabelle 11 dargestellt.

**Tab. 11: Identifikationsmerkmale der wesentlichen Komponenten der Fraktion 2 nach Rückwärtselution auf der Basis von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 59,7 | unbekannt |
| 2 | 2,9 | 31,4 | unbekannt |
| 11 | 35,2 | 2,6 | Cohumulon |
| 12 | 37,4 | 2,1 | Humulon |
| 16 | 42,6 | 1,6 | Colupulon |
| 17 | 44,1 | 1,1 | Lupulon + Adlupulon |
| Σ | | 98,5 | |

Die mit reinem Wasser in der Rückwärtselution ausgewaschene Fraktion 1 (siehe Fig. 18) zeigt im HPLC-Chromatogramm mindestens zwei Banden, nämlich die Banden 1 und 2. Die 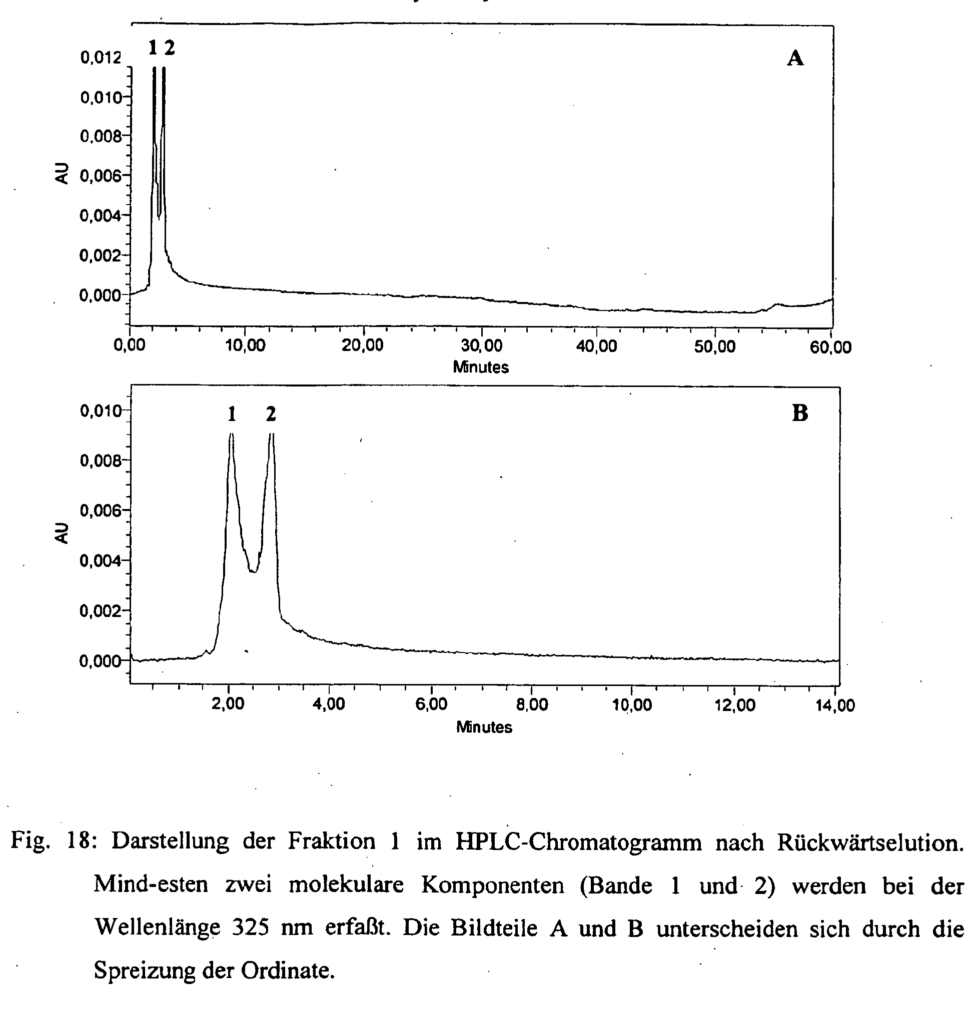 Bande 1 entspricht einem Mengenanteil von 57,0 Gew.-%, die Bande 2 entspricht einem Mengenanteil von 43,0 Gew.-% in der Fraktion 1 (siehe Tab. 12).

**Tab. 12: Identifikationsmerkmale der wesentlichen Komponenten der Fraktion 1 aufgrund von Messungen im Absorptionsmaximum bei 325 nm.**

| Elutionsbande Nr. | Retentionszeit (min) | Mengenanteil Gew.-% | Substanz |
|---|---|---|---|
| 1 | 2,1 | 56,96 | unbekannt |
| 2 | 2,9 | 43,04 | unbekannt |
| Σ | | 100 | |

Ein Vergleich der Fraktionen 1 bis 5 unter alleiniger Berücksichtigung der Banden 1 bis 3 nach Vorwärts- und Rückwärtselution:

Im folgenden Kapitel (siehe Tab. 13) werden die in der Vorwärts- und Rückwärtselution ausgewaschenen Fraktionen 1 bis 5 unter alleiniger Berücksichtigung der Banden 1 bis 3 miteinander verglichen. Sowohl die in der Vorwärtselution als auch in der Rückwärtselution gewonnenen Fraktionen 1 bis 5 bzw. 5 bis 1 lassen sich mit einem sehr kostengünstigen Verfahren und mit relativ geringem Aufwand als Vorprodukte oder als ein, zwei, drei oder mehrere Produkte gewinnen.

**Tab. 13: Vergleich der Fraktionen 1 bis 5 bzw. 5 bis 1 nach Vorwärtselution bzw. nach Rückwärtselution unter alleiniger Berücksichtigung der Banden 1, 2 und 3, die im Zeitinterwall von 1 bis 6 Minuten auftreten. VL, Vorlauf; NL, Nachlauf; ÜS, Überstand; RS, Rückstand.**

| Fraktion Nr. | Vorwärtselution (Gew.-%) | Fraktion Nr. | Rückwärtselution (Gew.-%) |
|---|---|---|---|
| 1 | VL: 93,0 NL: 99,5 | 5 | 2,1 |
| 2 | 37,8 | 4 | 78,8 |
| 3 | 1,5 | 3 | 80,5 |
| 4 | ÜS:11,5 RS: 0,2 | 2 | 91,1 |
| 5 | RS: 0,7 ÜS: 14,9 | 1 | 100 |

Der große Vorteil der beiden oben beschriebenen Elutionsmethoden besteht in einer praktisch vollständigen Abtrennung der noch nicht einer molekularen Entität zugeordneten Banden 1, 2 und 3 von den übrigen bereits eindeutig identifizierten Hopfeninhaltsstoffen. Die Wirkung dieser Substanzen ist ebenfalls noch nicht genau bekannt. In der Fraktion 1 der Vorwärtselution und in der Fraktion 5 der Rückwärtselution lassen sich die Banden 1 bis 3 in reiner Form gewinnen und von den übrigen Hopfeninhaltsstoffen gut abtrennen.

Die Fraktionen 4, 3, 2 und 1, die durch Rückwärtselution gewonnen werden (siehe Tab. 13), bieten ebenfalls die Möglichkeit, die Banden 1 bis 3 von den übrigen Hopfeninhaltsstoffen fast vollständig zu entfernen. Nach Abtrennung der Banden 1 bis 3 können die Eluate nochmals auf Hopfen-Eigencellulose aufgetragen und nachgereinigt oder einer speziellen Chromatographie unterworfen werden, um Produkte mit verschiedenen Zusammensetzungen der Hopfeninhaltsstoffe zu erhalten.

Trennung der aus ihren Lösungsmitteln ausgefällten Hopfeninhaltsstoffe mittels der gereinigten Hopfen-Eigencellulose:
Nach der Anwendung der oben beschriebenen, erfindungsgemäßen Verfahren werden die in den bekannten Fraktionen eluierten Hopfeninhaltsstoffe wieder ausgefällt und auf die bereits gereinigte Hopfen-Eigencellulose aufgetragen. Auch andere aus Naturstoffen eluierte Substanzen lassen sich in einem Wasser-/Alkohohl-Gemisch resuspendierten und auf die gereinigte Hopfen-Eigencellulose auftragen. Nach der Auftragung werden die erfindungsgemäßen Elutionsschritte angewendet. Mit Hilfe dieses Verfahrens wird gezeigt, daß die Eigencellulose als stationäre Phase zur Elution und zur Trennung beliebiger Substanzen aus pflanzlichen Rohstoffen geeignet ist.

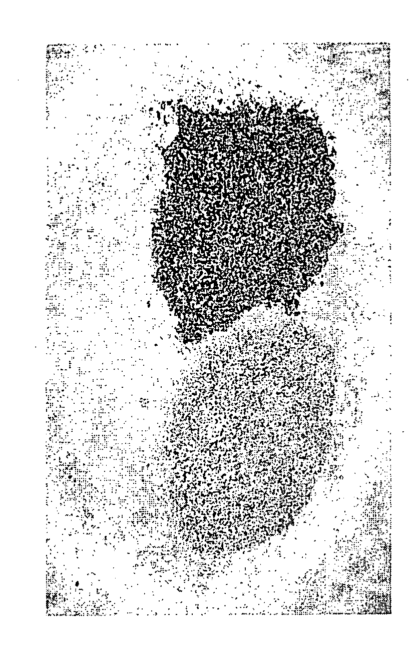

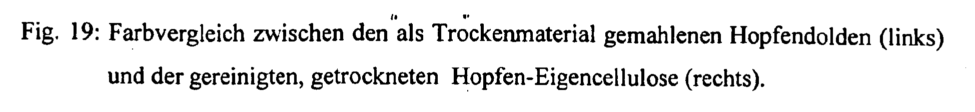

Die Vorbreitung der gereinigten Hopfen-Eigencellulose als stationäre Phase erfolgt beim Durchlaufen der bereits vorher beschriebenen Elutionsschritte. Nach dem Auswaschen aller Hopfeninhaltsstoffe bleibt im Eluator (B) die Hopfen-Eigencellulose zurück (siehe Fig. 19).

Abhängig vom angewandten Elutionsverfahren bleibt die gereinigte Hopfen-Eigencellulose zurück, die am Schluß des Elutionsverfahrens in Ethanol bzw.in Wasser aufgeschlämmt ist.

Die Hopfeninhaltsstoffe werden z.B. in Wasser oder in Alkohohl-/Wasser-Gemischen suspendiert, an die Hopfen-Eigencellulose gebunden und anschließend stufenweise, wie beschriebenen, durch Vorwärtselution ausgewaschen. Im Falle der Rückwärtselution sind die Hopfeninhaltsstoffe und Hopfen-Eigencellulose in Ethanol zu suspendieren.

Die Vorlage der gereinigten Hopfen-Eigencellulose, die Herstellung des an der Eigencellulose aufzutrennenden Extraktes, die Auftragung des Extraktes auf die Oberfläche der gereinigten Eigencellulose und die Durchführung der Elutionsschritte werden an Hand eines Beispieles beschrieben. Zunächst werden 0,2 kg gereinigte Hopfen-Eigencellulose als Pulver in den Elutionsbehälter (B) eingefüllt und auf einem 4 Liter-Nutschtrichter Pore 3 gelagert. Durch wiederholtes Anlegung eines Vakuums wird das Material verdichtet (B1). Es wird anschließend ein Hopfenextrakt dadurch hergestellt, daß 0,2 kg Hopfen der Sorte Taurus (Ernte Jahrgang 2005) der Hopfenveredelungsgesellschaft mit einer Schneidemühle pulverisiert werden. Danach wird das Pulver mit 70 bis 96%-igem Ethanol durch 24-stündiges Verrühren extrahiert. Der Extrakt wird auf 100 ml Volumen eingeengt und im Anschluß daran mit Wasser auf 1.000 ml verdünnt. Es resultiert ein konzentrierter Extrakt, nämlich die Lösung L1. Dieser Extrakt L1 wird auf die vorbereitete gereinigte Eigenzellulose (B1) langsam aufgetragen. Sobald der Extrakt vollständig in die Oberfläche der Eigenzellulose eingedrungen ist, wird die 1. Stufe der Elution des Hopfenextraktes an Eigencellulose mit reinem Wasser (Trinkwasserqualität) mit Hilfe des Lösungsmittels LM1 durchgeführt. Dabei wird LM1 so lange durch die Hopfen-Eigencellulose geleitet, bis sämtliche wasserlösliche Bestandteile vollständig von der Eigencellulose entfernt wird (siehe Fig. 6).

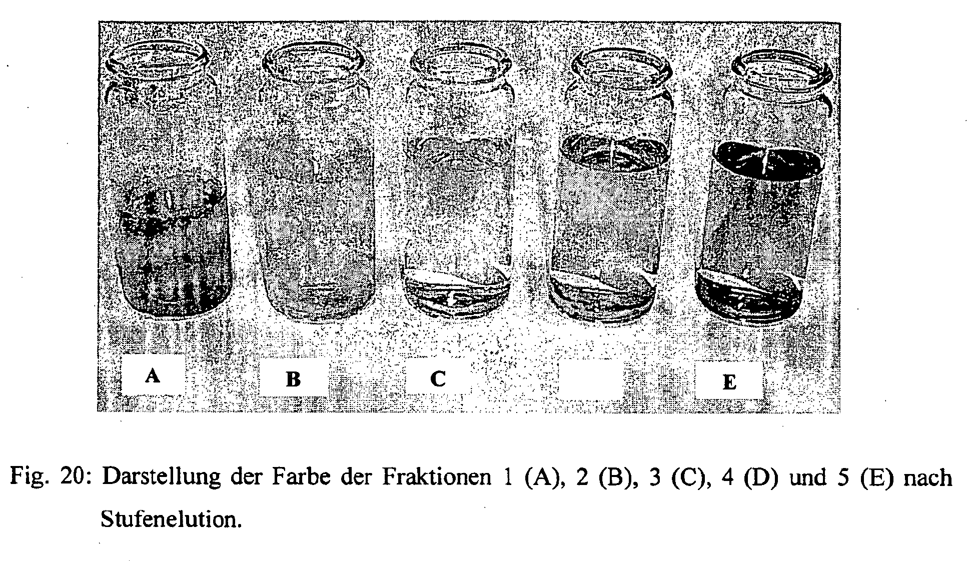

Die Durchführung des zweiten Elutionsschrittes erfolgt mit Hilfe des Lösungsmittels LM2, bestehend aus einer Ethanol/Wasser-Mischung mit einem Gehalt von 20 bis 40 Vol.- %, vorzugsweise mit einem Gehalt von 25 bis 35 Vol.-%, insbesondere mit einem Gehalt von 30 Vol.-% Ethanol. Durch Elution mit LM2 wird die Fraktion 2 (siehe Fig.7) als gelbe Lösung gewonnen.

Es folgt anschließend die Elution mit einem dritten Lösungsmittelgemisch LM3, bestehend aus einer Ethanol/Wasser-Mischung mit einem Anteil von 40 bis 70 Vol.-%, vorzugsweise 45 bis 60 Vol.-%, insbesondere 45 bis 55 Vol.-% Ethanol. Die Fraktion 3 ist eine gelbe Lösung (siehe Fig. 8)

In einem vierten Auswaschvorgang wird unter Einsatz des Lösungsmittelgemisches LM4, bestehend aus einer Ethanol/Wasser-Mischung mit einem Anteil von 70 bis 90 Vol.-%, vorzugsweise 75 bis 85 Vol.-%, und insbesondere 80 bis 85 Vol.-% Ethanol die Fraktion 4 ausgewaschen (siehe Fig. 9), die eine grün-gelbe farbe aufweist (siehe Fig. 20 D).

Abschließend kann noch eine Elution des Extraktionsguts mit einer Ethanol/Wasser-Mischung LM5, die 90 bis 96 Vol.-%, vorzugsweise 94 bis 96 Vol.-%, und insbesondere 96 Vol.-% Ethanol enthält, durchgeführt werden. Man erthält die Fraktion 5 (siehe Fig.9), die eine grüne Farbe aufweist (siehe Fig. 20 E).

Chromatographische Trennung der nach Vorwärtselution auf gereinigter Hopfen-Eigencellulose gewonnenen Fraktionen 1 bis 5:
Mit Hilfe der HPLC-Chromatographie werden in Fraktion 1 die Banden 1 bis 6, 8, 10, 11 bis 13 und 16 bis 17 identifiziert (siehe Fig. 21). Die Fraktion 1 enthält im Wesentlichen zwei Stoffklassen, nämlich einen weiteren, weniger wasserlöslichen Anteil (ca. 51,8 Gew.%) der α-Bittersäuren Cohumulon (11), Humulon (12) und Adhumulon (13) sowie einen kleineren

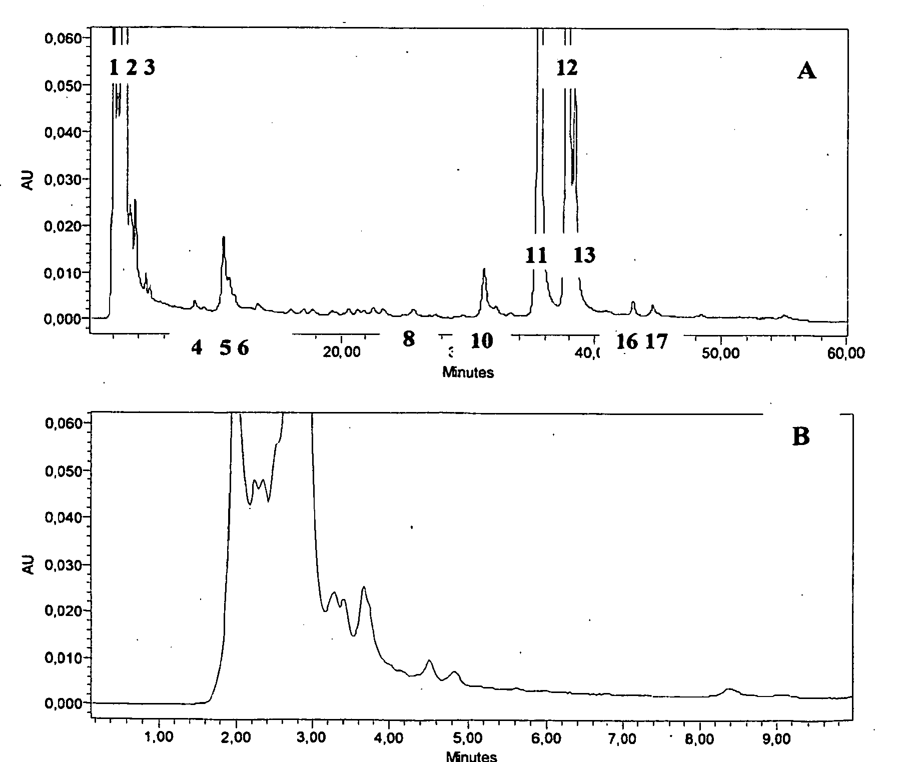

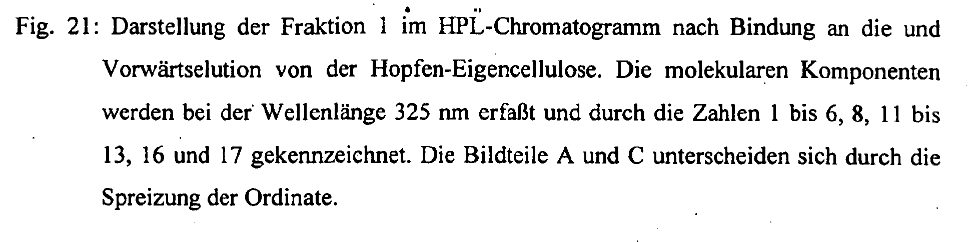

Anteil der Flavonoide, z.B. Xanthohumol (die Bande 8; 0,1 Gew.%) und Isoxanthohumol (die Bande 6: 0,7 Gew.%). Die Banden 5 und 10, die einen Anteil von 1,7 und 1,5 Gew.-% ausmachen, sind noch nicht charakterisierten Substanzen zugeordnet. In den Banden 16 und 17 werden die β-Bittersäuren Colupulon (16) Lupulon + Adlupulon (17) mit einem Anteil von 0,5 Gew.% nachgewiesen. Die Banden 1 bis 3 entsprechen einem Anteil von 36,0 Gew.% und sind noch nicht charakterisierten Substanzen zuzuordnen. Die wesentlichen Identifikationsmerkmale, insbesondere die prozentualen Mengenanteile, sind in Tabelle 14 dargestellt. Da als stationäre Phase Hopfen-Eigencellulose verwendet wird, sollte die auf diese Weise gewonnene Fraktion 1 hinsichtlich der prozentualen Zusammensetzung seiner Inhaltsstoffe mit der Zusammensetzung der aus der Vorwärtselution gewonnen Fraktion entsprechen. Dieses ist jedoch nicht der Fall. Die prozentuale Zusammensetzung der Inhaltsstoffe der Fraktion 1, die aus der Eigencellulose ausgewaschen wird, ähnelt eher der prozentualen Zusammensetzung der Inhaltsstoffe der Fraktion 2, die bei der natürlichen Vorwärtselution gewonnen wird. Möglicherweise ist der Ausgangsextrakt nicht völlig von Ethanol befreit.

**Tab. 14: Darstellung der Identifikationsmerkmale der wesentlichen Komponenten der Fraktion 1, die in der Vorwärtselution auf Eigencellulose gewonnen wird, aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 5,8 | unbekannt |
| 2 | 2,9 | 27,7 | unbekannt |
| 3 | 3,1 | 2,5 | unbekannt |
| 4 | 7,2 | 0,1 | unbekannt |
| 5 | 8,5 | 1,7 | unbekannt |
| 6 | 11,2 | 0,7 | Isoxanthohumol |
| 8 | 24,9 | 0,1 | Xanthohumol |
| 10 | 26,9 | 1,5 | unbekannt |
| 11 | 35,2 | 22,8 | Cohumulon |
| 12 | 37,4 | 23,3 | Humulon |
| 13 | 37,9 | 5,7 | Adhumulon |
| 16 | 42,6 | 0,3 | Colupulon |
| 17 | 44,1 | 0,2 | Lupulon + Adlupulon |
| Σ | | 92,4 | |

Die Fraktion 2, die nach Vorwärtselution der Hopfeninhaltsstoffe von der bereits gereinigten Hopfen-Eigencellulose erhalten wird, ist mit der Fraktion 2, die durch natürliche Vorwärtselution gewonnen wird, vergleichbar. Sie enthält nämlich im Wesentlichen die α-Bittersäuren Cohumulon (11), Humulon (12), Adhumulon (13) mit einem Anteil von 81,1 Gew.-% sowie die β-Bittersäuren Colupulon (16) und Lupulon + Adlupulon (17) mit einem

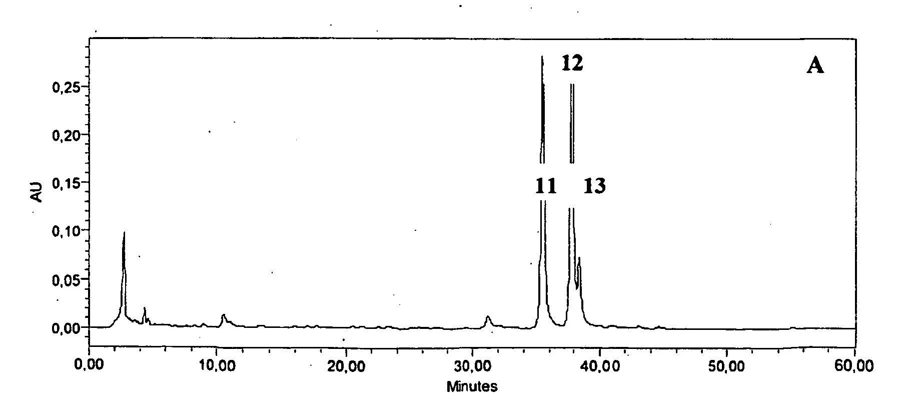

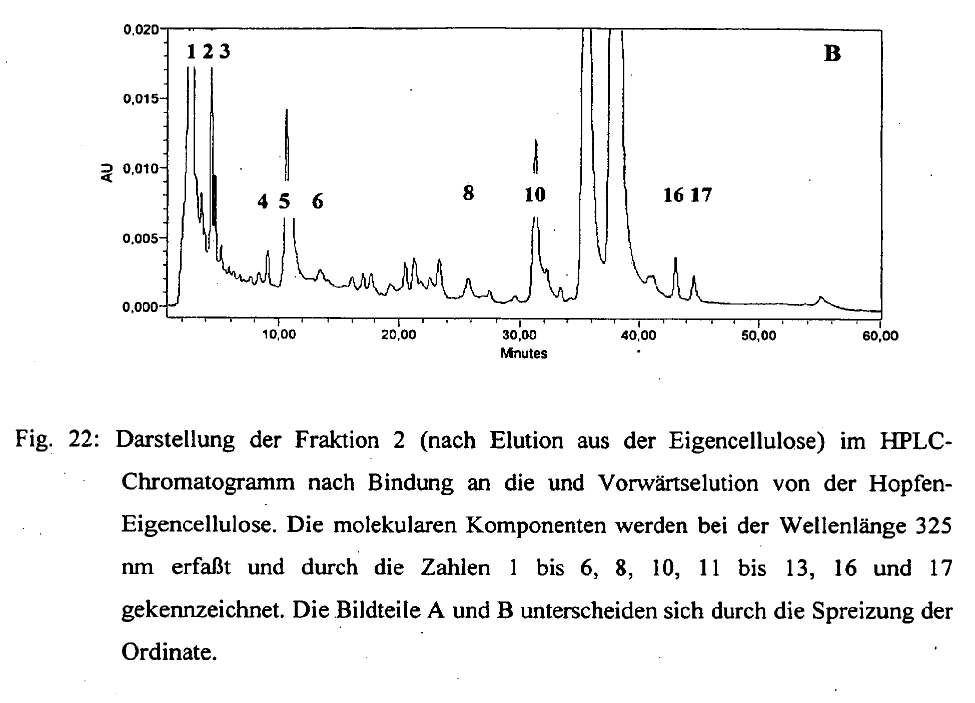

**Tab. 15: Darstellung der Identifikationsmerkmale der wesentlichen Komponenten der Fraktion 2, die in der Vorwärtselution auf Eigencellulose gewonnen wird, aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 11,5 | unbekannt |
| 2 | 2,9 | 0,7 | unbekannt |
| 3 | 3,1 | 1,3 | unbekannt |
| 4 | 7,2 | 0,1 | unbekannt |
| 5 | 8,5 | 0,2 | unbekannt |
| 6 | 11,2 | 1,0 | Isoxanthohumol |
| 7 | 18,5 | 0,1 | unbekannt |
| 8 | 24,9 | 0,1 | Xanthohumol |
| 10 | 26,9 | 1,4 | unbekannt |
| 11 | 35,2 | 32,6 | Cohumulon |
| 12 | 37,4 | 39,7 | Humulon |
| 13 | 37,9 | 8,8 | Adhumulon |
| 16 | 42,6 | 0,3 | Colupulon |
| 17 | 44,1 | 0,2 | Lupulon + Adlupulon |
| Σ | | 98,0 | |

Anteil von 0,5 Gew.-% (siehe Fig. 22 und Tab. 15). Die Fraktion 2 enthält außerdem die Banden 1, 2 und 3 mit einem Anteil von 13,5 Gew.-%. Diese Banden sind jedoch noch nicht bestimmten, charakterisierten Substanzen zugeordnet. Die Banden 4 bis 6 sind in der Fraktion 2 mit einem Anteil von 1,3 Gew.-% nachweisbar. Die Bande 8 enthält das Flavonoid Xanthohumol.

Die Fraktion 3, die durch Elution von der bereits gereinigten Hopfen-Eigencellulose erhalten wird, ist mit der Fraktion 3, die durch natürliche Vorwärtselution gewonnen wird, vergleichbar. In beiden Fällen stellt die Fraktion 3 ein Gemisch aus Hopfenbittersäuren (siehe Fig. 23 und Tab. 16) dar, das aus den α-Bittersäuren Cohumulon (11), Humulon (12), Adhumulon (13) mit einem Anteil von 75,4 Gew.% und aus den β-Bittersäuren Colupulon (16), Lupulon + Adlupulon (17) mit einem Anteil von 8,9 Gew.% besteht. Die Einzelfraktionen können in weitgehend reiner Form gewonnen werden. Weiterhin enthält die Fraktion 3 Flavonoide, insbesondere Xanthohumol (die Bande 8; 13,3 Gew.-%). Die in der Retentionszeit von 1,5 bis 24,0 Minuten erscheinenden Banden machen zusammen 0,8 Gew.-% aus. Sie repräsentieren Spuren der auch in den Fraktionen 1 und 2 vorkommenden Substanzen.

Im HPLC-Chromatogramm der Fraktion 4 repräsentiert die Bande 8 das Xanthohumol, welches 5,3 Gew.-% der Fraktion 4 ausmacht (siehe Fig. 24 und Tab. 17). Die Banden 11 bis 13 sind den α-Bittersäuren Cohumulon (11), Humulon (12), und Adhumulon (13) mit einem

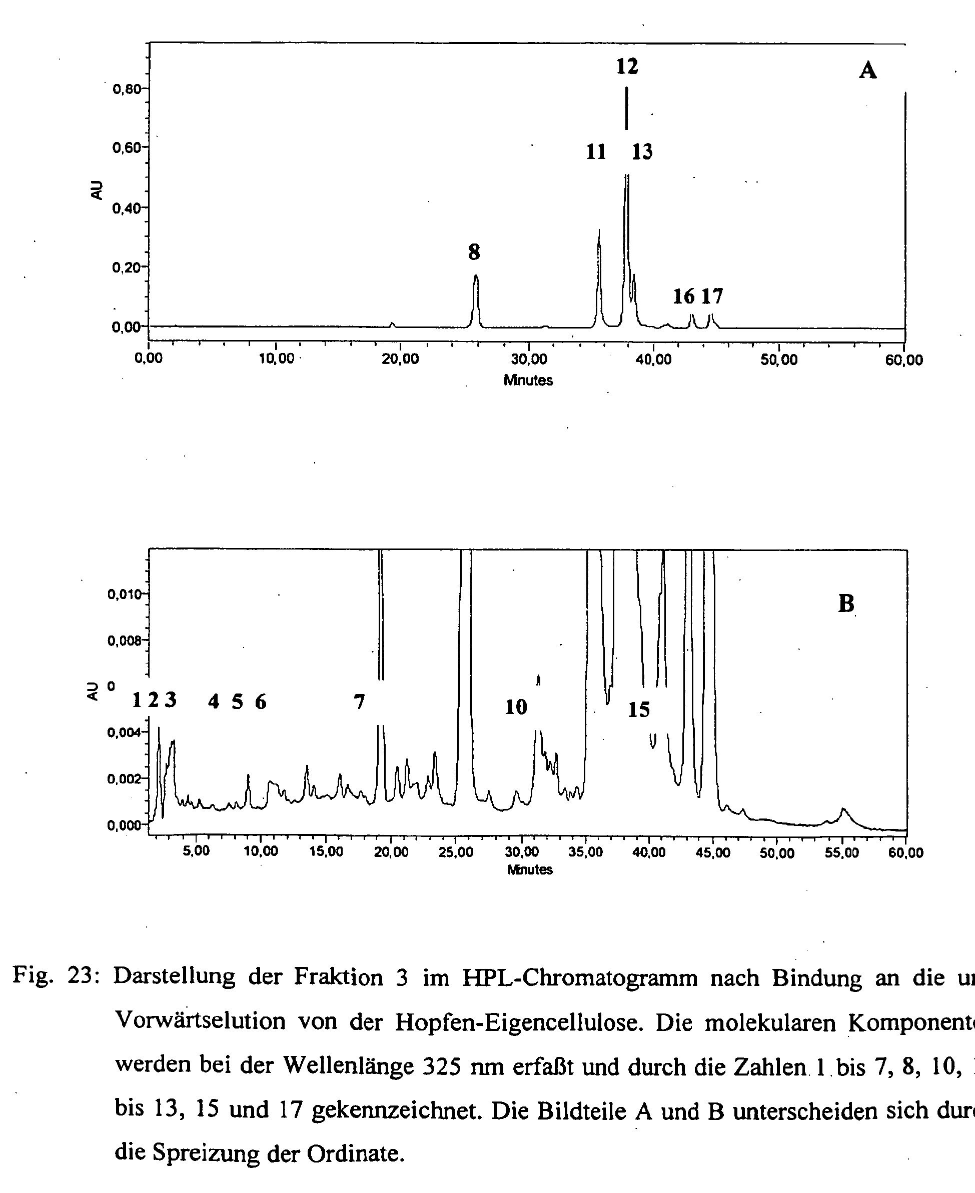

**Tab. 16: Darstellung der Identifikationsmerkmale der wesentlichen Komponenten der Fraktion 3, die in der Vorwärtselution auf Eigencellulose gewonnen wird, aufgrund von Messungen im Absorptionsmaximum bei 325 nm.**

| Elutionsbande Nr. | Retentionszeit (min) | Mengenanteil Gew.-(%) | Substanz |
|---|---|---|---|
| 1 | 2,1 | 0,2 | unbekannt |
| 2 | 2,9 | 0,2 | unbekannt |
| 3 | 3,1 | 0,1 | unbekannt |
| 4 | 7,2 | 0,1 | unbekannt |
| 5 | 8,5 | 0,1 | unbekannt |
| 6 | 11,2 | 0,1 | Isoxanthohumol |
| 7 | 18,5 | 0,8 | unbekannt |
| 8 | 24,9 | 13,3 | Xanthohumol |
| 10 | 26,9 | 0,3 | unbekannt |
| 11 | 35,2 | 18,9 | Cohumulon |
| | | | |
| 12 | 37,4 | 45,6 | Humulon |
| 13 | 37,9 | 10,9 | Adhumulon |
| 15 | 40,8 | 0,6 | unbekannt |
| 16 | 42,6 | 4,0 | Cohumulon |
| 17 | 44,1 | 3,9 | Lupulon + Adlupulon |
| Σ | | 99,1 | |

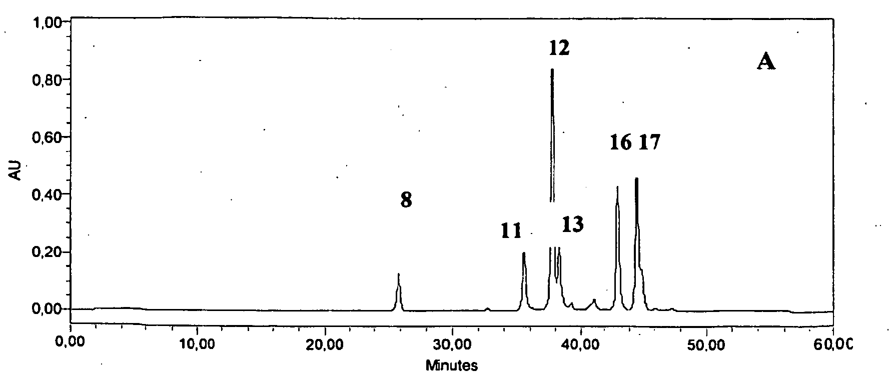

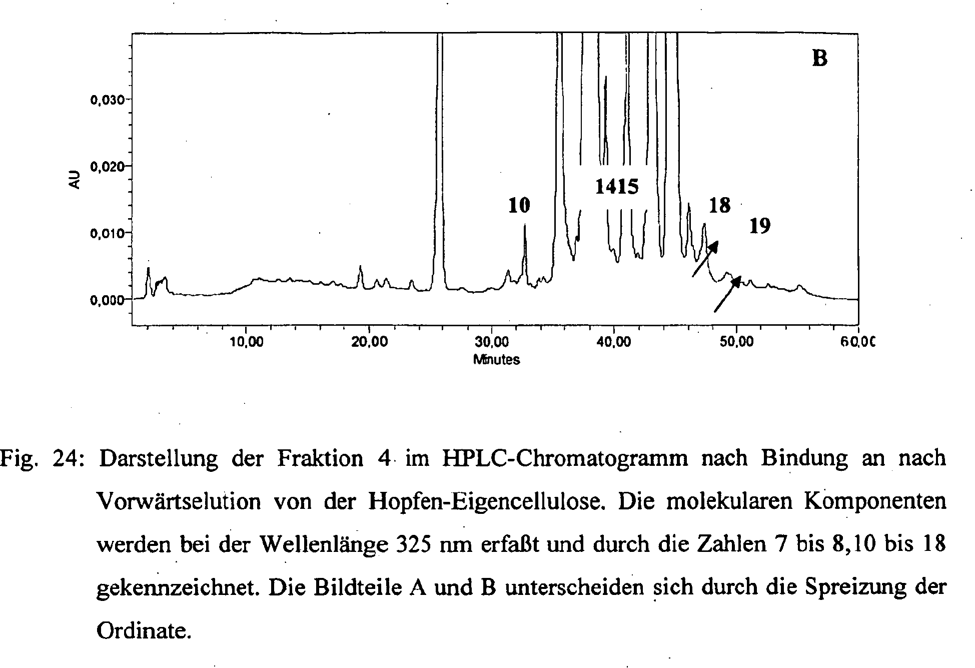

Gesamtanteil von 51,2 Gew.-% zuzuschreiben. Die Banden 16 und 17 lassen sich den β-Bittersäuren Colupulon (16) und Lupulon + Adlupulon (17) mit einem Gesamtgehalt von 32,8 Gew.-% zuordnen. Die wesentlichen Charakteristika, insbesondere die prozentualen Mengenanteile, sind in Tab. 17 dargestellt. Die Hopfeninhaltsstoffe, insbesondere die mono- und diprenylierten Hopfenflavonoide, deren Banden im Zeitintervall 8 bis 24 Minuten des HPLC-Chromatogrammes auftreten, sind praktisch nicht nachweisbar. In der Fraktion 4 sind also die Elutionsbanden 8, 10 bis 19 (bei 325 nm gemessen) von allein entscheidender Bedeutung.

**Tab. 17: Darstellung der Identifikationsmerkmale der wesentlichen Komponenten der Fraktion 4, die in der Vorwärtselution auf Eigencellulose gewonnen wird, aufgrund von Messungen im Absorptionsmaximum bei 325 nm.**

| Elutionsbande Nr. | Retentionszeit (min) | Mengenanteil Gew.-(%) | Substanz |
|---|---|---|---|
| 8 | 24,9 | 5,3 | Xanthohumol |
| 10 | 26,9 | 0,3 | unbekannt |
| 11 | 35,2 | 8,2 | Cohumulon |
| 12 | 37,4 | 34,0 | Humulon |
| 13 | 37,9 | 9,0 | Adhumulon |
| 14 | 38,9 | 1,1 | unbekannt |
| 15 | 40,8 | 2,3 | unbekannt |
| 16 | 42,6 | 15,8 | Cohumulon |
| 17 | 44,1 | 17,0 | Lupulon + Adlupulon |
| 18 | 45,0 | 4,5 | unbekannt |
| 19 | 47,0 | 0,4 | unbekannt |
| Σ | | 97,9 | |

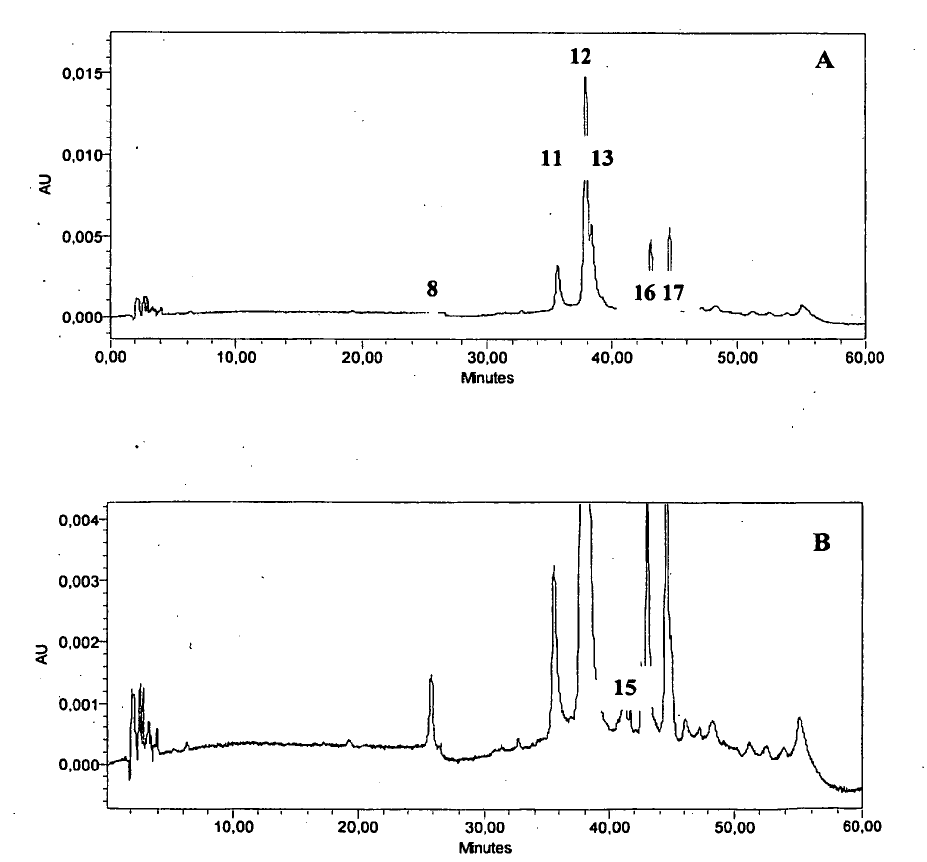

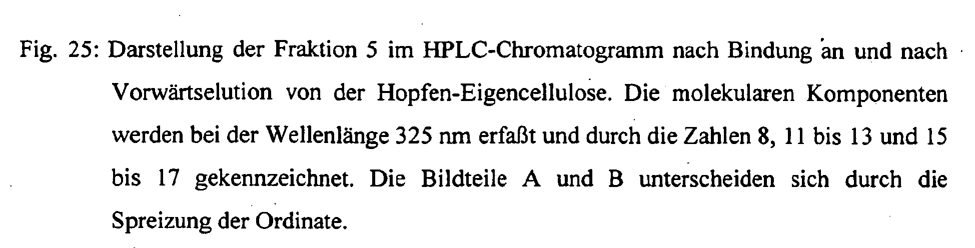

Diese Fraktion 4 ist hinsichtlich der Anzahl und der prozentualen Anteile der Inhaltsstoffe mit dem Rückstand der Fraktion 5, der durch Vorwärtselution gewonnen wird, vergleichbar. Der Rückstand der Fraktion 5 liefert nämlich das Produkt "Hopfenbitter pur", das die Banden 8 und 10 bis 18 enthält. Dieses Produkt entspricht zugleich der Fraktion 5, die mit den Banden 8 bis 18 durch Rückwärtselution gewonnen wird.

Die Fraktion 5, die durch Elution nach Bindung an die gereinigte Hopfen-Eigencellulose erhalten wird, ist bezüglich ihrer molekularen Komponenten mit dem Rückstand, der aus Fraktion 5 (siehe Fig. 13 und Tab. 7) durch Vorwärtselution gewonnen werden, vergleichbar. Die Fraktion 5 enthält nämlich im Wesentlichen die α-Bittersäuren Cohumulon (11), Humulon (12) und Adhumulon (13) mit einem Anteil von 69,2 Gew.-% sowie die β-Bittersäuren Colupulon (16) und Lupulon + Adlupulon (17) mit einem Anteil von 23,6 Gew.-% (siehe Fig. 25 und Tab. 18). Die Bande 8 enthält das Flavonoid Xanthohumol (entsprechend 2,3 Gew.-%).

**Tab. 18: Darstellung der Identifikationsmerkmale der wesentlichen Komponenten der Fraktion 5 nach Vorwärtselution auf gereinigter Eigencellulose durch Messung der Absorption im Maximum bei 325 nm.**

| Elutionsbande Nr. | Retentionszeit (min) | Mengenanteil Gew.-(%) | Substanz |
|---|---|---|---|
| 8 | 24,9 | 2,3 | Xanthohumol |
| 11 | 35,2 | 8,5 | Cohumulon |
| 12 | 37,4 | 41,8 | Humulon |
| 13 | 37,9 | 18,9 | Adhumulon |
| 15 | 40,9 | 1,8 | unbekannt |
| 16 | 42,6 | 9,0 | Colupulon |
| 17 | 44,1 | 14,6 | Lupulon + Adlupulon |
| Σ | | 96,9 | |

Aus den Rückständen der Fraktion 4 (siehe Banden 8, 10-18) und Fraktion 5 (siehe Banden 8, 10-19) wird das Produkt "Hopfenbitter pur" gewonnen. Dieses Produkt wird auch aus der Fraktion 5 (Bande 8-18), die bei der Rückwärtselution ausgewaschen wird, erhalten.

Die vorstehend beschriebenen Fraktionen 1 bis 5 können hinsichtlich ihrer einzelnen Inhaltsstoffe angereichert oder abgereichert werden. Spezifische Inhaltsstoffe können aus dem Fraktionen 1 bis 5 mit bestimmter Reinheit oder mit gewünschter Zusam-mensetzung isoliert werden, um diese anschließend im Falle von Anwendungen kontrolliert zuzusetzen. Für diese weitergehende An- bzw. Abreicherung können verschiedene, dem Fachmann bekannte Verfahren, beispielsweise chromatographische Reinigungsverfahren, eingesetzt werden.

Die oben beschriebenen erfindungsgemäßen Verfahren bieten insbesondere den Vorteil, daß eine vollständige oder auch teilweise Abtrennung der im Hopfen vorkommenden Substanzen 6-Prenylnaringenin und 8-Prenylnaringenin möglich wird. Im Falle der beiden genannten, abgetrennten Stoffe handelt es sich um solche, die im Verdacht stehen beziehungsweise von denen nachgewiesen ist, daß sie im Menschen hormonähnliche Wirkungen entfalten. Die Wirkung dieser beiden Substanzen entspricht der Wirkung von weiblichen Sexualhormonen aus der Gruppe der Östrogene. Speziell im Falle von 8-Prenylnaringenin ist nachgewiesen worden, daß dessen Wirkung derjenigen des 17 β-Östradiol sehr ähnlich ist. Um unerwünschte oder gar gesundheitsschädliche Wirkungen auf den Konsumenten mit Sicherheit zu vermeiden, ist es wünschenswert, daß Vorprodukte auf Hopfenbasis breitgestellt werden, aus denen diese unerwünschten Inhaltstoffe ganz oder teilweise entfernt sind. Dieses Erfordernis ist nicht nur für die Herstellung von Bier oder Brauereiprodukten sondern auch für die Erzeugung von hopfenprodukthaltigen Lebensmitteln, Kosmetika und Pharmaka von großer Bedeutung.

Dieses oben beschriebene Ziel wird bei der Anwendung des erfindungsgemäßen Extraktionsverfahrens erreicht, das den vorstehend angegebenen Elutionsschritt 3 umfaßt, wobei bei Anwendung des Elutionsschrittes 3 die Substanzen 6-Prenylnaringenin und 8-Prenylnaringenin abgetrennt werden. Ein Hopfenextrakt, der von 6-Prenylnaringenin und 8-Prenylnaringenin befreit ist, kann bereits dadurch erhalten werden, daß die Extrakte, die bei mindestens einem weiteren (d.h. vorhergehenden und/oder nachfolgenden) Stufenelutionsschritt gewonnen werden, vereinigt werden, wobei in optimaler Weise auch mittels dem Fachmann bekannter, üblicher Verfahren ein von 6-Prenylnaringenin und 8-Prenylnaringenin befreiter Hopfenextrakt der Fraktion 3 zugesetzt werden kann.

Insbesondere zielt die vorliegende Erfindung daher auch auf eine Gewinnung eines phytohormon-freien oder phytohormon-abgereicherten Gemisches von Hopfeninhaltstoffen, vorzugsweise eines Gemisches von Hopfeninhaltstoffen, das frei von 6-Prenylnaringenin und/oder 8-Prenylnaringenin oder bezüglich 6-Prenylnaringenin und/oder 8-Prenylnaringenin abgereichert ist, für die Herstellung von Getränken und Brauereiprodukten, insbesondere von Bier.

Auf diese Weise ermöglicht die vorliegende Erfindung in stets reproduzierbarer Art und Weise die Gewinnung eines Hopfenextraktes, der von 6-Prenylnaringenin und 8-Prenylnaringenin völlig befreit oder in starkem Maße hinsichtlich dieser Substanzen abgereichert ist. In der hier vorliegenden Erfindung bedeutet der Begriff "in starkem Maße hinsichtlich einer Substanz abgereichert", daß die abzureichende Substanz nur noch mit einem Gehalt vorliegt, der weniger als 50 %, vorzugsweise weniger als 10 %, bevorzugterweise weniger als 1 % des ursprünglichen Gehaltes dieser Substanz im Trockengewicht beträgt. Der Begriff "hinsichtlich einer Substanz angereichert" ("an einer Substanz angereichert") bedeutet, daß die Substanz mit einem Gehalt vorliegt, der die Ursprungskonzentration der Substanz im Trockengewicht um mehr als 10 %, vorzugsweise um mehr als 50 %, bevorzugterweise um mehr als 100 % übersteigt.

Weiterhin wird durch die vorliegende Erfindung ermöglicht, daß bei Ausführung nur eines Auswaschungsschrittes ein Extrakt gewonnen wird, der nur die wasserlöslichen Hopfeninhaltstoffe, insbesondere die Gerbstoffe, enthält. Dieses Ziel wird erreicht, wenn in einem ersten Schritt das Extraktionsgut mit Wasser extrahiert und der derart gewonnene Hopfenextrakt aufgefangen wird.

Die vorliegende Erfindung beschreibt im allgemeinen ein Verfahren, das überraschenderweise in sehr einfacher, aber äußerst effektiver Weise eine Abtrennung oder Anreicherung von bestimmten Naturstoffen, insbesondere Flavonoiden, ermöglicht. Darüber hinaus bietet das erfindungsgemäße Verfahren den Vorteil, daß der Lösungsmittelbedarf gering gehalten wird. Ferner eröffnet es große Chancen für eine Automatisierung des Elutionsverfahrens.

Insbesondere ermöglicht die vorliegende Erfindung die Gewinnung eines Substanzgemisches oder einer einzelnen Substanz, die aus der Substanzklasse, bestehend aus Xanthohumol, xanthohumol-abgereichertem Hopfenextrakt, xanthohumol-angereichertem Hopfenextrakt, 6-Prenylnaringenin, 6-prenylnaringenin-abgereichertem Hopfenextrakt, 6-prenylnaringenin-angereichertem Hopfenextrakt, 8-Prenylnaringenin, 8-prenylnaringenin-abgereichertem Hopfen-extrakt, 8-prenylnaringenin-angereichertem Hopfenextrakt, Adlupulon, adlupulonabgerei-chertem Hopfenextrakt, adlupulon-angereichertem Hopfenextrakt, Colupulon, colupulon-abgereichertem Hopfenextrakt, colupulon-angereichertem Hopfenextrakt, Lupulon, lupulon-abgereichertem Hopfenextrakt, lupulon-angereichertem Hopfenextrakt, Humulon, humulon-abgereichertem Hopfenextrakt, humulon-angereichertem Hopfenextrakt, Adhumulon, ad-humulon-abgereichertem Hopfenextrakt, adhumulon-angereichertem Hopfenextrakt, Cohu-mulon, cohumulon-abgereichertem Hopfenextrakt, cohumulonangereichertem Hopfenex-trakt, α-Bittersäure-Gemischen, α-bittersäuren-abgereichertem Hopfenextrakt, α-bittersäuren-angereichertem Hopfenextrakt, β-Bittersäure-Gemischen, β-bittersäuren-abgereichertem Hopfenextrakt, β-bittersäuren-angereichertem Hopfenextrakt und Gemischen hiervon, aus-gewählt wird.

Hierbei können spezifische Hopfeninhaltstoffe in reiner Form oder in einer Form mit hoher Reinheit (beispielsweise mit einer Reinheit von 85 % oder mehr, vorzugsweise von 90 % oder mehr, bevorzugterweise von 97 % oder mehr) gewonnen werden. Außerdem können mit der vorliegenden Erfindung Gemische von α-Bittersäuren in reiner Form oder in einer Form mit hoher Reinheit, ein humulon-angereichertes Gemisch von α-Bittersäuren, ein lupulonange-reichertes Gemisch von β-Bittersäuren oder Humulon oder Lupulon in reiner Form oder in einer Form mit hoher Reinheit gewonnen werden.

Die vorliegende Erfindung umfaßt die vorstehend aufgeführten Substanzen und Substanzgemische. Der Begriff Hopfenextrakt kann in der vorliegender Erfindung sowohl den lösungs-mittelhaltigen Hopfenextrakt als auch das (die) nach Trocknung hieraus erhaltene Substanz-gemisch (Substanz) bezeichnen.

Diese Hopfeninhaltstoffe oder Substanzgemische von Hopfeninhaltsstoffen können für zahlreiche Anwendungen eingesetzt werden. Eine besonders wichtige Anwendung (nicht zur Erfindung gehörend) ist z.B. die Verwendung eines oder mehrerer der vorstehend genannten Hopfeninhaltsstoffe oder eines der nachfolgend genannten an- oder abgereicherten Substanzgemische der Hopfeninhaltsstoffe zur Realisierung einer schlaffördernden und/oder beruhigenden Wirkung. Vorzugsweise kommen hierfür α-Bittersäure-Gemische, α-bittersäuren-abgereicherte Hopfenextrakte, α-bittersäuren-angereicherte Hopfenextrakte, β-Bittersäure-Gemische, β-bittersäuren-abgereicherte Hopfenextrakte, β-bittersäuren-angereicherte Hopfenextrakte und Gemische hiervon zum Einsatz. Ein derartiger Extrakt kann beispielsweise das Einschlafen erleichtern, aber auch die Schlafphasen einer Person verlängern. Insbesondere ist er zur Anwendung beim Menschen und Säugetier gedacht. Die erforderliche Menge eines oder mehrerer Hopfeninhaltstoffe oder eines oder mehrerer an- oder abgereicherter Hopfeninhaltsstoffe in Substanzgemischen kann von einem Fachmann auf der Grundlage seines allgemeinen Wissens ausgewählt werden. Vorzugsweise kann ein derartiger Extrakt völlig frei von 6-Prenylnaringenin und 8-Prenylnaringenin sein oder aber er enthält weniger als 0,005 bis 5 Gew.-%, vorzugsweise weniger als 0,003 bis 3 Gew.-% des 6-Prenylnaringenin und/oder 8-Prenylnaringenin bezogen auf das Gesamttrockengewicht der Hopfeninhaltstoffe.

Vorzugsweise ist ein derartiger Extrakt zur oralen Einnahme (nicht zur Erfindung gehörend) geeignet. Für spezifische Anwendungen kann die Formulierung des Gemisches beispielweise in Form von Tabletten, Kapseln oder in einer flüssigen Verabreichungsform erfolgen. Die Verabreichung kann beispielweise auch oral, mittels i.v.- bzw. i.m.-Injektion oder nasal mittels Spray, durch Inhalation, vaginal, anal, topisch oder in einer anderen dem Fachmann bekannten Verabreichungsweise erfolgen. Beispielweise kann eine derartige Formulierung eine Dosis eines bestimmten Hopfeninhaltsstoffes oder eines Gemisches von Hopfeninhaltsstoffen im Bereich von 0,001 g bis 10 g enthalten, wobei eine derartige Formulierung einmal oder mehrmals täglich eingenommen werden kann. Optional kann das Gemisch weitere dem Fachmann bekannte Wirkstoffe und geeignete Formulierungszusatzstoffe enthalten.

Weiterhin kann einer der vorstehend genannten Hopfeninhaltsstoffe oder eines der vorstehend genannten Substanzgemische aus Hopfeninhaltsstoffen zur Herstellung eines Arzneimittels (nicht zur Erfindung gehörend) für die Behandlung und/oder zur Vorbeugung von Erkrankungen, die durch eine bakterielle Infektion, z.B. mit Staphylokokken, insbesondere mit Staphylokokken, die multiple Antibiotikaresistenzen aufweisen, eingesetzt werden. Denkbar wäre auch die Behandlung und/oder die Vorbeugung von Erkrankungen, die durch eine Infektion, z.B. mit Helicobacter pylori, verursacht werden. Vorzugsweise werden hierfür α-Bittersäure-Gemische, α-bitter-säuren-abgereicherte Hopfenextrakte, α-bittersäuren-angereicherte Hopfenextrakte, β-Bitter-säure-Gemische, β-bittersäuren-abgereicherte Hopfenextrakte, β-Bittersäuren-angerei-cherter Hopfenextrakte und Gemische hiervon verwendet. Vorzugsweise ist ein derartiges Medi-kament frei von 6-Prenylnaringenin und 8-Prenylnaringenin oder es enthält weniger als 5 Gew.-% des 6-Prenylnaringenin und/oder des 8-Prenylnaringenin, vorzugsweise weniger als 3 Gew.-% des 6-Prenylnaringenin und/oder des 8-Prenylnaringenin, jeweils bezogen auf das Trockengewicht der Hopfeninhaltsstoffe.

Darüber hinaus können die genannten Hopfeninhaltsstoffe oder eines der vorstehend genannten Substanzgemische der Hopfeninhaltsstoffe als Konservierungsmittel (nicht zur Erfindung gehörend) eingesetzt werden. Besonderes geeignet ist eine Anwendung als Konservierungsmittel für Lebensmittel und kosmetische Produkte, die von den Verbrauchern in hohem Maße akzeptiert wird. Vorzugsweise werden hierfür α-Bittersäure-Gemische, α-bittersäuren-abgereicherte Hopfenextrakte, α-bittersäuren-angereicherte Hopfenextrakte, β-Bittersäure-Gemische, β-bittersäuren-abgereicherte Hopfenextrakte, β-bittersäuren-angereicherte Hopfenextrakte und Gemische hiervon verwendet. Den zu konservierenden Produkten können dabei beispielweise zwischen 0,001 und 40 Gew.-%, vorzugsweise zwischen 0,005 und 2 Gew.-%, der Hopfeninhaltsstoffe oder ihrer Gemische zugesetzt werden, bezogen auf das Gewicht des zu konservierenden Produktes. Darüber hinaus können die Hopfeninhaltsstoffe oder Gemische der Hopfeninhalts-stoffe auch als ein Gemisch mit einem oder mehreren weiteren anorganischen und/oder organischen Konservierungsmitteln eingesetzt werden.

Weiterhin können die genannten Hopfeninhaltsstoffe oder eines der vorstehend genannten Substanzgemische der Hopfeninhaltstoffe als Pestizid, (nicht zur Erfindung gehörend) insbesondere als Insektizid, verwendet werden. Vorzugsweise werden hierfür α-Bittersäure-Gemische, α-bittersäuren-abgereicherte Hopfenextrakte, α-bittersäuren-angereicherte Hopfenextrakte, β-Bittersäure-Gemische, β-bittersäuren-abgereicherter Hopfenextrakte, β-bittersäuren-angereicherte Hopfenextrakte und Gemische hiervon verwendet. Die spezifische Formulierung des Pestizids erfolgt dabei gemäß dem Wissen des Fachmannes. Insbesondere kann die Pestizid- oder Insektizidformulierung weitere Substanzen oder Substanzgemische umfassen, die eine pestizide oder insektizide Wirkung aufweisen.

Die hier beschriebene Erfindung (Stufenelution als Vorwärts- und als Rückwärtselution) unterscheidet sich von breits bekannten chromatographischen Verfahren dadurch, daß das zu patentierende Verfahren vorzugsweise dann eingesetzt wird, wenn sehr große Mengen von Vorprodukten aus Pflanzen oder pflanzlichen Ausgangsstoffen auf möglichst natürliche Weise (Extraktion mit Hilfe von Ethanol-/Wasser-Gemischen) bei Raumtemperatur gewonnen werden sollen und die Anwendung bisher bekannter chromatographischer Verfahren bzw. chromatographischer Gradientenverfahren aus Kostengründen, Kapazitätsgründen oder Gründen der Prozeßdauer nicht oder nicht wirtschaftlich möglich sind.

Darüber hinaus werden durch die Nutzung der natürlich auffallenden pflanzlichen Eigencellulose erhebliche Kostenvorteile gegenüber kommerziell erhältlichen Zellstoffmaterialien (als stationäre Phase) erzielt. Insbesondere besteht die Möglichkeit, daß aus einer oder mehreren Fraktionen eine oder mehrere unerwünschte Substanzen mit Hilfe des beschriebenen Verfahrens abgetrennt und die derart aufbereiteten oder gereinigten Fraktionen mit anderen Extraktionsfraktionen wieder vereinigt werden, um einen Extrakt zu gewinnen, der von bestimmten unerwünschten, beispielweise gesundheitsschädlichen, Substanzen befreit ist.

Von hohem Interesse ist es hierbei, daß sich die aus ausgewaschenen Fraktionen oder Kombinationen derselben erhaltenen Substanzen oder Substanzgemische durch positive Wechselwirkungen auszeichnen können. Andererseits ist es möglich Substanzen mit negativen Wirkeigenschaften verstärktem Maße abzureichen oder abzutrennen.

Das vorliegende Verfahren ist wegen der Preisgünstigkeit, Einfachheit der Durchführung, Gewinnung großer Mengen und selektiver Gewinnung von Stoffen oder Stoffgemischen sehr vorteilhaft. Die Vorteile des hier beschriebenen Verfahrens bestehen darin, daß nicht nur eine chromatographische Trennung und Gewinnung von Stoffklassen oder Stoffgruppen in großen Mengen (im technischem Maßstab) an der Eigencellulose möglich ist, sondern daß durch das Hindurchleiten der mobilen Phase an der stationären Phase immer frisches, d.h. weitgehend "unbeladenes" Lösungsmittel, bereitsteht, das erst beim Passieren der stationären Phase nach und nach "beladen" wird, so daß eine erschöpfende Extraktion des Extraktionsgutes weitgehend gewährleistet ist.

Ein derartiges Verfahren sollte eine vergleichsweise geringe Menge der organischen Lösungsmittel (Alkohol) benötigen und deshalb den Vorteil aufweisen, daß es zumindest in Trennungsverfahren, wie es von der vorliegenden Erfindung angestrebt wird, eine gezielte Anreicherung oder Isolierung von bestimmten pflanzlichen Inhaltstoffen aus einem Naturstoffgemisch in einfacher und wirtschaftlicher Weise ermöglicht.

Die Erfindung betrifft im übrigen ein neues Verfahren zum Isolieren von beispielsweise ernährungsphysiologisch, kosmetisch, pharmazeutisch oder anderweitig bedeutsamen Substanzen in Form von Stoffgruppen oder in reiner Form durch Elution aus Pflanzen (Hopfen) oder Pflanzenprodukten (Hopfenprodukten) mit wäßrigen/alkoholischen Lösungsmitteln an der pflanzlichen Eigencellulose als stationäre Phase.

Besondere Vorteile der hier beschriebenen Erfindung sind die überragende prozeßtechnische Einfachheit, so daß die zur Umsetzung des Verfahrens erforderlichen Investitionen relativ gering sind. Ferner erfordert das Verfahren einen relativ geringen Energieverbrauch, da jede Art von Erwärmung während der Extraktion entfällt. Das Verfahren eignet sich zur selektiven Gewinnung von pflanzlichen Inhaltsstoffen nach Stoffklassen unter Normalbedingungen, was z.B. beim Hopfen die selektive Gewinnung von Alphasäuren, Betasäuren, Gerbstoffen, Flavonoiden und anderen Polyphenolen ermöglicht.

Die Vorteile des hier beschriebenen Verfahrens bestehen darin, daß nicht nur eine chromatographie-ähnliche Trennung und Gewinnung von Stoffklassen oder Stoffgruppen in großen Mengen (technischer Maßstab) durch der Bindung an die Eigenzellulose möglich ist, sondern daß beim Hindurchleiten der mobilen Phase immer frisches, weitgehend "unbeladenes" Lösungsmittel an der stationären Phase bereitsteht. Erst beim Passieren der stationären Phase wird die mobile Phase nach und nach "beladen", so daß eine erschöpfende Extraktion des Extraktionsgute s weitgehend gewährleistet ist. Bei Anwendung der üblichen Extraktionsverfahren wird das pflanzliche Extraktionsgut diskontinuierlich oder kontinuierlich mit Lösungsmittel versetzt und das ausgelaugte Extraktionsgut von dem die pflanzlichen Inhaltsstoffe enthaltenden Lösungsmittel getrennt. Dieser Vorgang liefert mehr oder weniger ausgeprägt die Gesamtheit der pflanzlichen Inhaltsstoffe. Im Gegensatz zu gängigen Extraktionsverfahren werden beim hier beschriebenen Verfahren, d.h. einem permanenten Auswaschungsprozeß mit wechselnden Lösungsmitteln und/oder Lösungsmittelmischungen verschiedener Zusammensetzung, die Inhaltskomponenten aus dem Extraktionsgut nacheinander getrennt herausgelöst. Dabei kann das Verfahren in Abhängigkeit von einerseits gewünschten und andererseits möglicherweise störenden Inhaltsstoffen so gesteuert werden, daß beispielsweise die störenden Inhaltsstoffe mit einem ersten Lösungsmittel oder Lösungsmittelgemisch herausgelöst werden, bevor die Extraktion der eigentlich interessierenden Inhaltsstoffe beginnt - bzw. umgekehrt.

Das Verfahren ermöglicht insbesondere die Gewinnung eines phytohormon-freien Hopfenextraktes, der im Übrigen alle restlichen Hopfeninhaltstoffe in natürlichen Anteilen enthält, so daß dieser beispielsweise in der Brauindustrie gemäß dem deutschen Reinheitsgebot verwendet werden kann. Die Substanzen mit phytohormonaler beziehungsweise österogen-artiger Wirkung (6- und 8-Prenylnaringenin) werden z.B. in der Vorwärtselution mit Hilfe von LM3 in der Fraktion 3 eluiert. Aus der Fraktion 3 können die phytohormonalen Substanzen mit laborüblichen Methoden, beispielweise VLC (Vaccum Liquid Chromatography), vollständig entfernt werden. Teilweise oder vollständig abgereicherte Flavonoidextrakte und die übrigen mit dem Verfahren gewonnenen Hopfenextrakte können entsprechend den Anforderungen der deutschen Brauer (Reinheitsgebot) vereinigt werden.

Märkte und Potential der durch die erfindungsgemäße Methode gewonnenen Hopfenprodukte:
Falls es erwünscht ist, können die gewonnenen Fraktionen der vorstehend aufgeführten Schritte hinsichtlich bestimmter Inhaltstoffe weiter angereichert oder abgereichert werden. Die Inhaltstoffe können aber auch hieraus in einer Reinheit gewonnen werden, die für die jeweils gewünschten Anwendungen geeignet sind. Hierfür können verschiedene bekannte Verfahren, wie chromatographische Reinigungsverfahren, verwendet werden.

Im Falle der Fraktion 1 erfolgt eine Gewinnung von Gerbstoffen, zuckerhaltigen Verbindungen, Hopfenbittersäuren und anderen in Wasser löslichen Hopfeninhaltstoffen. Diese Fraktion besitzt einen fruchtigen Geruch. Sie hat z.B. eine sedative Wirkung.

Im Falle der Fraktion 2 werden α-Bttersäuren aus Hopfen gewonnen, die beispielsweise als Schäumungs- und als Bitterungsmittel in der Bier-Branche Verwendung finden. Fraktion 2 enthält Komponenten, denen eine Wirkung gegen Staphylokokken und gegen multiple Antibiotikaresistenzen (MRS) nachgesagt wird. Diese Fraktion kann z.B. auch gegen Helicobacter pylori eingesetzt werden. Die Verwendung als Antibiotikum bzw. antibiotische Komponente in einer pharmazeutischen Formulierung ist daher grundsätzlich möglich. Diese Fraktion 2 läßt sich als Pestizid oder als antioxidatives, konservierendes oder als antibakterielles Produkt in den Branchen Nahrungsmittel, Kosmetik und Pharma einsetzen.

Mit der Fraktion 3 werden Flavonoide, beispielweise das Xanthohumol, gewonnen. Gegebenenfalls können mit Hilfe von nachfolgend verwendeten laborüblichen Verfahren, wie beispielweise VLC (Vacuum Liquid Chromatography), Flavonoide in angereicherter oder Flavonoide in reiner Form oder in einer Form mit hoher Reinheit erhalten werden. In in vitro Versuchen soll Xanthohumol eine vorbeugende Wirkung, z.B. gegen Osteoporose, Arteriosklerose, Erkrankungen der Herzkranzgefäße, Herzinfarkt und Schlaganfall, gezeigt haben. Zugleich wurde ein hohes antikanzerogenes Potential nachgewiesen. So zeigt Xanthohumol wachstumshemmende und zytotoxische Effekte gegenüber 60 verschiedenen menschlichen Krebszell-Linien, wie z.B. im Falle von Leukämie, Lungenkrebs, Dickdarmkrebs, Melanom, Brustkrebs usw.. In Tests mit isolierten Maus-Brustdrüsenzellen (MMOC-Test, Mouse Mammary Gland Organ Culture) zeigte sich Xanthohumol als 200-fach wirksamer als das aus dem Rotwein isolierte Resveratrol. In allen bisher beschriebenen in vitro Versuchen war keine zytotoxische Wirkung gegenüber gesunden Zellen nachweisbar [Lit.: Tabata et al. (1997), Tobe et al. (1997), Miranda et al. (1999, 2000), Stevens et al. (1998), Henderson et al. (2000), Gerhäuser et al. (2002)]. Mit Hilfe von in vivo Versuchen an Ratten konnten Metabolismus, Bioverfügbarkeit und Pharmakokinetik der Substanz bereits aufgeklärt werden. In diesen Versuchen zeigte Xanthohumol keinerlei toxische Wirkung (Dosierung: 1.000 mg Xanthohumol pro kg Tierkörpergewicht). Aus diesem Grunde könnte Xanthohumol, xanthohumol-abgereicherter Hopfenextrakt oder xanthohumol-angereicherter Hopfenextrakt wegen seiner nahrungsergänzenden Eigenschaften und darüber hinaus wegen seines beträchtlichen pharmakologischen Potentials erfolgreich in gesundheitsfördernden und das Altern hemmenden Produkten in den Branchen Nahrungsergänzungsmittel/Gesundheitsnahrung, Kosmetik, Pharmaka zur Anwendung kommen. Die erfindungsgemäßen Verfahren ermöglichen zugleich eine vollständige oder eine teilweise Abtrennung der im Hopfen vorkommenden Substanzen 6-Prenylnaringenin und 8-Prenylnaringenin (Hopfen-Phytohormone), bei denen es sich um Stoffe handelt, die im Verdacht stehen, beziehungsweise von denen nachgewiesen ist, daß sie bei Menschen hormonähnlich wirken. Die Wirkung ähnelt derjenigen von weiblichen Sexualhormonen aus der Gruppe der Österogene. Speziell im Falle von 8-Prenylnaringenin wurde nachgewiesen, daß es eine ähnliche Wirkung wie des 17β-Östradiol besitzt. Um unerwünschte oder gesundheitsschädliche Wirkungen im Konsumenten sicher zu vermeiden, besteht der Wunsch nach Produkten auf Hopfenbasis, aus denen diese Inhaltstoffe ganz oder teilweise entfernt wurden. Diese Anforderung ist nicht nur für Biere oder Brauereiprodukte sondern auch für hopfenprodukthaltige Kosmetika und Pharmaka von Bedeutung. Das Hopfen-Phytohormon 8-Prenylnaringenin stellt das bisher aktivste Phytohormon mit östrogener Wirkung dar.

Im Falle der Fraktion 4 werden reine Bittersäuren ( Gemisch aus α- und β-Bittersäuren) gewonnen. Die Fraktion 4 kann mittels bekannter Verfahren weiter aufgetrennt werden, wobei reine Bittersäuren beziehungsweise angereicherte Lupulon-Extrakte und/oder angereicherte Humulon-Extrakte gewonnen werden. Insbesondere kann diese Reinigung beispielweise durch eine Extraktion aus dem Gemisch einer wäßerigen Phase heraus bei saurem, neutralem oder basischem pH-Wert, beispielweise durch einen Säure-Base-Trennungsgang, in eine organische Phase hinein erfolgen. Der aus der Fraktion 4 gewonnene Hopfenextrakt und/oder die darin enthaltenen Substanzen, insbesondere die Bittersäuren, sind durch beruhigende und schlaffördernde Eigenschaften charakterisiert und können zur Herstellung beruhigender oder schlaffördernder Produkte bzw. Medikamente eingesetzt werden. Weiterhin wirken die Komponenten der Fraktion 4, z.B. die Hopfenbittersäuen Humulon, Adhumulon, Cohumulon, Prehumulon und Posthumulon, die entsprechenden bittersäure-abgereicherten Hopfenextrakte, die entsprechenden bittersäure-angereicherten Hopfenextrakte sowie die Bittersäuren Lupulon, Adlupulon, Colupulon, Prelupulon und Postlupulon, die entsprechenden bittersäure-abgereicherten Hopfenextrakte und die entsprechenden bittersäure-angereicherten Hopfenextrakte als Konservierungsmittel für die Verwendung in den Branchen Lebensmittel, Kosmetik und Pharma. Die β-Bittersäuren zeigen z.B. eine Aktivität gegen Staphylococcus aureus, Listeria monocytogenes, Lactobacillus helveticus, multiresistente Staphylococcen Stämme (MRS), Akne verursachende Mikroben sowie gegen Helicobacter pylori und können als Pestizide eingesetzt werden.

In Fraktion 5 werden Chlorophylle und weitere, nicht wasserlösliche oder in einem Gemisch aus Wasser und Ethanol mit einem Ethanolgehalt von weniger als 90 % nicht lösliche Hopfeninhaltstoffe gesammelt. Diese Fraktion kann als natürlicher grüner Farbstoff in den Branchen Lebensmittel, Kosmetik und Pharma eingesetzt werden.

### Stufen-Extraktion

Das Ausgangsmaterial (5 kg Hopfendolden) wird mit einer Alpin-Rotoplex-Schneidmühle vom Typ RO 20/10 B fein zermahlen, wobei die Stücke maximal eine Größe von 1 mm³ erhalten. Anschließend wird das zermahlene Ausgangsmaterial in ein Extraktionsfass eingefüllt (Firma Ecker Hydro-Anlagen GmbH), und wie folgt in vier Stufen bei Raumtemperatur (20 °C) extrahiert:
A. Extraktion mit 25 Liter Wasser
B. Extraktion mit 25 Liter einer Ethanol/Wasser-Mischung mit einem Ethanolgehalt von 30 Vol.-% an Ethanol
C. Extraktion mit 20 Liter einer Ethanol/Wasser-Mischung mit einem Ethanolgehalt von 50 % an Ethanol,
D. Extraktion mit 30 Liter einer Ethanol/Wasser-Mischung mit einem Ethanolgehalt von 80 Vol.-% an Ethanol,
E. Extraktion mit 15 Liter einer Ethanol/Wasser-Mischung mit einem Ethanolgehalt von 96 Vol.-% an Ethanol.

Hierbei werden durch eine Extraktion mit Wasser Gerb- und Zuckerstoffe, sowie ein Teil der α-Bittersäuren, insbesondere Pre- und Posthumulon, entfernt. Bei einer Extraktion mit einem Lösungsmittelgemisch gemäß Stufe B werden die α-Bittersäuren Humulon, Cohumulon und Adhumulon im Extrakt gewonnen, während bei der Extraktion mit einem Lösungsmittelgemisch gemäß Stufe C alle oder im Wesentlichen alle Flavonoide im Extrakt gewonnen werden, insbesondere Xanthohumol. Hierbei ist anzumerken, dass das beim Filtrationsprozess eingesetzte Polyvinylpolypyrrolidon (PVPP-) Gel Flavonoide und insbesondere Xanthohumol nahezu quantitativ zurückhält. Daher kann bei herkömmlichen Filtrationsprozessen eine Xanthohumolgabe erst nach der Filtration erfolgen. Bei einer Extraktion mit einem Lösungsmittelgemisch gemäß Stufe D wird ein kleiner Teil α-Bittersäuren und alle oder im Wesentlichen alle β-Bittersäuren, so auch insbesondere Lupulon, Adlupulon und Colupulon, im Extrakt gewonnen. Bei einer Extraktion mit einem Lösungsmittelgemisch gemäß Stufe E werden Chlorophylle und Fette, sowie andere in den zuvor eingesetzten Lösungsmittelgemischen nicht lösliche Substanzen gewonnen.

Falls gewünscht können die vorstehend beschriebenen Extrakt-Fraktionen (A bis E) der Stufenextraktion weiteren Bearbeitungsstufen gemäß dem allgemeinen Fachwissen unterworfen werden beispielsweise einer VLC (vacuum liquid chromatography) und es können gezielt Produkte in angereicherter oder Extraktform gewonnen werden. Alternativ ermöglicht die Stufenextraktion die Fraktionierung der Hopfeninhaltsstoffe in Klassen beziehungsweise Gruppen mit einheitlichem Lösungsverhalten.

Analog kann zu der vorstehend beschriebenen Stufenextraktion eine Stufenextraktion ausgehend von handelsüblichen Hopfenpellets (beispielsweise Hopfenpellets der Firma HHV - Hallertauer Hopfenveredelungsgesellschaft, Mainburg oder Hopfenpellets der Firma NATECO₂, Wolnzach) oder alkoholischen oder Kohlendioxid-Extrakten des Hopfens oder den Rückständen der alkoholischen oder Kohlendioxid-Extraktion des Hopfens durchgeführt werden.

### Gewinnung eines östrogenfreien Hopfenextrakts

Dieses Beispiel beschreibt die Gewinnung eines östrogenfreien Hopfenextrakts, der jedoch alle restlichen Hopfeninhaltsstoffe umfasst, welche für die jeweilige Verwendung, beispielsweise in der Brauindustrie, erwünscht sind.

Die Substanzen mit phytohormonaler Wirkung beziehungsweise die östrogen-artig wirkenden Substanzen (6- und 8-Prenylnaringenin) werden bei einer Extraktion mit einem Lösungsmittelgemisch gemäß Stufe C gewonnen. Aus dem derart erhaltenen Flavonoidextrakt werden phytohormonale Substanzen (6- und 8-Prenylnaringenin) mit laborüblichen Methoden, beispielsweise einer VLC (vacuum liquid chromatography) entfernt und der teilweise oder vollständig abgereicherte Flavonoidextrakt und die anderen gewonnenen Hopfen-Extrakte (Stufe A, B, D, E) werden vereinigt.

### Gewinnung von angereicherten Hopfen-Flavonoiden-Extrakten

Bei einer Extraktion mit einem Lösungsmittelgemisch gemäß Stufe B, wie in Beispiel 1 beschrieben, können bestimmte Flavonoide angereichert werden. Insbesondere kann ein Xanthohumol / xanthohumolreicher Hopfenextrakt, ein 6- und/oder 8- Prenylnaringenin angereicherter Hopfenextrakt, Lupulon- und Humulon-angereicherter Hopfenbitterstoffextrakt, hergestellt werden.

### Gewinnung von reinen Hopfeninhaltsstoffen

Aus den Extrakten gemäß Stufe A bis E des vorstehenden Beispiels 1 können reine Hopfeninhaltsstoffe gewonnen werden. Insbesondere kann Xanthohumol je nach Anwendungszweck in reiner Form oder in einer Form mit hoher Reinheit wie in Beispiel 3 beschrieben gewonnen werden. Weiterhin kann 6- und 8-Prenylnaringenin je nach Anwendungszweck in reiner Form oder in einer Form mit hoher Reinheit wie in Beispiel 2 beschrieben gewonnen werden. Darüber können in analoger Weise gemäß dem Wissen des Fachmanns α-Bittersäuren in reiner Form oder in einer Form mit hoher Reinheit, ein Humulon-angereichertes Gemisch von α-Bittersäuren, ein Lupulon-angereichertes Gemisch von β-Bittersäuren oder Humulon in reiner Form oder in einer Form mit hoher Reinheit gewonnen werden. Insbesondere bietet die vorliegende Erfindung die Möglichkeit den Humulongehalt in einem Gemisch von α-Bittersäuren oder den Lupulongehalt in einem Gemisch von β-Bittersäuren reproduzierbar und zuverlässig einzustellen.

### Effekt von PVPP-Gel auf Xanthohumol-Gehalt

Eine ethanolische Stammlösung von Xanthohumol wurde angesetzt wobei 1 g der Stammlosung 19,13 mg Xanthohumol enthielten.

100 ml Bier (Biersorte: Bière Blonde de luxe, Brasserie de Saverne/Frankreich, Alkoholgehalt 4,5%.) wurden mit 32,8 mg Stammlösung versetzt, entsprechend einer Xanthohumolkonzentration von 6,3 mg pro Liter Bier.

Die Bierprobe wurde über eine mit PVPP-Gel befüllte Saule abgesaugt Die das Gel passierte Lösung wurde anschließend am Rotationsverdampfer bis zur Trockene eingeengt und der Rückstand einer HPLC unterzogen. Der Vergleich des Chromatogramms mit dem Referenzchromatogramm fur reines Xanthohumol zeigt, dass allenfalls Spuren von Xanthohumol die PVPP-Saule passiert haben (Ergebnisse nicht gezeigt).

Das PVPP-Gel wurde mit einem Eluierungsmittel eluiert und anschließend das Eluens entfernt. Das HPLC-Chromatogramm des Rückstands zeigt ein ausgeprägtes Xanthohumolsignal bei einer Retentionszeit von 3,8 Minuten.

### Sudversuche mit verschiedenen Hopfenextrakten und Zusatz von Xanthohumol bzw. 8-Prenylnaringenin zu Bier

Die Extrakte Hopsteiner Ethanol- bzw. CO₂-Extrakt (herkömmliche Extrakte) und HOP RE (erfindungsgemäßer Extrakt mit allen natürlichen Inhaltsstoffen) und HOP R5R (erfindungsgemäßer Extrakt an 8-Prenylnaringenin abgereichert) wurden hinsichtlich ihrer Zusammensetzung untersucht.

Zunächst wurden die verschiedenen Extrakte mittels HPLC auf ihre Gehalte an α- und β-Säuren analysiert. Die Ergebnisse sind in Tabelle 19 dargestellt.

**Tabelle 19: Hopfenextrakt-Analyse**

| Parameter | Hopst EtOH | Hopst. CO2 | HOP RE | HOP R5R |
|---|---|---|---|---|
| co-Humulon [ppm] | 9,23 | 12,02 | 8,61 | 5,19 |
| n-/ad-Humulon [ppm] | 28,95 | 38,40 | 26,24 | 15,85 |
| Σ α-Säuren [ppm] | 38,18 | 50,42 | 34,85 | 21,04 |
| co-Lupulon [ppm] | 7,43 | 9,11 | 6,61 | 8,00 |
| n-/ad-Lupulon [ppm] | 8,24 | 9,88 | 7,09 | 8,75 |
| Σ β-Säuren [ppm] | 15,67 | 18,99 | 13,70 | 16,75 |

Auf der Basis der festgestellten Gehalte an α -Säuren erfolgte später die Dosage bei den veschiedenen Suden mit einem Zielwert von 35 Bittereinheiten in den einzelnen Bieren.

Im Folgenden wurden vier 150 L-Sude unter Verwendung der unterschiedlichen Hopfenextrakte mit einem für Vollbiere üblichen Stammwürze-Gehalt zwischen 11 und 11.5 % eingebraut. Zur Beurteilung der Würzequalität und der Bitterstoffausnutzung wurden die wichtigsten Parameter analytisch bestimmt. In Tabelle 20 sind die Ergebnisse der Würzeanalysen zusammengefasst.

**Tabelle 20: Würzeanalyse**

| Parameter | Hopst EtOH | Hopst. CO₂ | HOP RE | HOP R5R |
|---|---|---|---|---|
| Stammwürze [%] | 11,34 | 11,31 | 11,28 | 11,27 |
| Bittereinheiten [BE] | 56 | 63 | 54 | 76 |
| co-Humulon [ppm] | 1,08 | 1,36 | 1,25 | 1,62 |
| n-/ad-Humulon [ppm] | 2,28 | 2,93 | 2,48 | 2,15 |
| Σ α-Säuren [ppm] | 3,36 | 4,29 | 3,73 | 3,77 |
| co-Isohumulon [ppm] | 1,97 | 2,48 | 2,05 | 2,37 |
| n-Isohumulon [ppm] | 4,65 | 5,13 | 4,12 | 4,65 |
| ad-Isohumulon [ppm] | 11,33 | 13,03 | 11,95 | 15,30 |
| Σ iso- α-Sauren [ppm] | 17,95 | 20,64 | 18,13 | 22,32 |
| Xanthohumol [ppm] | 0,7 | 0,6 | 0,9 | 1,6 |
| 8-Prenylnaringenin [ppm] | n.n. | n.n. | n.n. | n.n. |
| pH | 5,62 | 5,74 | 5,72 | 5,63 |
| Farbe [EBC] | 8,8 | 7,9 | 7,5 | 8,2 |
| Gesamtstickstoff [mg/L] | 866 | 878 | 891 | 864 |
| Koagul. Stickstoff [mg/L] | 20 | 17 | 19 | 17 |
| MgSO₄-Stickstoff [mg/L] | 197 | 192 | 204 | 191 |
| Polyphenole [mg/L] | 152 | 136 | 115 | 132 |
| Anthocyanogene [mg/L] | 36 | 34 | 34 | 34 |
| Trübung 20° | 2,5 | 1,7 | 1,7 | 1,8 |

Beim Blick auf die Bittereinheiten zeigt sich bei den Würzen eine gute Obereinstimmung zwischen Hopsteiner Ehanol und HOP RE, was mit den vergleichbaren Werten für die iso- α-Sauren korreliert, die den Hauptbeitrag zur Bittere liefern. Entsprechend ist der Bitterwert bei Hopsteiner CO₂ noch höher. Bei HOP R5R wurde ein sehr hoher Bitterwert gemessen, auch die Gehalte an den einzelnen Bitterkomponenten sind deutlich höher. Wegen einer gleichen Vorgehensweise bei der Herstellung der Sude kann nicht von einer unterschiedlichen Bitterstoffausnutzung ausgegangen werden. Üblicherweise machen die α-Gehalte 1/3 der durch den Bitterwert ausgedruckten Bitterstoffe aus. Dieses Verhältnis ist bei allen Suden annähernd gegeben. Das 8-Prenylnaringenin ist erwartungsgemäß nur in der Würze nachweisbar, bei welcher die Substanz als Reinstoff zugegeben wurde. Der pH liegt bei Hopsteiner CO₂ und HOP RE leicht oberhalb des üblichen Bereichs. Die Werte der Farbe sind typisch für die Würze eines hellen Vollbieres. Von Bedeutung sind weiterhin die Stickstoffverbindungen. Wahrend die Aminosäuren die wichtigsten N-Quellen für den Hefestoffwechsel darstellen und über diesen maßgeblichen Einfluss auf die Bildung von Gärungsnebenprodukten haben, sind die mittel- und hochmolekularen N-Verbindungen vor allem für die kolloidale Stabilität, Vollmundigkeit und Schaumhaltbarkeit von Bedeutung. Deshalb wurden neben der Ermittlung des Gesamtstickstoffs, der für alle Würzen im üblichen Bereich liegt, der koagulierbare Anteil (Vollmundigkeit, Schaum und Eiweißstabilität) und der MgSO₄-fällbar Anteil (Schaumhaltbarkeit) bestimmt. Beide Stickstoff-Anteile liegen im für helle Vollbierwürzen üblichen Bereich und variieren entsprechend den vergleichbaren Extraktgehalten (Stammwürze) nur wenig. Die phenolischen Verbindungen nehmen je nach Struktur und Molekülgröße starken Einfluss auf verschiedene Biereigenschaften wie Farbe, Geschmack, Schaum sowie die chemischphysikalische Stabilität (Trübungsbildung). Der Gang der Polyphenole beeinflusst entsprechend den gemessenen Wert die Farbe. Die Anthocyanogene nehmen Einfluss auf die Ausbildung kolloidaler Trübungen im Bier und sind deshalb von besonderem Interesse. Entsprechend dem leicht erhöhten Polyphenolgehalt bei Hopsteiner EtOH ist auch dessen Anthocyanogengehalt etwas höher. Sowohl der Gesamtpolyphenolgehalt als auch der Gehalt an Anthocyanogenen liegt im unteren Bereich der für Vollbierwürzen üblichen Gehalte. Die Würzen sind bei Trübungswerten um 2 als sehr klar einzustufen. Die Würzen wurden unter gleichen Bedingungen fermentiert und nach dem Abschlauchen gelagert und filtriert. Nach der Carbonisierung erfolgte die teilweise Abfüllung in Flaschen. Von dem Sud HOP R5R wurde vor der Carbonisierung ein Teil mit Xanthohumol (Zielkonzentration: 5,6 mg/L; R5R + Xn), ein anderer Teil mit 8-Prenylnaringenin (Zielkonzentration: 2,5 mg/L; R5R + 8PN) dotiert. Die Ergebnisse der Analysen der abgefüllten Biere sind in Tabelle 21 zusammengefasst.

**Tabelle 21: Bieranalyse**

| Parameter | Hopst, EtOH | Hopst. CO₂ | HOP RE | HOP R5R | R5R + Xn | R5R + 8PN |
|---|---|---|---|---|---|---|
| Stammwürze [%] | 11,31 | 11,33 | 11,39 | 11,35 | 11,34 | 11,31 |
| Alkohol [Vol.-%] | 4,36 | 4,34 | 4,32 | 4,42 | 4,42 | 4,42 |
| Vergärungsgrad [%] | 72,9 | 71,9 | 71,1 | 73,1 | 73,1 | 73,4 |
| Bittereinheiten [BE] | 34 | 35 | 36 | 44 | 42 | 42 |
| co-Humulon [ppm] | 0,58 | 0,63 | n.n. | 0,54 | 0,42 | 0,46 |
| n-/ad-Humulon [ppm] | 0,79 | 0,61 | 0,17 | 0,34 | 0,28 | 0,30 |
| Σ α-Säuren [ppm] | 1,37 | 1,24 | 0,17 | 0,88 | 0,70 | 0,76 |
| co-Isohumulon [ppm] | 2,93 | 2,89 | 2,72 | 3,37 | 3,03 | 3,13 |
| n-Isohumulon [ppm] | 4,94 | 4,82 | 3,90 | 4,90 | 3,93 | 4,49 |
| ad-Isohumulon [ppm] | 11,79 | 11,26 | 11,02 | 12,99 | 10,79 | 12,19 |
| Σ iso-α-Säuren [ppm] | 19,66 | 18,97 | 17,64 | 21,26 | 17,75 | 19,81 |
| Xanthohumol [ppm] | 0,3 | 0,5 | 0,5 | 0,3 | 5,7 | 0,3 |
| 8-Prenylnaringenin [ppm] | n.n. | n.n. | n.n. | n.n. | n.n. | 8,0 |
| pH | 4,47 | 4,49 | 4,52 | 4,51 | 4,49 | 4,49 |
| Farbe [EBC] | 5,5 | 5,4 | 4,9 | 5,5 | 6,2 | 5,0 |
| Gesamtstickstoff [mg/L] | 663 | 668 | 676 | 664 | 679 | 660 |
| Koagul. Stickstoff [mg/L] | 18 | 20 | 21 | 19 | 20 | 20 |
| MgSO₄-Stickstoff [mg/L] | 156 | 145 | 141 | 144 | 142 | 140 |
| Polyphenole [mg/L] | 146 | 129 | 97 | 120 | 118 | 95 |
| Antbocyanogene [mg/L] | 34 | 33 | 27 | 31 | 30 | 27 |

Die Stammwürzegehalte korrelieren erwartungsgemäß mit denen der Würzen. Entsprechend einem Vergärungsgrad von 71-73 % liegt der Alkoholgehalt bei 4,3-4,4 Vol.-%. Auf dem Wege von der Würze zum Bier gehen von den Bittereinheiten üblicherweise 1/3 durch die Gärung und weitere 7-8 Bittereinheiten durch die Filtration verloren. Dies wird durch die gemessenen Bitterwerte bestätigt. Wahrend gegenüber der Würze die α-Säuregehalte sehr stark abnehmen, ändert sich der Gehalt an iso-α-Säuren nur wenig. Im mit Xanthohumol dotierten Sud wurde ein Gehalt von 5,7 mg/L gemessen. Abzüglich eines Blindgehaltes aus dem Ursprungssud von 0,3 mg/L ergibt sich demnach eine hohe Wiederfindung von 96 %. Der mit 8- Prenylnaringenin dotierte Sud zeigt ebenfalls eine Wiederfindung von knapp 100%. Der pH und die Farbe liegen im üblichen Bereich eines untergarigen Vollbieres, wobei letztere wie bei den Wurzen mit dem Gehalt an Polyphenolen gut korreliert. Die Gehalte für den Gesamtstickstoff und die Stickstoff-Fraktionen liegen ebenfalls im üblichen Bereich eines untergarigen Vollbieres. Bei den Polyphenolen spiegelt sich der geringere Gehalt in der Würze bei HOP RE auch im Bier wieder. Sowohl Gesamtpolyphenole als auch Anthocyanogene liegen für alle Biere im üblichen Bereich.

### Sensorische Analyse von Bieren mit erfindungsgemäßen Hopefenextrakt

Die sensorische Analyse wurde nach dem DLG-Prüfschema für Bier durchgeführt. Bei der Einzelproben-Prüfung wurden Merkmale nach Art und Ausprägung nach einem festgelegten Punkte-Schema (1-5) bewertet. Zu den Merkmalen gehören der Geruch, die Reinheit des Geschmacks, die Vollmundigkeit, die Rezenz und die Qualität und Intensität der Bittera. Die Ergebnisse sind in nachstehender Tabelle 22 zusammenfasst.

**Tabelle 22: sensorische Analyses gemäß DLG-Prüfschema**

| Prüfmerkmal | Hopst. EtOH | Hopst. CO₂ | HOP RE | HOP R5R | R5R + XN | R5R + 8PN |
|---|---|---|---|---|---|---|
| Geruch | 4.6 | 4,0 | 4,8 | 4.8 | 4.4 | 4,4 |
| Reinheit des Geschmacks | 4,8 | 4,4 | 4,8 | 4.6 | 4,8 | 4.4 |
| Vollmundigkeit | 4,8 | 4,2 | 4.6 | 4.8 | 5,0 | 4,6 |
| Rezenz | 5,0 | 4,8 | 5.0 | 5,0 | 4,6 | 4.8 |
| Qualität der Bittere | 4,0 | 4,6 | 4.6 | 4,4 | 4,2 | 4,2 |
| Intensität der Bittere | 4,0 | 3.8 | 3,8 | 3,8 | 3.8 | 4,0 |

Hinsichtlich des Geruchs werden die Biere HOP RE und HOP R5R als "rein" klassifiziert, die Erwartungen an den Geruch sind voll erfüllt. Selbst der Geruch des mit dem niedrigsten Wert beurteilten Hopst. CO₂ kann hierbei als "noch rein" bezeichnet werden. Die Abweichungen sind demnach geringfügig und rühren daher, dass zwei der Prüfer bei diesem Bier den Geruch von Diacetyl wahrgenommen haben, was einem Geruchsfehler gleichkommt.

Im Hinblick auf den Geschmack werden die Biere Hopst. EtOH, HOP RE und R5R+XN als "rein" bewertet, die Erwartungen sind hier voll erfüllt. Bei den anderen Bieren haben im Schnitt mehr Prüfer eine geringfügige Abweichung des erwarteten Geschmacks konstatiert, aber man kann selbst bei den niedrigsten Werten von einem weitgehend reinen Geschmack sprechen.

Die Vollmundigkeit wird bei R5R+XN von allen Prüfern als sortentypisch bewertet. Selbst das mit dem geringsten Wert beurteilte Hopst. CO₂ wird noch als "typisch" bewertet, die Abweichungen sind demnach selbst bei diesem Bier geringfügig.

Die Erwartung an die Rezenz wird bei den Bieren Hopst EtOH, HOP RE und HOP R5R nach Meinung aller Prüfer voll erfüllt. Diese Biere werden durchweg als "angenehm rezent" bewertet. Aber auch die Abweichungen bei den anderen Bieren sind sehr geringfügig.

Die Qualität der Bittere kann bei den Bieren Hopst. CO₂ und HOP RE als "sehr fein" bezeichnet werden. Aber auch bei dem mit der niedrigsten Note bewerteten Bier Hopst. EtOH wird die Qualität der Bittere insgesamt noch als "fein" beurteilt. Die Intensität der Bittere wird im Durchschnitt bei allen Bieren als "bitter" bewertet, die Abweichung von der erwarteten Intensität ist geringfügig. Diese Beurteilung entspricht bei einem Pils eher einer mittleren Bittere. Stärker gehopfte Pils-Biere werden üblicherweise mit 5 bewertet. Die analytisch gemessenen, wesentlich höheren Bittereinheiten bei HOP R5R und den aus diesem Bier hergestellten Bieren mit Zusatz an Xanthohumol und 8-Prenylnaringenin haben offenbar keinen Einfluss auf die sensorische Geschmacksqualität hinsichtlich der Bittere.

Insgesamt ergibt sich für die verschiedenen Biere ein vergleichbares und zudem gutes sensorisches Ergebnis. Die Gleichartigkeit ist nahe liegend, da es sich um frisches Bier handelt. bei dem bis auf die Hopfengabe die Zutaten und der gesamte Brauprozess vergleichbar sind.

## Patentansprüche

1. Chromatographisches Elutionsverfahren zur klassifizierten Gewinnung und Trennung von pflanzlichen Inhaltsstoffen mittels mehrerer Auswaschungsvorgänge, wobei das Verfahren umfasst:
Zerkleinern der die pflanzlichen Inhaltsstoffe umfassenden pflanzlichen Ausgangsmaterialien,
Befüllen eines Elutionsbehälters mit den zerkleinerten pflanzlichen Ausgangsmaterialien, wobei der Elutionsbehälter mindestens einen Zufluss und mindestens einen Abfluss aufweist, wobei vor dem mindestens einen Abfluss ausschließlich eine oder mehrere Rückhaltevorrichtungen für das zerkleinerte Material angeordnet sind,
Durchführen eines ersten kontinuierlichen Auswaschungsvorgangs mit einem ersten Lösungsmittel oder Lösungsmittelgemisch,
Durchführen eines zweiten kontinuierlichen Auswaschungsvorgangs mit einem zweiten Lösungsmittel oder Lösungsmittelgemisch, wobei sich das zweite Lösungsmittel oder Lösungsmittelgemisch von dem ersten Lösungsmittel oder Lösungsmittelgemisch unterscheidet,
optional Durchführen weiterer kontinuierlicher Auswaschungsvorgänge mit jeweils einem weiteren Lösungsmittel oder Lösungsmittelgemisch, wobei sich jedes weitere Lösungsmittel oder Lösungsmittelgemisch von jedem zuvor verwendeten Lösungsmittel oder Lösungsmittelgemisch unterscheidet,
wobei die Elution unter Verwendung der pflanzlichen Eigencellulose der pflanzlichen Ausgangsmaterialien als feste/stationäre Phase durchgeführt wird und wobei die Auswaschungsvorgänge bei Raumtemperatur (20-24°C) und Normaldruck (950-1060 mbar) durchgeführt werden.

2. Verfahren nach Anspruch 1, wobei das Zerkleinern der die pflanzlichen Inhaltsstoffe umfassenden pflanzlichen Ausgangsmaterialien auf Stücke von maximal 1 cm³ Rauminhalt, vorzugsweise von maximal 10 mm³ Rauminhalt, vorzugsweise von maximal 1 mm³ Rauminhalt erfolgt.

3. Verfahren nach einem der vorstehenden Ansprüche, wobei das Ausgangsmaterial in zerkleinerter Form mit Auflockerungsmaterialien vermischt wird, wobei die Auflockerungsmaterialien vorzugsweise ausgewählt sind, aus der Gruppe bestehend aus, Sand, Kieselgur, Zellulose.

4. Verfahren nach einem der vorstehenden Ansprüche, wobei der Elutionsbehälter in Fließrichtung unterhalb der das Ausgangsmaterial in zerkleinerter Form umfassenden Schicht frei von einer Schicht aus zugegebenen Material mit Auftrennungseigenschaften ist.

5. Verfahren nach einem der vorstehenden Ansprüche, wobei das Ausgangsmaterial in trockener Form vorliegt.

6. Verfahren nach einem der vorstehenden Ansprüche, wobei die kontinuierlichen Auswaschvorgänge mit Lösungsmitteln oder Lösungsmittelgemischen abnehmender Polarität durchgeführt werden.

7. Verfahren nach Anspruch 1, wobei das pflanzliche Ausgangsmaterial Hopfenmaterial ist.

8. Verfahren nach Anspruch 7, wobei das Hopfenmaterial ausgewählt ist, aus der Gruppe bestehend aus, Hopfendolden und Hopfenverarbeitungsprodukte, wobei die Hopfenverarbeitungsprodukte vorzugsweise Hopfenpellets, Hopfeneluate und Rückstandsmaterial einer Hopfen-Elution umfassen.

9. Verfahren nach einem der Ansprüche 7 bis 8, wobei das Zerkleinern der die pflanzlichen Inhaltsstoffen umfassenden pflanzlichen Ausgangsmaterialien auf Stücke von maximal 1 cm³ Rauminhalt, vorzugsweise von maximal 10 mm³ Rauminhalt, bevorzugter Weise von maximal 1 mm³ Rauminhalt erfolgt.

10. Verfahren nach einem der vorstehenden Ansprüche 7 bis 9, wobei das Ausgangsmaterial in zerkleinerter Form mit Auflockerungsmaterialien vermischt wird, wobei die Auflockerungsmaterialien vorzugsweise ausgewählt sind, aus der Gruppe bestehend aus, Sand, Kieselgur, Zellulose.

11. Verfahren nach einem der Ansprüche 7 bis 10, wobei eine Auswaschung mit mindestens einem Lösungsmittel oder Lösungsmittelgemisch erfolgt, das ausgewählt ist, aus der Gruppe bestehend aus Wasser, Gemisch bestehend aus Wasser und Ethanol, Gemisch umfassend mindestens 70 Gew.-% an Wasser und/oder Ethanol, und Ethanol.

12. Verfahren nach einem der Ansprüche 7 bis 11, wobei der mehrstufige Auswaschungsvorgang mindestens zwei, vorzugsweise mindestens drei, bevorzugter Weise mindestens vier der nachstehenden Stufenelutionen umfasst, A) Elution mit Wasser, B) Elution mit Ethanol/Wasser-Mischung 25 bis 35 Vol.-% an Ethanol, C) Elution mit Ethanol/Wasser-Mischung 45 bis 55 Vol.-% an Ethanol, D) Elution mit Ethanol/Wasser-Mischung mit 75 bis 85 Vol.-% an Ethanol, E) Elution mit Ethanol/Wasser-Mischung mit 90 bis 96 Vol.-% an Ethanol.

13. Verfahren nach einem der Ansprüche 7 bis 12 zur Gewinnung von 6-Prenylnaringenin und/oder 8-Prenylnaringenin.

14. Verfahren nach einem der Ansprüche 7 bis 12 zur Gewinnung von wasserlöslichen Hopfeninhaltsstoffen, insbesondere Gerbstoffen.

15. Verfahren nach einem der Ansprüche 7 bis 12 zur Gewinnung von Hopfeneluat, der an einem oder mehreren Hopfeninhaltstoffen angereichert oder abgereichert ist.

16. Verfahren nach einem der Ansprüche 7 bis 15 zur Gewinnung einer Substanz oder eines Substanzgemisches ausgewählt aus der Gruppe bestehend aus: Xanthohumol, Xanthohumol-abgereicherter Hopfeneluat, Xanthohumol-angereicherter Hopfeneluat, 6-Prenylnaringenin, 6-Prenylnaringenin-abgereicherter Hopfeneluat, 6-Prenylnaringenin-angereicherter Hopfeneluat, 8-Prenylnaringenin, 8-Prenylnaringenin-abgereicherter Hopfeneluat, 8-Prenylnaringenin-angereicherter Hopfeneluat, Adlupulon, Adlupulonabgereicherter Hopfeneluat, Adlupulon-angereicherter Hopfeneluat, Colupulon, Colupulonabgereicherter Hopfeneluat, Colupulon-angereicherter Hopfeneluat, Lupulon, Lupulonabgereicherter Hopfeneluat, Lupulon-angereicherter Hopfeneluat, Humulon, Humulonabgereicherter Hopfeneluat, Humulon-angereicherter Hopfeneluat, Adhumulon, Adhumulonabgereicherter Hopfeneluat, Adhumulon-angereicherter Hopfeneluat, Cohumulon, Cohumulon-abgereicherter Hopfeneluat, Cohumulon-angereicherter Hopfeneluat, Bittersäuren, Bittersäuren-abgereicherter Hopfeneluat, Bittersäuren-angereicherter Hopfeneluat, ß-Bittersäuren, ß-Bittersäuren-abgereicherter Hopfeneluat, ß-Bittersäuren-angereicherter Hopfeneluat, Humulon-angereichertes Gemisch von Bittersäuren, Lupulonangereichertes Gemisch von ß-Bittersäuren oder Gemische hiervon.

17. Verfahren nach einem der Ansprüche 7 bis 16, wobei das Ausgangsmaterial in trockener Form vorliegt.

18. Verfahren nach einem der Ansprüche 7 bis 17, wobei die kontinuierlichen Auswaschvorgänge mit Lösungsmitteln oder Lösungsmittelgemischen abnehmender Polarität durchgeführt werden.

## Claims

1. A chromatographic elution method for the graded extraction and separation of vegetable ingredients by means of a plurality of elution procedures, wherein the method comprises:
crushing the vegetable starting materials containing the vegetable ingredients;
filling an elution container with the crushed vegetable starting materials, wherein the elution container comprises at least one inlet and at least one outlet, wherein solely one or more devices for retaining the crushed material are arranged upstream of the at least one outlet;
carrying out a first continuous elution procedure with a first solvent or solvent mixture;
carrying out a second continuous elution procedure with a second solvent or solvent mixture, wherein the second solvent or solvent mixture is different from the first solvent or solvent mixture;
optionally, carrying out further continuous elution procedures each time with a further solvent or solvent mixture, wherein each further solvent or solvent mixture is different from each of the previously employed solvents or solvent mixtures;
wherein elution is carried out using vegetable cellulose belonging to the vegetable starting materials as the solid/stationary phase and wherein the elution procedure is carried out at ambient temperature (20-24°C) and under normal pressure (950-1060 mbar).

2. The method according to claim 1, wherein the vegetable starting materials comprising the vegetable ingredients are crushed into pieces with a maximum volume of 1 cm³, preferably a maximum volume of 10 mm³, preferably a maximum volume of 1 mm³.

3. The method according to one of the preceding claims, wherein the starting material in its crushed form is mixed with disaggregation materials, wherein the disaggregation materials are preferably selected from the group consisting of sand, diatomaceous earth and cellulose.

4. The method according to one of the preceding claims, wherein the elution container has no layer of added material with separation properties below the layer comprising the starting material in crushed form in the direction of flow.

5. The method according to one of the preceding claims, wherein the starting material is in the dry form.

6. The method according to one of the preceding claims, wherein the continuous elution procedures are carried out with solvents or solvent mixtures of decreasing polarity.

7. The method according to claim 1, wherein the vegetable starting material is hop material.

8. The method according to claim 7, wherein the hop material is selected from the group consisting of whole hops and processed hop products, wherein the processed hop products preferably comprise hop pellets, hop eluates and residues from hop elution.

9. The method according to claim 7 or claim 8, wherein the vegetable starting material comprising the vegetable ingredients is preferably crushed into pieces with a maximum volume of 1 cm³, preferably a maximum volume of 10 mm³, more preferably a maximum volume of 1 mm³.

10. The method according to one of the preceding claims 7 to 9, wherein the starting material in crushed form is mixed with disaggregation materials, wherein the disaggregation materials are preferably selected from the group consisting of sand, diatomaceous earth and cellulose.

11. The method according to one of claims 7 to 10, wherein an elution with at least one solvent or solvent mixture is carried out with a solvent selected from the group consisting of water, a mixture consisting of water and ethanol, a mixture comprising at least 70% by weight of water and/or ethanol, and ethanol.

12. The method according to one of claims 7 to 11, wherein the multi-step elution procedure comprises at least two, preferably at least three, more preferably at least four of the following elution steps: A) elution with water; B) elution with an ethanol/water mixture containing 25% to 35% by volume of ethanol; C) elution with an ethanol/water mixture containing 45% to 55% by volume of ethanol; D) elution with an ethanol/water mixture containing 75% to 85% by volume of ethanol; E) elution with an ethanol/water mixture containing 90% to 96% by volume of ethanol.

13. The method according to one of claims 7 to 12, for the extraction of 6-prenylnaringenin and/or 8-prenylnaringenin.

14. The method according to one of claims 7 to 12, for the extraction of water-soluble hop ingredients, in particular tannins.

15. The method according to one of claims 7 to 12, for the extraction of hop eluate which is enriched or depleted in one or more hop ingredients.

16. The method according to one of claims 7 to 15, for the extraction of a substance or a mixture of substances selected from the group consisting of: xanthohumol, xanthohumol-depleted hop eluate, xanthohumol-enriched hop eluate, 6-prenylnaringenin, 6-prenylnaringenin-depleted hop eluate, 6-prenylnaringenin-enriched hop eluate, 8-prenylnaringenin, 8-prenylnaringenin-depleted hop eluate, 8-prenylnaringenin-enriched hop eluate, adlupulone, adlupulone-depleted hop eluate, adlupulone-enriched hop eluate, colupulone, colupulone-depleted hop eluate, colupulone-enriched hop eluate, lupulone, lupulone-deleted hop eluate, lupulone-enriched hop eluate, humulone, humulone-depleted hop eluate, humulone-enriched hop eluate, adhumulone, adhumulone-depleted hop eluate, adhumulone-enriched hop eluate, cohumulone, cohumulone-depleted hop eluate, cohumulone-enriched hop eluate, bitter acids, hop eluate depleted in bitter acids, hop eluate enriched in bitter acids, β-bitter acids, hop eluate depleted in β-bitter acids, hop eluate enriched in β-bitter acids, mixture of bitter acids enriched in humulone, mixture of β-bitter acids enriched in lupulone, or mixtures thereof.

17. The method according to one of claims 7 to 16, wherein the starting material is in the dry form.

18. The method according to one of claims 7 to 17, wherein the continuous elution procedure is carried out with solvents or solvent mixtures of decreasing polarity.

## Revendications

1. Procédé d'élution chromatographique pour la récupération classifiée et la séparation de composants végétaux au moyen de plusieurs processus de lavage, le procédé comprenant :
un broyage des matières végétales de départ comprenant les composants végétaux,
le remplissage d'un réservoir d'élution aves les matières végétales de départ broyées, le réservoir d'élution comportant au moins une entrée et une sortie, à l'avant de l'au moins une sortie étant disposé(s) exclusivement un ou plusieurs dispositifs de retenue pour la matière broyée,
la réalisation d'un premier processus de lavage continu avec un premier solvant ou mélange de solvants,
la réalisation d'un deuxième processus de lavage continu avec un deuxième solvant ou mélange de solvants, le deuxième solvant ou mélange de solvant étant différent du premier solvant ou mélange de solvants,
en option, la réalisation de processus de lavage continus supplémentaires avec chaque fois un autre solvant ou mélange de solvants, chaque solvant ou mélange de solvants supplémentaire étant différent de chaque solvant ou mélange de solvants préalablement utilisé,
l'élution étant réalisée sous utilisation de la propre cellulose végétale des matières végétales de départ en tant que phase solide/stationnaire et les processus de lavage étant réalisés à une température ambiante (20 à 24°C) et sous une pression normale (950-1060 mbar).

2. Procédé selon la revendication 1, le broyage des matières végétales de départ comprenant les composants végétaux s'effectuant en des morceaux d'un volume maximum de 1 cm ³ de préférence d'un volume maximum de 10 mm³ de préférence d'un volume maximum de 1 mm ³.

3. Procédé selon l'une quelconque des revendications précédentes, la matière de départ étant mélangée sous forme broyée avec des matières d'ameublissement, les matières d'ameublissement étant choisies de préférence dans le groupe consistant dans le sable, la diatomite, la cellulose.

4. Procédé selon l'une quelconque des revendications précédentes, dans le sens d'écoulement, en-dessous de la couche comprenant la matière de départ sous forme broyée, le réservoir d'élution étant exempt d'une couche de matière rajoutée avec caractéristiques de sectionnement.

5. Procédé selon l'une quelconque des revendications précédentes, la matière de départ étant présente sous forme sèche.

6. Procédé selon l'une quelconque des revendications précédentes, les processus de lavage continu étant réalisés avec des solvants ou des mélanges de solvants de polarité décroissante.

7. Procédé selon la revendication 1, la matière végétale de départ étant de la matière issue du houblon.

8. Procédé selon la revendication 7, la matière issue du houblon étant choisie dans le groupe consistant dans les cônes du houblon et les produits de traitement du houblon, les produits de traitement du houblon étant de préférence des boulettes de houblon, des éluats du houblon et des matières résiduelles.

9. Procédé selon l'une quelconque des revendications 7 ou 8, le broyage des matières végétales de départ comprenant les composants végétaux s'effectuant en des morceaux d'un volume maximum de 1 cm ^{1,} de préférence d'un volume maximum de 10 mm³ de préférence d'un volume maximum de 1 mm ³.

10. Procédé selon l'une quelconque des revendications précédentes 7 à 9, la matière de départ étant mélangée sous forme broyée avec des matières d'ameublissement, les matières d'ameublissement étant choisies de préférence dans le groupe comprenant le sable, la diatomite, la cellulose.

11. Procédé selon l'une quelconque des revendications 7 à 10, un lavage étant effectué avec au moins un solvant ou un mélange de solvants qui est choisi dans le groupe consistant dans l'eau, dans un mélange d'eau et d'éthanol, dans un mélange comprenant au moins 70 % en poids d'eau et/ou d'éthanol et dans l'éthanol.

12. Procédé selon l'une quelconque des revendications 7 à 11, le processus de lavage en plusieurs étapes comprenant au moins deux, de préférence au moins trois, de manière plus préférée au moins quatre des étapes d'élution suivantes, A) élution à l'eau, B) élution avec un mélange d'éthanol/d'eau à de 25 à 35 % en volume d'éthanol, C) élution avec un mélange d'éthanol/d'eau à de 45 à 55 % en volume d'éthanol, D) élution avec un mélange d'éthanol/d'eau à de 75 à 85 -% en volume d'éthanol, E) élution avec un mélange d'éthanol/d'eau à de 90 à 96 % en volume d'éthanol.

13. Procédé selon l'une quelconque des revendications 7 à 12 pour la récupération de 6-prénylnaringénine et/ou de 8-prénylnaringénine.

14. Procédé selon l'une quelconque des revendications 7 à 12 pour la récupération de composants hydrosolubles du houblon, notamment de tannins.

15. Procédé selon l'une quelconque des revendications 7 à 12 pour la récupération d'éluat du houblon enrichi ou appauvri d'un ou de plusieurs composants du houblon.

16. Procédé selon l'une quelconque des revendications 7 à 15 pour la récupération d'une substance ou d'un mélange de substances consistant dans : le xanthohumol, un éluat du houblon appauvri en xanthohumol, un éluat du houblon enrichi en xanthohumol, la 6-prénylnaringénine, un éluat du houblon appauvri en 6-prénylnaringénine, un éluat du houblon enrichi en 6-prénylnaringénine, la 8-prénylnaringénine, un éluat du houblon appauvri en 8-prénylnaringénine, un éluat du houblon enrichi en 8-prénylnaringénine, l'adlupulone, un éluat du houblon appauvri en adlupulone, un éluat du houblon enrichi en adlupulone, la colupulone, un éluat du houblon appauvri en colupulone, un éluat du houblon enrichi en colupulone, la lupulone, un éluat du houblon appauvri en lupulone, un éluat du houblon enrichi en lupulone, l'humulone, un éluat du houblon appauvri en humulone, un éluat du houblon enrichi en humulone, l'adhumulone, un éluat du houblon appauvri en adhumulone, un éluat du houblon enrichi en adhumulone, la cohumulone, un éluat du houblon appauvri en cohumulone, un éluat du houblon enrichi en cohumulone, des acides amers, un éluat du houblon appauvri en acides amers, un éluat du houblon enrichi en acides amers, les β-acide amers, un éluat du houblon appauvri en β-acides amers, un éluat du houblon enrichi en β-acides amers, un mélange d'acides amers enrichi en humulone, un mélange de β-acides amers enrichi en lupulone ou des mélanges de ces derniers.

17. Procédé selon l'une quelconque des revendications 7 à 16, la matière de départ étant présente sous forme sèche.

18. Procédé selon l'une quelconque des revendications 7 à 17, les processus de lavages continus étant réalisés avec des solvants de polarité décroissante.
